# EUROPEAN PATENT APPLICATION

(11) **EP 4 851 193 A1**
(43) Date of publication of application: **09.12.2026**
(21) Application number: 25747838.8
(22) Date of filing: 24.01.2025
(51) Int. Cl.: C07K 16/28, C12N 15/13, C12N 5/10, A61K 39/395, A61K 47/68, A61P 35/00

(54) **ANTI-CLDN6 ANTIBODY, ANTIBODY-DRUG CONJUGATE THEREOF, AND MEDICAL USE THEREOF**

(30) Priority: 01.02.2024 CN 202410142018
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YU, Xiaolan, Shanghai 200245 (CN); WANG, Shuting, Shanghai 200245 (CN); YAO, Qingqing, Shanghai 200245 (CN); JIN, Xinsheng, Shanghai 200245 (CN); LIU, Shimeng, Shanghai 200245 (CN); HE, Feng, Shanghai 200245 (CN)
(74) Representative: Dragotti & Associati S.P.A.
(86) International application number: PCT/CN2025/074743
(87) International publication number: WO 2025/162270

(57) **Abstract**

Provided are an anti-CLDN6 antibody, an antibody-drug conjugate thereof, and a medical use thereof.

## Description

The present application claims priority to Chinese Patent Application No. 2024101420189 filed on February 1, 2024.

### TECHNICAL FIELD

The present disclosure pertains to the technical field of biology and relates to an anti-CLDN6 antibody, an antibody-drug conjugate thereof, and pharmaceutical use thereof.

### BACKGROUND

The statements herein only provide background information related to the present disclosure and may not necessarily constitute the prior art.

Claudin (hereinafter referred to as CLDN) is a tight junction protein. Human CLDN6 (Claudin 6, hereinafter referred to as hCLDN6) is one of the CLDN family proteins and is a four-pass transmembrane protein containing 220 amino acid residues. CLDN6 expression is not found in adult tissues, but it is expressed at relatively high levels in a variety of solid tumors, including testicular cancer, ovarian cancer, endometrial cancer, non-small cell lung cancer, etc. In contrast, CLDN9, which shares the highest sequence similarity with it, is expressed to some extent in normal tissues.

Among the disclosed CLDN6 antibody-drug conjugates (ADCs), two utilize pyrrolo benzodiazepine (PBD) toxins, and they are Abbive's SC-004 (WO2017096163A1) and Daiichi's DS9606a (WO2019065964A1, WO2020196474A1, and WO2020196712A1). The antibody moieties of the two ADCs described above are both bispecific monoclonal antibodies targeting CLDN6/9 simultaneously. SC-004 was discontinued due to the high toxicity and low efficacy that it showed in clinical trials. Given the extremely low maximum tolerated dose (MTD) of SC-004, which is only 0.2 mg/kg, it remains unclear whether CLDN9-mediated off-tumor toxicity exists at the effective dose. In contrast, the antibody moiety of DS9606a has better cell-binding activity and can well recognize the CLDN6 variant I143V while exhibiting relatively good endocytic activity. The antibody moiety has been engineered to have a silenced Fc region, and the PBD has been attenuated, though its clinical safety profile remains unknown.

When an antibody is conjugated with a highly toxic PBD toxin, simultaneously targeting CLDN9 may pose an off-tumor risk. Therefore, there is an urgent clinical need to develop antibodies that specifically bind to CLDN6 and their corresponding ADC drugs.

### SUMMARY

The present disclosure relates to an anti-CLDN6 antibody comprising a heavy chain variable region and a light chain variable region, wherein the heavy chain variable region comprises a HCDR1, a HCDR2, and a HCDR3 that comprise the amino acid sequences of a HCDR1, a HCDR2, and a HCDR3 from SEQ ID NO: 12, respectively, and the light chain variable region comprises a LCDR1, a LCDR2, and a LCDR3 that comprise the amino acid sequences of a LCDR1, a LCDR2, and a LCDR3 from SEQ ID NO: 32, 31, 30, 33, 34, or 35, respectively.

In some embodiments, provided is the anti-CLDN6 antibody according to the foregoing, wherein the HCDR1, the HCDR2, and the HCDR3 of the heavy chain variable region and the LCDR1, the LCDR2, and the LCDR3 of the light chain variable region are defined according to the same numbering scheme selected from the group consisting of Kabat, IMGT, Chothia, AbM, and Contact. In some embodiments, the CDRs are defined according to the Kabat numbering scheme. In some embodiments, the CDRs are defined according to the IMGT numbering scheme. In some embodiments, the CDRs are defined according to the Chothia numbering scheme. In some embodiments, the CDRs are defined according to the AbM numbering scheme. In some embodiments, the CDRs are defined according to the Contact numbering scheme.

In some embodiments, provided is the anti-CLDN6 antibody according to any one of the foregoing, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 26, 25, 17, 27, 28, or 29, a LCDR2 that comprises the amino acid sequence of SEQ ID NO: 24, and a LCDR3 that comprises the amino acid sequence of SEQ ID NO: 19.

In some embodiments, provided is the anti-CLDN6 antibody according to any one of the foregoing, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 26 or 25, a LCDR2 that comprises the amino acid sequence of SEQ ID NO: 24, and a LCDR3 that comprises the amino acid sequence of SEQ ID NO: 19.

In some embodiments, provided is the anti-CLDN6 antibody according to any one of the foregoing, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 26, a LCDR2 that comprises the amino acid sequence of SEQ ID NO: 24, and a LCDR3 that comprises the amino acid sequence of SEQ ID NO: 19.

In some embodiments, provided is the anti-CLDN6 antibody according to any one of the foregoing, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 25, a LCDR2 that comprises the amino acid sequence of SEQ ID NO: 24, and a LCDR3 that comprises the amino acid sequence of SEQ ID NO: 19.

In some embodiments, the anti-CLDN6 antibody according to any one of the foregoing is a murine-derived antibody, a chimeric antibody, a humanized antibody, or a fully human-derived antibody, preferably a humanized antibody.

In some embodiments, provided is the anti-CLDN6 antibody according to any one of the foregoing, wherein the heavy chain variable region comprises the amino acid sequence of SEQ ID NO: 12, and the light chain variable region comprises the amino acid sequence of SEQ ID NO: 32, 31, 30, 33, 34, or 35.

In some embodiments, provided is the anti-CLDN6 antibody according to any one of the foregoing, wherein the heavy chain variable region comprises the amino acid sequence of SEQ ID NO: 12, and the light chain variable region comprises the amino acid sequence of SEQ ID NO: 32 or 31.

In some embodiments, provided is the anti-CLDN6 antibody according to any one of the foregoing, wherein the heavy chain variable region comprises the amino acid sequence of SEQ ID NO: 12, and the light chain variable region comprises the amino acid sequence of SEQ ID NO: 32.

In some embodiments, provided is the anti-CLDN6 antibody according to any one of the foregoing, wherein the heavy chain variable region comprises the amino acid sequence of SEQ ID NO: 12, and the light chain variable region comprises the amino acid sequence of SEQ ID NO: 31.

In some embodiments, provided is the anti-CLDN6 antibody according to any one of the foregoing, wherein the anti-CLDN6 antibody is an antibody fragment; preferably, the antibody fragment is a Fab, Fab', F(ab')2, Fd, Fv, scFv, dsFv, or dAb.

In some embodiments, provided is the anti-CLDN6 antibody according to any one of the foregoing, wherein the anti-CLDN6 antibody comprises a heavy chain constant region and a light chain constant region; preferably, the heavy chain constant region is a heavy chain constant region of human IgG1, IgG2, IgG3, or IgG4 or a variant thereof, and the light chain constant region is a light chain constant region of human κ or λ or a variant thereof; more preferably, the heavy chain constant region comprises the amino acid sequence of SEQ ID NO: 20, and the light chain constant region comprises the amino acid sequence of SEQ ID NO: 21.

In some embodiments, provided is the anti-CLDN6 antibody according to any one of the foregoing, wherein the anti-CLDN6 antibody comprises a heavy chain and a light chain, wherein the heavy chain comprises the amino acid sequence of SEQ ID NO: 22, and the light chain comprises the amino acid sequence of SEQ ID NO: 38, 37, 36, 39, 40, or 41.

In some embodiments, provided is the anti-CLDN6 antibody according to any one of the foregoing, wherein the heavy chain comprises the amino acid sequence of SEQ ID NO: 22, and the light chain comprises the amino acid sequence of SEQ ID NO: 38 or 37.

In some embodiments, provided is the anti-CLDN6 antibody according to any one of the foregoing, wherein the heavy chain comprises the amino acid sequence of SEQ ID NO: 22, and the light chain comprises the amino acid sequence of SEQ ID NO: 38.

In some embodiments, provided is the anti-CLDN6 antibody according to any one of the foregoing, wherein the heavy chain comprises the amino acid sequence of SEQ ID NO: 22, and the light chain comprises the amino acid sequence of SEQ ID NO: 37.

In some embodiments, provided is the anti-CLDN6 antibody according to any one of the foregoing, wherein the amino acid sequence of the heavy chain is set forth in SEQ ID NO: 22, and the amino acid sequence of the light chain is set forth in SEQ ID NO: 38.

In some embodiments, provided is the anti-CLDN6 antibody according to any one of the foregoing, wherein the amino acid sequence of the heavy chain is set forth in SEQ ID NO: 22, and the amino acid sequence of the light chain is set forth in SEQ ID NO: 37.

In another aspect, the present disclosure relates to an antibody-drug conjugate or a pharmaceutically acceptable salt thereof, which is represented by general formula (I): 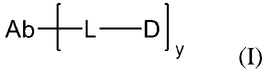 wherein Ab is the anti-CLDN6 antibody according to any one of the foregoing;
L is a linker linking Ab and D;
Ab binds directly to L via an amino acid thereof, or Ab binds to L via a glycan chain or remodeled glycan chain thereof;
y is 1 to 10;
D is represented by general formula (D1), formula (D1-S), or formula (D1-R): 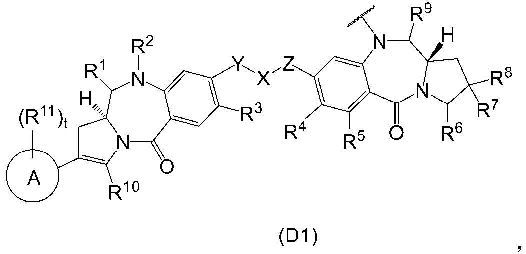 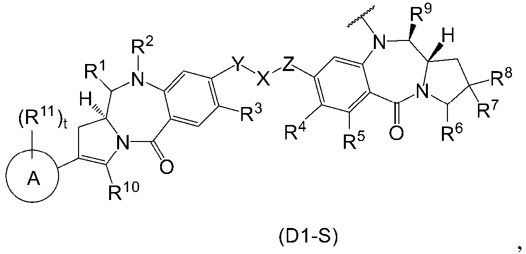 or 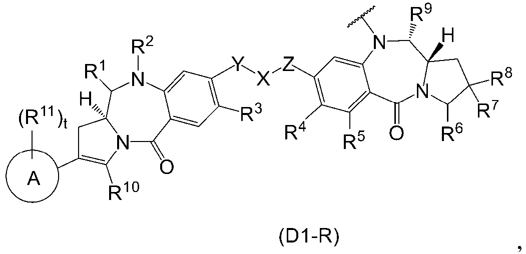 wherein:
   X is (CR^{a}R^{b})ₛ;
   Y and Z are identical or different and are each independently selected from the group consisting of oxygen, sulfur, and NR^{c};
   R¹ is selected from the group consisting of hydrogen, halogen, and alkyl; or R¹, together with the carbon atom to which it is attached, forms C=O;
   R² is hydrogen or alkyl;
   or R¹ and R², together with the carbon atom and the nitrogen atom to which they are attached, form C=N;
   R³ and R⁴ are identical or different and are each independently selected from the group consisting of hydrogen, alkyl, OR^{d}, halogen, haloalkyl, hydroxyalkyl, SH, S-alkyl, and NR^{e}R^{f};
   R⁵ is selected from the group consisting of hydrogen, halogen, alkyl, and haloalkyl; R⁶ is selected from the group consisting of hydrogen, halogen, alkyl, and haloalkyl; or R⁶, together with the carbon atom to which it is attached, forms C=O;
   R⁷ and R⁸ are identical or different and are each independently selected from the group consisting of hydrogen, halogen, alkyl, and haloalkyl; or R⁷ and R⁸, together with the carbon atom to which they are attached, form C=O; or R⁷ and R⁸, together with the carbon atom to which they are attached, form cycloalkyl or heterocyclyl, wherein the cycloalkyl or heterocyclyl is independently and optionally substituted with one or more substituents selected from the group consisting of oxo, halogen, alkyl, haloalkyl, cyano, NR^{g}R^{h}, and ORⁱ;
   R⁹ is hydroxy or alkoxy;
   R¹⁰ is selected from the group consisting of hydrogen, hydroxy, alkyl, haloalkyl, hydroxyalkyl, and alkoxy;
   ring A is selected from the group consisting of cycloalkyl, heterocyclyl, aryl, and heteroaryl;
   R¹¹ is selected from the group consisting of hydrogen, halogen, alkyl, haloalkyl, OR^{j}, C(O)R^{k}, C(O)OR^{k}, cycloalkyl, heterocyclyl, cycloalkylalkyl, heterocyclylalkyl, aryl, heteroaryl, arylalkyl, and heteroarylalkyl;
   R^{a} and R^{b} are identical or different and are each independently selected from the group consisting of hydrogen, halogen, alkyl, haloalkyl, alkoxy, hydroxy, cyano, amino, and hydroxyalkyl;
   R^{c}, R^{e}, R^{f}, R^{g}, and R^{h} are identical or different and are each independently selected from the group consisting of hydrogen, alkyl, and haloalkyl;
   R^{d}, Rⁱ, and R^{j} are identical or different and are each independently selected from the group consisting of hydrogen, alkyl, haloalkyl, hydroxyalkyl, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, cyano, amino, hydroxy, alkoxy, and hydroxyalkyl;
   R^{k} are identical or different and are each independently selected from the group consisting of hydrogen, alkyl, haloalkyl, hydroxy, and hydroxyalkyl;
   s is 1, 2, 3, 4, 5, or 6;
   t is 1, 2, 3, 4, or 5.

In some embodiments, provided is the antibody-drug conjugate or the pharmaceutically acceptable salt thereof according to any one of the foregoing, wherein Y and Z are both oxygen.

In some embodiments, provided is the antibody-drug conjugate or the pharmaceutically acceptable salt thereof according to any one of the foregoing, wherein R¹ is hydrogen. In some embodiments, provided is the antibody-drug conjugate or the pharmaceutically acceptable salt thereof according to any one of the foregoing, wherein R² is hydrogen. In some embodiments, provided is the antibody-drug conjugate or the pharmaceutically acceptable salt thereof according to any one of the foregoing, wherein ring A is 6- to 10-membered aryl; preferably, ring A is phenyl.

In some embodiments, provided is the antibody-drug conjugate or the pharmaceutically acceptable salt thereof according to any one of the foregoing, wherein D is represented by general formula (D2), formula (D2-S), or formula (D2-R): 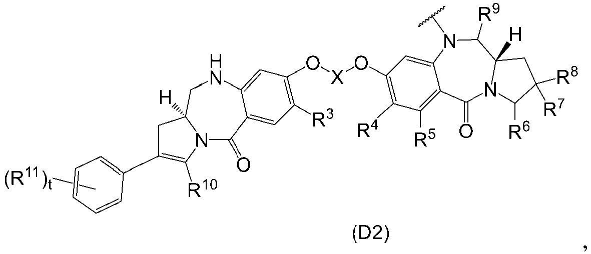 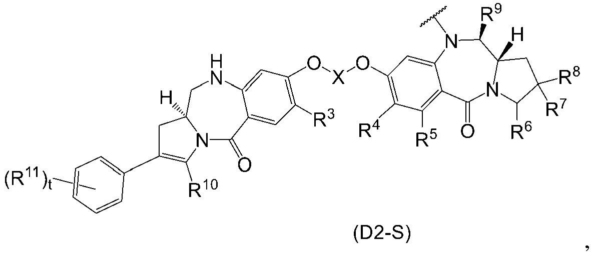 or 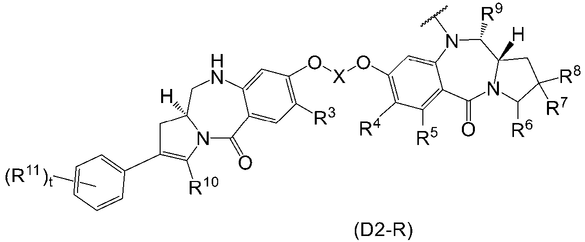 wherein X, R³ to R¹¹, and t are as defined in general formula (I).

In some embodiments, provided is the antibody-drug conjugate or the pharmaceutically acceptable salt thereof according to any one of the foregoing, wherein X is (CR^{a}R^{b})ₛ, wherein R^{a} and R^{b} are identical or different and are each independently selected from the group consisting of hydrogen, halogen, C₁₋₆ alkyl, C₁₋₆ haloalkyl, C₁₋₆ alkoxy, hydroxy, and C₁₋₆ hydroxyalkyl; preferably, X is (CH₂)ₛ, wherein s is 1, 2, 3, 4, 5, or 6.

In some embodiments, provided is the antibody-drug conjugate or the pharmaceutically acceptable salt thereof according to any one of the foregoing, wherein R³ and R⁴ are both OR^{d}, and R^{d} is selected from the group consisting of hydrogen, C₁₋₆ alkyl, C₁₋₆ haloalkyl, C₁₋₆ hydroxyalkyl, 3- to 6-membered cycloalkyl, and 3- to 6-membered heterocyclyl, wherein the 3- to 6-membered cycloalkyl or 3- to 6-membered heterocyclyl is independently and optionally substituted with one or more substituents selected from the group consisting of oxo, halogen, C₁₋₆ alkyl, C₁₋₆ haloalkyl, cyano, amino, hydroxy, C₁₋₆ alkoxy, and C₁₋₆ hydroxyalkyl; preferably, R³ and R⁴ are both OR^{d}, and R^{d} is C₁₋₆ alkyl or 3- to 6-membered cycloalkyl; more preferably, R³ is C₁₋₆ alkoxy, and R⁴ is 3- to 6-membered cycloalkyloxy; most preferably, R³ is methoxy, and R⁴ is cyclopropyloxy.

In some embodiments, provided is the antibody-drug conjugate or the pharmaceutically acceptable salt thereof according to any one of the foregoing, wherein R⁵ is hydrogen. In some embodiments, provided is the antibody-drug conjugate or the pharmaceutically acceptable salt thereof according to any one of the foregoing, wherein R⁶ is hydrogen. In some embodiments, provided is the antibody-drug conjugate or the pharmaceutically acceptable salt thereof according to any one of the foregoing, wherein R⁷ and R⁸ are identical or different and are each independently selected from the group consisting of hydrogen, halogen, C₁₋₆ alkyl, and C₁₋₆ haloalkyl; or R⁷ and R⁸, together with the carbon atom to which they are attached, form 3- to 6-membered cycloalkyl or 3- to 6-membered heterocyclyl, wherein the 3- to 6-membered cycloalkyl or 3- to 6-membered heterocyclyl is independently and optionally substituted with one or more substituents selected from the group consisting of oxo, halogen, C₁₋₆ alkyl, C₁₋₆ haloalkyl, hydroxy, and amino; preferably, R⁷ and R⁸, together with the carbon atom to which they are attached, form 3-to 6-membered cycloalkyl, wherein the 3- to 6-membered cycloalkyl is optionally substituted with one or more substituents selected from the group consisting of oxo, halogen, C₁₋₆ alkyl, C₁₋₆ haloalkyl, hydroxy, C₁₋₆ hydroxyalkyl, and amino; more preferably, R⁷ and R⁸, together with the carbon atom to which they are attached, form 3-to 6-membered cycloalkyl; most preferably, R⁷ and R⁸, together with the carbon atom to which they are attached, form cyclopropyl.

In some embodiments, provided is the antibody-drug conjugate or the pharmaceutically acceptable salt thereof according to any one of the foregoing, wherein R⁹ is hydroxy.

In some embodiments, provided is the antibody-drug conjugate or the pharmaceutically acceptable salt thereof according to any one of the foregoing, wherein R¹⁰ is selected from the group consisting of hydrogen, hydroxy, C₁₋₆ alkyl, C₁₋₆ haloalkyl, C₁₋₆ hydroxyalkyl, and C₁₋₆ alkoxy; preferably, R¹⁰ is hydrogen.

In some embodiments, provided is the antibody-drug conjugate or the pharmaceutically acceptable salt thereof according to any one of the foregoing, wherein R¹¹ is selected from the group consisting of hydrogen, halogen, C₁₋₆ alkyl, C₁₋₆ haloalkyl, hydroxy, and C₁₋₆ alkoxy; preferably, R¹¹ is hydrogen or C₁₋₆ alkoxy; more preferably, R¹¹ is methoxy.

In some embodiments, provided is the antibody-drug conjugate or the pharmaceutically acceptable salt thereof according to any one of the foregoing, wherein D is selected from the group consisting of the following structures: 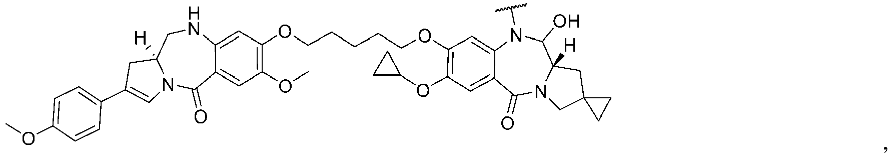 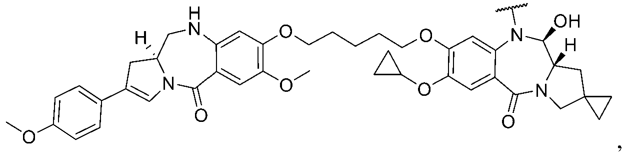 and

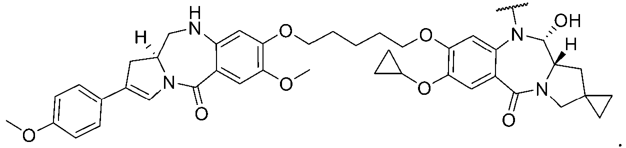

In some embodiments, provided is the antibody-drug conjugate or the pharmaceutically acceptable salt thereof according to any one of the foregoing, wherein L is -L^{a}-L^{b}-L^{c}-L^{d}-; L^{a} is selected from the group consisting of 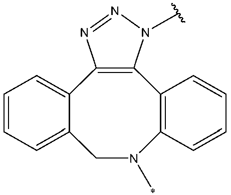 and 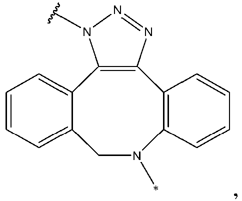 wherein the asterisk * indicates binding to L^{b}, and the wavy line 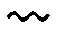 indicates binding to the glycan chain or remodeled glycan chain of Ab;
L^{b} is selected from the group consisting of -C(O)-(CR^{m}Rⁿ-CR^{p}R^{q})ₜ₁-C(O)-, -C(O)-(CR^{m}Rⁿ-CR^{p}R^{q})ₜ₁-C(O)-NR^{s}-(CR^{m}Rⁿ-CR^{p}R^{q})ₜ₂-C(O)-, -C(O)-(CR^{m}Rⁿ-CR^{p}R^{q})t₁-C(O)-NR^{s}-(CR^{m}Rⁿ-CR^{p}R^{q}-O)ₜ₂-CR^{u}R^{v}-C(O)-, -C(O)-(CR^{m}Rⁿ-CR^{p}R^{q})ₜ₁-NR^{s}-C(O)-(CR^{m}Rⁿ-CR^{p}R^{q}-O)ₜ₂-(CR^{m}Rⁿ-CR^{p}R^{q})ₜ₃-C(O)-, and -(CR^{u}R^{v})ₜ₄-O-C(O)-;
L^{c} is a peptide residue composed of 2 to 7 amino acids;
L⁴ is -NR^{w}-W-CR^{x}R^{y}O-C(O)-, -NR^{w}-CR^{x}R^{y}O-R^{z}-C(O)-, or a chemical bond;
R^{m}, Rⁿ, R^{p}, R^{q}, R^{u}, R^{v}, R^{x}, and R^{y} are identical or different and are each independently selected from the group consisting of hydrogen, halogen, alkyl, haloalkyl, alkoxy, hydroxyalkyl, hydroxy, cyano, amino, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
or R^{m} and Rⁿ, together with the carbon atom to which they are attached, form cycloalkyl or heterocyclyl, R^{p} and R^{q}, together with the carbon atom to which they are attached, form cycloalkyl or heterocyclyl, R^{u} and R^{v}, together with the carbon atom to which they are attached, form cycloalkyl or heterocyclyl, and R^{x} and R^{y}, together with the carbon atom to which they are attached, form cycloalkyl or heterocyclyl, wherein the cycloalkyl or heterocyclyl is independently and optionally substituted with one or more substituents selected from the group consisting of oxo, halogen, alkyl, haloalkyl, alkoxy, hydroxyalkyl, hydroxy, cyano, and amino;
or R^{m} and R^{p}, together with the carbon atoms to which they are attached, form cycloalkyl or heterocyclyl, wherein the cycloalkyl or heterocyclyl is independently and optionally substituted with one or more substituents selected from the group consisting of oxo, halogen, alkyl, haloalkyl, alkoxy, hydroxyalkyl, hydroxy, cyano, and amino;
R^{s}, R^{w}, and R^{z} are identical or different and are each independently selected from the group consisting of hydrogen, alkyl, haloalkyl, hydroxyalkyl, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
W is aryl or heteroaryl, wherein the aryl or heteroaryl is independently and optionally substituted with one or more substituents selected from the group consisting of halogen, hydroxy, cyano, amino, alkyl, haloalkyl, and alkoxy; preferably, W is phenyl or 5- or 6-membered heteroaryl, wherein the phenyl or 5- or 6-membered heteroaryl is independently and optionally substituted with one or more substituents selected from the group consisting of halogen, oxo, hydroxy, cyano, amino, C₁₋₆ alkyl, C₁₋₆ haloalkyl, and C₁₋₆ alkoxy;
t1, t2, t3, and t4 are identical or different and are each independently 1, 2, 3, 4, 5, or 6.

In some embodiments, provided is the antibody-drug conjugate or the pharmaceutically acceptable salt thereof according to any one of the foregoing, wherein L^{b} is -C(O)-(CR^{m}Rⁿ-CR^{p}R^{q})ₜ₁-C(O)-, wherein R^{m}, Rⁿ, R^{p}, and R^{q} are identical or different and are each independently hydrogen or C₁₋₆ alkyl, and t1 is 1, 2, 3, 4, 5, or 6; preferably, L^{b} is - C(O)-CH₂-CH₂-C(O)-.

In some embodiments, provided is the antibody-drug conjugate or the pharmaceutically acceptable salt thereof according to any one of the foregoing, wherein L^{c} is selected from the group consisting of peptide residues formed from amino acids among phenylalanine (F), alanine (A), proline (P), isoleucine (I), leucine (L), glycine (G), valine (V), lysine (K), citrulline (Cit), serine (S), glutamic acid (E), and aspartic acid (D), wherein the peptide residues are each independently and optionally substituted with one or more substituents selected from the group consisting of halogen, hydroxy, cyano, amino, C₁₋₆ alkyl, C₁₋₆ haloalkyl, C₁₋₆ alkoxy, 3- to 6-membered cycloalkyl, 3- to 6-membered heterocyclyl, 6- to 10-membered aryl, and 5- to 10-membered heteroaryl; preferably, L^{c} is selected from the group consisting of -GGVA- (SEQ ID NO: 45), -GG-(D-)VA- (SEQ ID NO: 46), -VA-, -GGFG- (SEQ ID NO: 44), -GGPI- (SEQ ID NO: 47), -GGVCit- (SEQ ID NO: 48), -GGVK- (SEQ ID NO: 49), -GG-(D-)PI- (SEQ ID NO: 50), and -GGPL-(SEQ ID NO: 51), wherein (D-)V represents D-valine, and (D-)P represents D-proline; more preferably, L^{c} is -GGVA-(SEQ ID NO: 45).

In some embodiments, provided is the antibody-drug conjugate or the pharmaceutically acceptable salt thereof according to any one of the foregoing, wherein L^{d} is -NR^{w}-W-CR^{x}R^{y}O-C(O)-, wherein R^{w}, R^{x}, and R^{y} are identical or different and are each independently hydrogen or C₁₋₆ alkyl, and W is selected from the group consisting of 1,4-phenyl, 2,5-pyridyl, 3,6-pyridyl, 2,5-pyrimidinyl, and 2,5-thienyl; preferably, L^{d} is -NH-W-CH₂-O-C(O)-, and W is 1,4-phenyl.

In some embodiments, provided is the antibody-drug conjugate or the pharmaceutically acceptable salt thereof according to any one of the foregoing, wherein L is

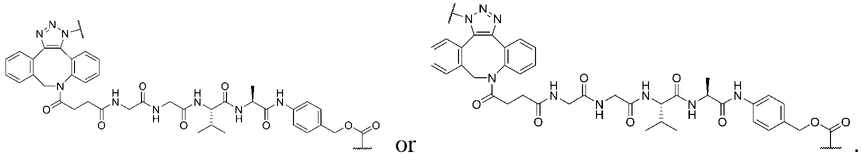

In some embodiments, provided is the antibody-drug conjugate or the pharmaceutically acceptable salt thereof according to any one of the foregoing, wherein Ab binds directly to L via an amino acid residue at position 297 thereof, or Ab binds to L via a glycan chain or remodeled glycan chain at position 297 thereof; preferably, Ab binds to L via a remodeled glycan chain at Asn297 thereof.

In some embodiments, provided is the antibody-drug conjugate or the pharmaceutically acceptable salt thereof according to any one of the foregoing, wherein the remodeled glycan chain has a structure of: 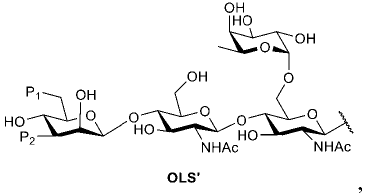 wherein the wavy line 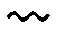 indicates binding to the Asn at position 297 of a heavy chain of Ab;
P₁ and P₂ are identical or different and are each independently selected from the group consisting of hydroxy, *-(CR^{p1}R^{q1}-CR^{s1}R^{t1}-O)s¹-, and *-(CR^{p1}R^{q1}-CR^{s1}R^{t1}-O)s²-(CR^{p1}R^{q1}-CR^{s1}R^{t1}-CR^{x1}R^{y1}-O)s³-(CR^{p1}R^{q1}-CR^{s1}R^{t1}-O)s⁴-, wherein R^{p1}, R^{q1}, R^{s1}, R^{t1}, R^{x1}, and R^{y1} are identical or different and are each independently selected from the group consisting of hydrogen, halogen, C₁₋₆ alkyl, C₁₋₆ haloalkyl, C₁₋₆ alkoxy, C₁₋₆ hydroxyalkyl, hydroxy, cyano, amino, 3- to 6-membered cycloalkyl, 3- to 6-membered heterocyclyl, 6-to 10-membered aryl, and 5- to 10-membered heteroaryl, wherein the 3- to 6-membered cycloalkyl, 3- to 6-membered heterocyclyl, 6- to 10-membered aryl, and 5- to 10-membered heteroaryl are each independently and optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxy, cyano, and amino; the asterisk * indicates binding to the linker L;
s¹ is 1-10, preferably 1-5; s² is 0-10, preferably 1-5; s³ is 1-10, preferably 1-5; s⁴ is 0-10, preferably 1-5;
provided that P₁ and P₂ are not simultaneously hydroxy or *-(CH₂CH₂O)s¹-.

In some embodiments, provided is the antibody-drug conjugate or the pharmaceutically acceptable salt thereof according to any one of the foregoing, wherein the remodeled glycan chain has a structure of: 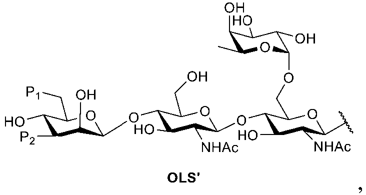 wherein the wavy line 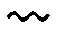 indicates binding to the Asn at position 297 of a heavy chain of Ab; P₁ is hydroxy, and P₂ is *-(CR^{p1}R^{q1} -CR^{s1}R^{t1}-O)ₛ₂-(CR^{p1}R^{q1}-CR^{s1}R^{t1}-CR^{x1}R^{y1}-O)ₛ₃-(CR^{p1}R^{q1}-CR^{s1}R^{t1}-O)ₛ₄-, wherein R^{p1}, R^{q1}, R^{s1}, R^{t1}, R^{x1}, and R^{y1} are identical or different and are each independently selected from the group consisting of hydrogen, halogen, C₁₋₆ alkyl, and C₁₋₆ haloalkyl, s2 is 1, s3 is 1, and s4 is 1.

In some embodiments, provided is the antibody-drug conjugate or the pharmaceutically acceptable salt thereof according to any one of the foregoing, wherein the remodeled glycan chain has a structure of:

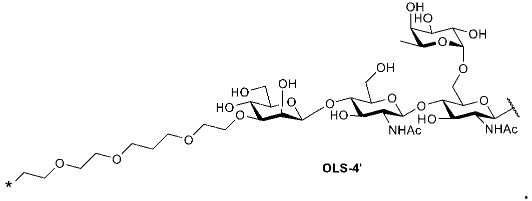

In some embodiments, the antibody-drug conjugate or the pharmaceutically acceptable salt thereof according to any one of the foregoing is represented by general formula (II): 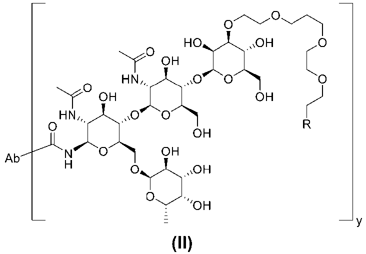 wherein: 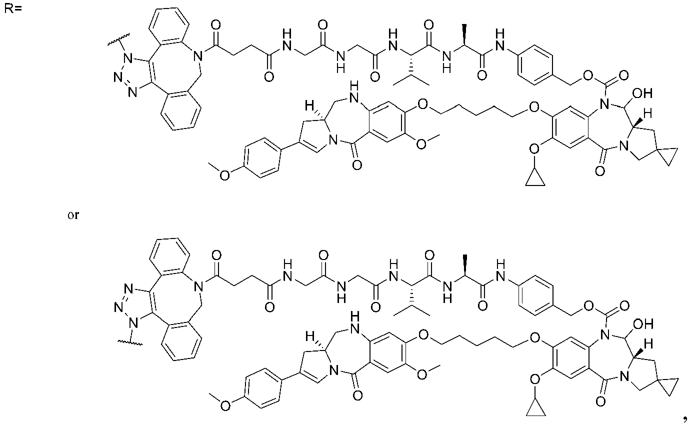 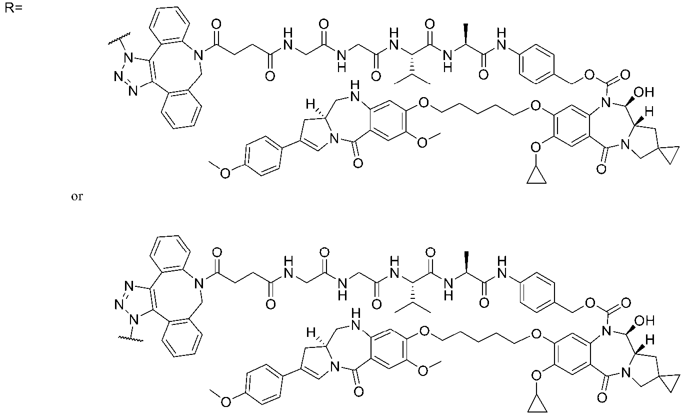 or 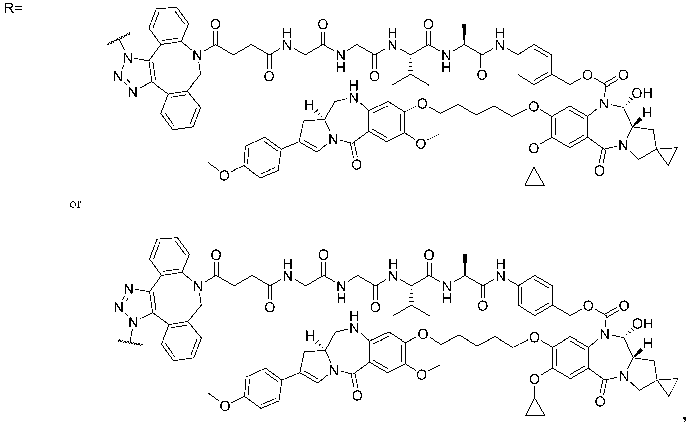
Ab is the anti-CLDN6 antibody according to any one of the foregoing;
y is 1 to 10; preferably, y is 1 to 4; more preferably, y is 1 to 2; most preferably, y is 2. In another aspect, the present disclosure relates to a method for preparing an antibody-drug conjugate represented by general formula (II) or a pharmaceutically acceptable salt thereof, comprising the following step: 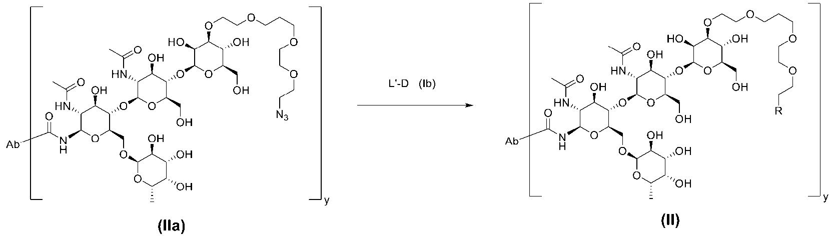
reacting a compound represented by general formula (IIa) or a salt thereof with a compound represented by general formula (Ib) or a salt thereof to give the antibody-drug conjugate represented by general formula (II) or the pharmaceutically acceptable salt thereof,
wherein:
   L' is L^{aa}-L^{b}-L^{c}-L^{d}-;
   L^{aa} is selected from the group consisting of 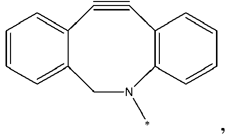 wherein the asterisk * indicates binding to L^{b};
   Ab is the anti-CLDN6 antibody according to any one of the foregoing;
   y is 1 to 10; preferably, y is 1 to 4; more preferably, y is 1 to 2; most preferably, y is 2;
   L^{b}, L^{c}, and L^{d} are as defined in general formula (I);
   R is as defined in general formula (II);
   D is as defined in general formula (I).

In some embodiments, the present disclosure relates to a method for preparing an antibody-drug conjugate represented by general formula (II) or a pharmaceutically acceptable salt thereof, comprising the following step: 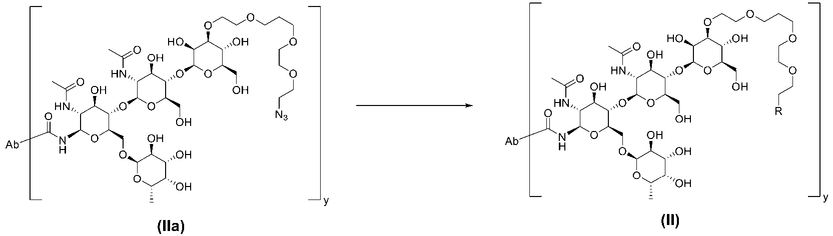 reacting a compound represented by general formula (IIa) or a salt thereof with compound LD-11 or a salt thereof to give the antibody-drug conjugate represented by general formula (II) or the pharmaceutically acceptable salt thereof, 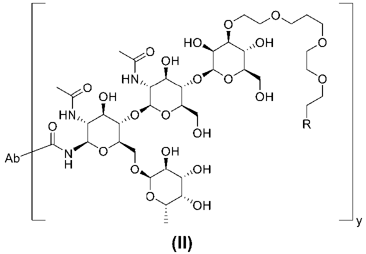 wherein: 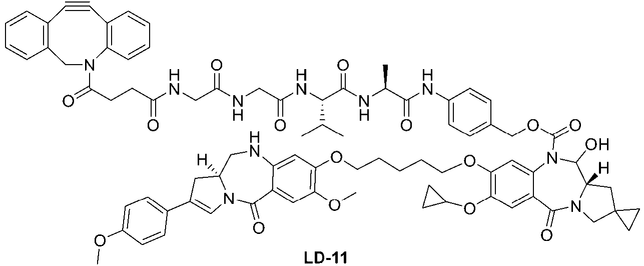
Ab is the anti-CLDN6 antibody according to any one of the foregoing;
y is 1 to 10; preferably, y is 1 to 4; more preferably, y is 1 to 2; most preferably, y is 2;
R is as defined in general formula (II).

In some embodiments, provided is the antibody-drug conjugate or the pharmaceutically acceptable salt thereof according to any one of the foregoing, wherein y is an average of 1, 2, 3, 4, 5, 6, 7, 8, 9, or 10, or a numerical value between any two of these numerical values; preferably, y is 1-10; more preferably, y is an average of 1-8, 1-7, 1-6, 1-5, 1-4, 1-3, 1-2, or 2-8, or 2-7, or 2-6, or 2-5, or 2-4, or 3-8, or 3-7, or 3-6, or 4-8, or 4-7, or 4-6, or 4-5; most preferably, y is 2.

In another aspect, the present disclosure relates to an antibody-drug conjugate or a pharmaceutically acceptable salt thereof, which is represented by general formula (IM): 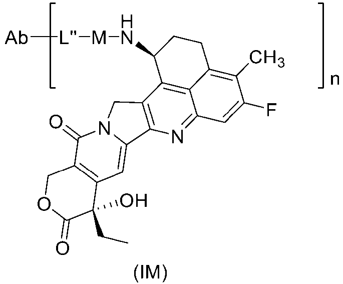 wherein Ab is the anti-CLDN6 antibody according to any one of the foregoing;
M is selected from the group consisting of -O-(CR^{12a}R^{12b})ₘ₁-CR^{13a}R^{13b}-C(O)-, -O-CR^{13a}R^{13b}-(CR^{12a}R^{12b})ₘ₁-, -O-CR^{13a}R^{13b}-, -NH-(CR^{12a}R^{12b})ₘ₁-CR^{13a}R^{13b}-C(O)-, and -S-(CR^{12a}R^{12b})ₘ₁-CR^{13a}R^{13b}-C(O)-;
R^{12a} and R^{12b} are identical or different and are each independently selected from the group consisting of hydrogen, halogen, alkyl, haloalkyl, alkoxy, hydroxy, amino, cyano, nitro, hydroxyalkyl, cycloalkyl, heterocyclyl, aryl, and heteroaryl;
or, R^{12a} and R^{12b}, together with the carbon atom to which they are attached, form cycloalkyl, heterocyclyl, aryl, or heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
R^{13a} is selected from the group consisting of halogen, alkyl, haloalkyl, hydroxy, hydroxyalkyl, alkoxy, cyano, amino, carboxyl, cycloalkyl, cycloalkylalkyl, alkoxyalkyl, heterocyclyl, heterocyclylalkyl, aryl, heteroaryl, arylalkyl, and heteroarylalkyl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
R^{13b} is selected from the group consisting of hydrogen, halogen, alkyl, haloalkyl, hydroxy, hydroxyalkyl, alkoxy, cyano, amino, -(CR^{19a}R^{19b})ₘ₂-NR^{20a}R^{20b}, -(CR^{19a}R^{19b})ₘ₂-COOH, cycloalkyl, cycloalkylalkyl, alkoxyalkyl, heterocyclyl, heterocyclylalkyl, aryl, heteroaryl, arylalkyl, and heteroarylalkyl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
or, R^{13a} and R^{13b}, together with the carbon atom to which they are attached, form cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
or, R^{12a} and R^{13a}, together with the carbon atoms to which they are attached, form cycloalkyl, heterocyclyl, aryl, or heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
R^{19a} and R^{19b} are identical or different and are each independently selected from the group consisting of hydrogen, halogen, alkyl, haloalkyl, alkoxy, hydroxy, amino, cyano, nitro, hydroxyalkyl, cycloalkyl, heterocyclyl, aryl, and heteroaryl;
or, R^{19a} and R^{19b}, together with the carbon atom to which they are attached, form cycloalkyl, heterocyclyl, aryl, or heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
R^{20a} and R^{20b} are identical or different and are each independently selected from the group consisting of hydrogen, alkyl, haloalkyl, hydroxyalkyl, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
or R^{20a} and R^{20b}, together with the nitrogen atom to which they are attached, form heterocyclyl, wherein the heterocyclyl is optionally substituted with one or more substituents selected from the group consisting of oxo, halogen, alkyl, haloalkyl, alkoxy, hydroxyalkyl, hydroxy, cyano, and amino;
m1 is 0, 1, 2, 3, or 4;
m2 is 0, 1, 2, 3, 4, 5, or 6;
n is 1 to 10;
L" is a linker unit.

In some embodiments, provided is the antibody-drug conjugate or the pharmaceutically acceptable salt thereof according to the foregoing, wherein M is -O-(CR^{12a}R^{12b})ₘ₁-CR^{13a}R^{13b}-C(O)-,
wherein R^{12a} and R^{12b} are identical or different and are each independently selected from the group consisting of hydrogen, halogen, and C₁₋₆ alkyl;
R^{13a} is 3- to 6-membered cycloalkyl-C₁₋₆ alkyl or 3- to 6-membered cycloalkyl;
R^{13b} is selected from the group consisting of hydrogen, C₁₋₆ haloalkyl, and 3- to 6-membered cycloalkyl;
or, R^{13a} and R^{13b}, together with the carbon atom to which they are attached, form 3- to 6-membered cycloalkyl;
m1 is 0, 1, 2, 3, or 4.

In some embodiments, provided is the antibody-drug conjugate or the pharmaceutically acceptable salt thereof according to any one of the foregoing, wherein M is -O-(CR^{12a}R^{12b})ₘ₁-CR^{13a}R^{13b}-C(O)-,
wherein R^{12a} and R^{12b} are identical or different and are each independently hydrogen or C₁₋₆ alkyl;
R^{13a} is 3- to 6-membered cycloalkyl;
R^{13b} is selected from the group consisting of hydrogen, C₁₋₆ haloalkyl, and 3- to 6-membered cycloalkyl;
or, R^{13a} and R^{13b}, together with the carbon atom to which they are attached, form 3- to 6-membered cycloalkyl;
m1 is 0, 1, 2, 3, or 4.

In some embodiments, provided is the antibody-drug conjugate or the pharmaceutically acceptable salt thereof according to any one of the foregoing, wherein -L"- is -L¹-L²-L³-L⁴-,
wherein L¹ is selected from the group consisting of -(succinimid-3-yl-*N*)-W¹-C(O)-, - CH₂-C(O)-NR¹⁴-W¹-C(O)-, and -C(O)-W¹-C(O)-, wherein W¹ is selected from the group consisting of C₁₋₆ alkylene and C₁₋₆ alkylene-3- to 6-membered cycloalkyl, wherein the C₁₋₆ alkylene or C₁₋₆ alkylene-3- to 6-membered cycloalkyl is independently and optionally substituted with one or more substituents selected from the group consisting of halogen, hydroxy, cyano, amino, alkyl, C₁₋₆ haloalkyl, C₁₋₆ alkoxy, and 3- to 6-membered cycloalkyl;
L² is selected from the group consisting of -NR¹⁵(CH₂CH₂O)ₚ₁CH₂CH₂C(O)-, - NR¹⁵(CH₂CH₂O)ₚ₁CH₂C(O)-, -S(CH₂)ₚ₁C(O)-, and a chemical bond, wherein p1 is an integer from 1 to 20;
L³ is a peptide residue composed of 2 to 7 amino acid residues, wherein the amino acid residues are selected from the group consisting of amino acid residues formed from amino acids among phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ alkoxy, and 3- to 6-membered cycloalkyl;
L⁴ is selected from the group consisting of -NR¹⁶(CR¹⁷R¹⁸)_{q}-, -C(O)NR¹⁶-, - C(O)NR¹⁶(CH₂)_{q}-, and a chemical bond, wherein q is 1, 2, 3, 4, 5, or 6;
R¹⁴, R¹⁵, and R¹⁶ are identical or different and are each independently selected from the group consisting of hydrogen, C₁₋₆ alkyl, C₁₋₆ haloalkyl, and C₁₋₆ hydroxyalkyl;
R¹⁷ and R¹⁸ are identical or different and are each independently selected from the group consisting of hydrogen, halogen, C₁₋₆ alkyl, C₁₋₆ haloalkyl, hydroxy, C₁₋₆ alkoxy, C₁₋₆ haloalkoxy, and C₁₋₆ hydroxyalkyl.

In some embodiments, provided is the antibody-drug conjugate or the pharmaceutically acceptable salt thereof according to any one of the foregoing, wherein L¹ is 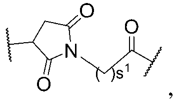 wherein s¹ is 1, 2, 3, 4, 5, or 6; preferably, L¹ is 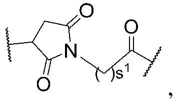 wherein s¹ is 5.

In some embodiments, provided is the antibody-drug conjugate or the pharmaceutically acceptable salt thereof according to any one of the foregoing, wherein L⁴ is - NR¹⁶(CR¹⁷R¹⁸)_{q}-, wherein R¹⁶, R¹⁷, and R¹⁸ are identical or different and are each independently hydrogen or C₁₋₆ alkyl, and q is 1 or 2; preferably, L⁴ is -NR¹⁶(CR¹⁷R¹⁸)_{q}-, wherein R¹⁶, R¹⁷, and R¹⁸ are all hydrogen, and q is 1.

In some embodiments, provided is the antibody-drug conjugate or the pharmaceutically acceptable salt thereof according to any one of the foregoing, wherein -L"- is -L¹-L²-L³-L⁴-;
L¹ is 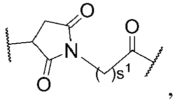 wherein s¹ is 1, 2, 3, 4, 5, or 6;
L² is a chemical bond;
L³ is a tetrapeptide residue; preferably, L³ is a tetrapeptide residue of GGFG (SEQ ID NO: 44);
L⁴ is -NR¹⁶(CR¹⁷R¹⁸)_{q}-, wherein R¹⁶, R¹⁷, and R¹⁸ are identical or different and are each independently hydrogen or C₁₋₆ alkyl, and q is 1 or 2;

the L¹ end of -L"- is linked to Ab, and the L⁴ end is linked to Y.

In some embodiments, provided is the antibody-drug conjugate or the pharmaceutically acceptable salt thereof according to any one of the foregoing, wherein R^{12a} and R^{12b} are identical or different and are each independently hydrogen or C₁₋₆ alkyl; preferably, R^{12a} and R^{12b} are both hydrogen.

In some embodiments, the antibody-drug conjugate or the pharmaceutically acceptable salt thereof according to any one of the foregoing is an antibody-drug conjugate represented by general formula (IIM) or a pharmaceutically acceptable salt thereof: 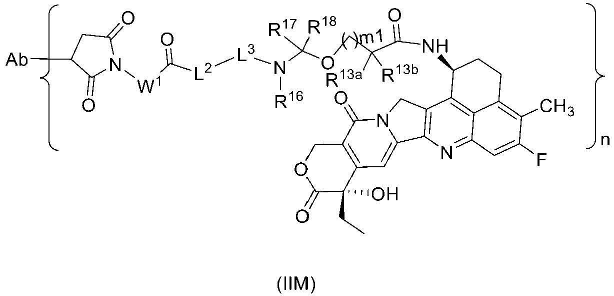 wherein:
Ab is the anti-CLDN6 antibody according to any one of the foregoing;
m1 is 0, 1, 2, 3, or 4;
n is 1 to 10;
R^{13a} is 3- to 6-membered cycloalkyl-C₁₋₆ alkyl or 3- to 6-membered cycloalkyl;
R^{13b} is selected from the group consisting of hydrogen, C₁₋₆ haloalkyl, and 3- to 6-membered cycloalkyl;
or, R^{13a} and R^{13b}, together with the carbon atom to which they are attached, form 3- to 6-membered cycloalkyl;
W¹ is selected from the group consisting of C₁₋₆ alkylene and C₁₋₆ alkylene-3- to 6-membered cycloalkyl, wherein the C₁₋₆ alkylene and C₁₋₆ alkylene-3- to 6-membered cycloalkyl are each independently and optionally substituted with one or more substituents selected from the group consisting of halogen, hydroxy, cyano, amino, C₁₋₆ alkyl, C₁₋₆ haloalkyl, C₁₋₆ alkoxy, and 3- to 6-membered cycloalkyl;
L² is selected from the group consisting of -NR¹⁵(CH₂CH₂O)p¹CH₂CH₂C(O)-, - NR¹⁵(CH₂CH₂O)p¹CH₂C(O)-, -S(CH₂)p¹C(O)-, and a chemical bond, wherein p¹ is 1 to 20;
L³ is a peptide residue composed of 2 to 7 amino acid residues, wherein the amino acid residues are selected from the group consisting of peptide residues formed from amino acids among phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ alkoxy, and 3- to 6-membered cycloalkyl;
R¹⁵ and R¹⁶ are selected from the group consisting of hydrogen, C₁₋₆ alkyl, C₁₋₆ haloalkyl, and C₁₋₆ hydroxyalkyl;
R¹⁷ and R¹⁸ are identical or different and are each independently selected from the group consisting of hydrogen, halogen, C₁₋₆ alkyl, C₁₋₆ haloalkyl, and C₁₋₆ hydroxyalkyl.

In some embodiments, provided is the antibody-drug conjugate or the pharmaceutically acceptable salt thereof according to any one of the foregoing, wherein R^{13a} is 3- to 6-membered cycloalkyl; R^{13b} is hydrogen; or R^{13a} and R^{13b}, together with the carbon atom to which they are attached, form 3- to 6-membered cycloalkyl.

In some embodiments, provided is the antibody-drug conjugate or the pharmaceutically acceptable salt thereof according to any one of the foregoing, wherein m1 is 0 or 1; preferably, m1 is 0.

In some embodiments, provided is the antibody-drug conjugate or the pharmaceutically acceptable salt thereof according to any one of the foregoing, wherein M is selected from the group consisting of 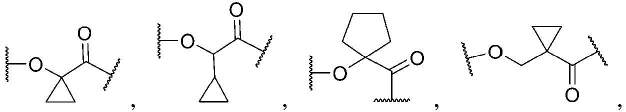 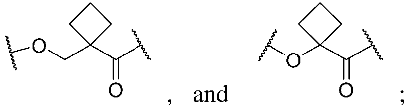 preferably, M is 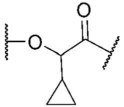 (including 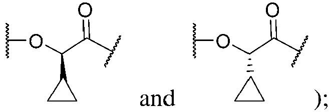 the O-end of M is attached to L".

In some embodiments, provided is the antibody-drug conjugate or the pharmaceutically acceptable salt thereof according to any one of the foregoing, wherein L² is a chemical bond.

In some embodiments, provided is the antibody-drug conjugate or the pharmaceutically acceptable salt thereof according to any one of the foregoing, wherein L³ is a tetrapeptide residue; preferably, L³ is a tetrapeptide residue of GGFG (SEQ ID NO: 44).

In some embodiments, the antibody-drug conjugate or the pharmaceutically acceptable salt thereof according to any one of the foregoing is represented by general formula (IIIM), general formula (IIIM-R), or general formula (IIIM-S): 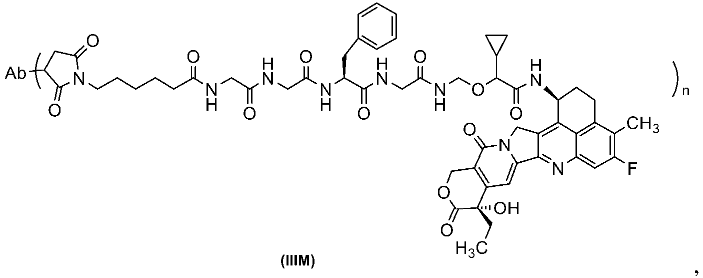 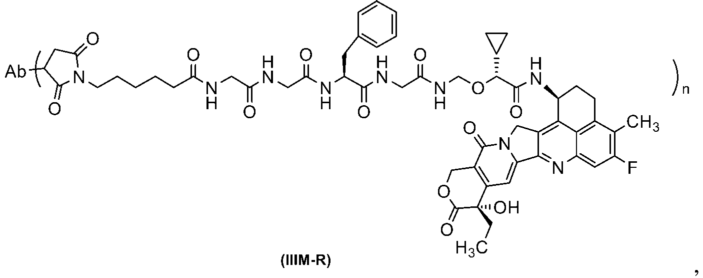 or 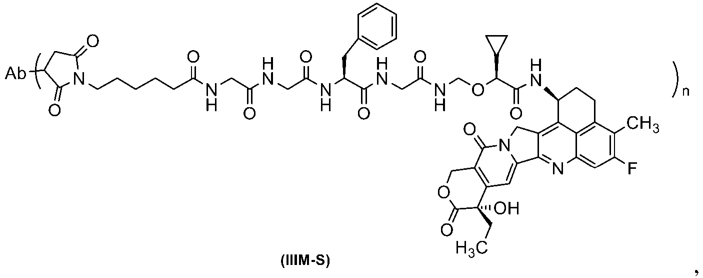 wherein Ab is the anti-CLDN6 antibody according to any one of the foregoing;
n is 1 to 10; preferably, n is 2 to 8; more preferably, n is 4 to 8; most preferably, n is 8. In some embodiments, the antibody-drug conjugate or the pharmaceutically acceptable salt thereof according to any one of the foregoing is represented by general formula (IIIM-R): 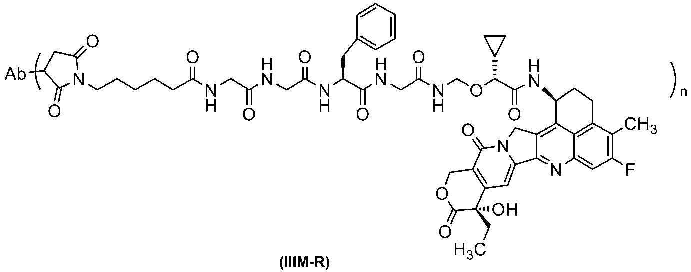
wherein Ab is the anti-CLDN6 antibody according to any one of the foregoing;
n is 1 to 10; preferably, n is 2 to 8; more preferably, n is 4 to 8; most preferably, n is 8. In some embodiments, provided is the antibody-drug conjugate or the pharmaceutically acceptable salt thereof according to any one of the foregoing, wherein n is an average of 1, 2, 3, 4, 5, 6, 7, 8, 9, or 10, or a numerical value between any two of these numerical values; preferably, n is 1-10; more preferably, n is an average of 1-8, 1-7, 1-6, 1-5, 1-4, 1-3, 1-2, or 2-8, or 2-7, or 2-6, or 2-5, or 2-4, or 3-8, or 3-7, or 3-6, or 4-8, or 4-7, or 4-6, or 4-5; most preferably, n is 8.

In another aspect, the present disclosure relates to a method for preparing an antibody-drug conjugate represented by general formula (IIM) or a pharmaceutically acceptable salt thereof, comprising the following step: 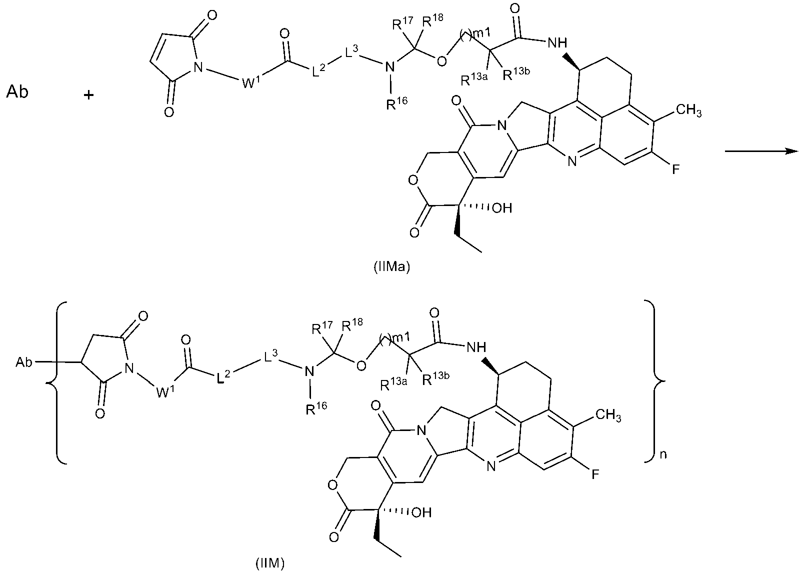 conducting a coupling reaction of reduced Ab with a compound represented by general formula (IIMa) or a salt thereof to give the antibody-drug conjugate represented by general formula (IIM) or the pharmaceutically acceptable salt thereof,
wherein:
Ab is the anti-CLDN6 antibody according to any one of the foregoing;
W¹, L², L³, R^{13a}, R^{13b}, R¹⁶ to R¹⁸, m1, and n are as defined in general formula (IIM).

In some embodiments, the present disclosure relates to a method for preparing an antibody-drug conjugate represented by general formula (IIIM-R) or a pharmaceutically acceptable salt thereof, comprising the following step: 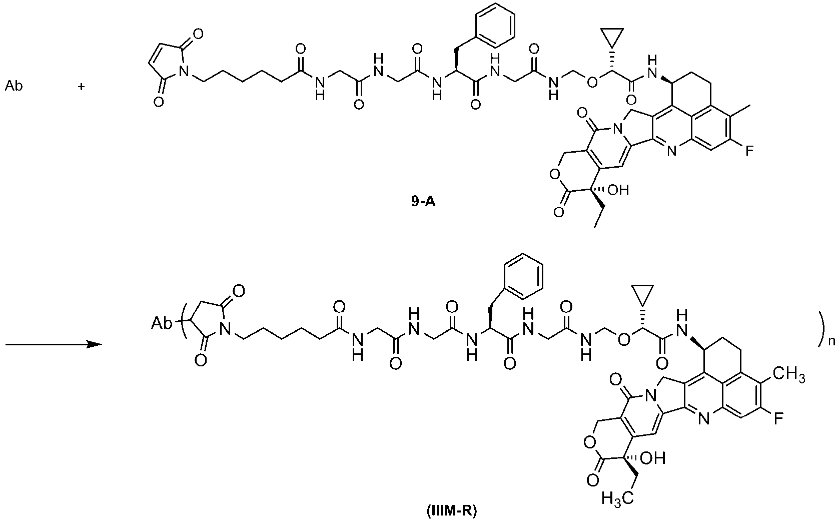 conducting a coupling reaction of reduced Ab with compound 9-A or a salt thereof to give the antibody-drug conjugate represented by general formula (IIIM-R) or the pharmaceutically acceptable salt thereof.

In some embodiments, for a preparation method for the antibody-drug conjugate represented by general formula (IIIM) or general formula (IIIM-S) or the pharmaceutically acceptable salt thereof of the present disclosure, reference may be made to the preparation method for the antibody-drug conjugate represented by general formula (IIIM-R) or the pharmaceutically acceptable salt thereof, and a corresponding chiral starting material is used in place of compound 9-A.

In another aspect, the present disclosure relates to a pharmaceutical composition comprising the anti-CLDN6 antibody according to any one of the foregoing or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof and one or more pharmaceutically acceptable carriers, diluents, or excipients.

In some embodiments, the present disclosure relates to a pharmaceutical composition comprising the anti-CLDN6 antibody according to any one of the foregoing and one or more pharmaceutically acceptable carriers, diluents, or excipients.

In some embodiments, the present disclosure relates to a pharmaceutical composition comprising the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof and one or more pharmaceutically acceptable carriers, diluents, or excipients. In some embodiments, the pharmaceutical composition comprises 0.01-99.99% of the anti-CLDN6 antibody or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof based on the total weight of the pharmaceutical composition.

In some embodiments, the pharmaceutical composition comprises 0.1-99.9% of the ligand-drug conjugate or the pharmaceutically acceptable salt thereof described above, the anti-CLDN6 antibody according to any one of the foregoing, or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof.

In some embodiments, the pharmaceutical composition comprises 0.5%-99.5% of the anti-CLDN6 antibody or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof.

In some embodiments, the pharmaceutical composition comprises 1%-99% of the anti-CLDN6 antibody or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof.

In some embodiments, the pharmaceutical composition comprises 2%-98% of the anti-CLDN6 antibody or the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof.

In some embodiments, the pharmaceutical composition comprises 0.01%-99.99% of the pharmaceutically acceptable diluents or excipients based on the total weight of the pharmaceutical composition.

In some embodiments, the pharmaceutical composition comprises 0.1%-99.9% of the pharmaceutically acceptable diluents or excipients.

In some embodiments, the pharmaceutical composition comprises 0.5%-99.5% of the pharmaceutically acceptable excipients.

In some embodiments, the pharmaceutical composition comprises 1%-99% of the pharmaceutically acceptable diluents or excipients.

In some embodiments, the pharmaceutical composition comprises 2%-98% of the pharmaceutically acceptable diluents or excipients.

In another aspect, the present disclosure relates to an isolated nucleic acid encoding the anti-CLDN6 antibody according to any one of the foregoing.

In another aspect, the present disclosure relates to a host cell comprising the isolated nucleic acid described above.

In another aspect, the present disclosure relates to a method for preventing or treating a tumor, the method comprising administering to a subject the anti-CLDN6 antibody according to any one of the foregoing, the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof, or the pharmaceutical composition according to the foregoing.

In some embodiments, the present disclosure relates to use in the manufacture of a medicament for preventing or treating a tumor, comprising administering to a subject the anti-CLDN6 antibody according to any one of the foregoing or the pharmaceutical composition according to the foregoing.

In some embodiments, the present disclosure relates to use in the manufacture of a medicament for preventing or treating a tumor, comprising administering to a subject the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof or the pharmaceutical composition according to the foregoing.

In another aspect, the present disclosure relates to the anti-CLDN6 antibody according to any one of the foregoing, the antibody-drug conjugate represented by general formula (I), general formula (II), general formula (IM), general formula (IIM), general formula (IIIM), general formula (IIIM-R), or general formula (IIIM-S) or the pharmaceutically acceptable salt thereof, or the pharmaceutical composition according to the foregoing for use as a medicament. In some embodiments, the medicament is used for preventing or treating a tumor.

In some embodiments, the present disclosure relates to an anti-CLDN6 antibody or a pharmaceutical composition thereof for use as a medicament. In some embodiments, the medicament is used for preventing or treating a tumor.

In some embodiments, the present disclosure relates to an antibody-drug conjugate represented by general formula (I), general formula (II), general formula (IM), general formula (IIM), general formula (IIIM), general formula (IIIM-R), or general formula (IIIM-S) or a pharmaceutically acceptable salt thereof or the pharmaceutical composition according to the foregoing for use as a medicament. In some embodiments, the medicament is used for preventing or treating a tumor.

The tumor described in the present disclosure is selected from the group consisting of ovarian cancer, lung cancer, endometrial cancer, gastric cancer, cervical cancer, testicular cancer, placental choriocarcinoma, renal cancer, urothelial carcinoma, colorectal cancer, prostate cancer, glioblastoma multiforme, brain tumor, pancreatic cancer, breast cancer, melanoma, liver cancer, bladder cancer, and esophageal cancer; preferably, the tumor is selected from the group consisting of ovarian cancer, lung cancer, endometrial cancer, and testicular cancer; more preferably, the tumor is ovarian cancer.

### BRIEF DESCRIPTION OF THE DRAWINGS

FIGs. 1 to 7 show the binding of antibodies to CLDN3 and CLDN4, wherein plots A show the binding to CLDN3, and plots B show the binding to CLDN4.
FIGs. 8A and 8B show evaluations of the efficacy of ADCs in a PA-1 *in vivo* model.
FIG. 8C shows an evaluation of the efficacy of ADCs in an OVCAR3 *in vivo* model.
FIG. 8D shows an evaluation of the efficacy of ADCs in an OV90 *in vivo* model.

### DETAILED DESCRIPTION

### Terminology

To facilitate the understanding of the present disclosure, certain technical and scientific terms are described below. Unless otherwise specifically defined herein, all technical and scientific terms used herein have the same meanings as commonly understood by those of ordinary skill in the art.

The singular forms "a", "an", and "the" used in the description and claims include plural references, unless the context clearly dictates otherwise.

Unless otherwise clearly stated in the context, throughout the description and the claims, the words "comprise", "have", "include", etc., should be construed in an inclusive sense as opposed to an exclusive or exhaustive sense.

The term "and/or" is meant to include the two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used in the present disclosure are as described in J. Biol. Chem., 243, p3558 (1968).

The term "amino acid" refers to naturally occurring and synthetic amino acids, as well as amino acid analogs and amino acid mimics that function similarly to naturally occurring amino acids. Naturally occurring amino acids are those that are encoded by the genetic code and those that are later modified, e.g., hydroxyproline, γ-carboxyglutamic acid, and O-phosphoserine. Amino acid analogs refer to compounds that have the same basic chemical structure (i.e., α carbons that bind to hydrogen, carboxyl, amino, and R groups) as naturally occurring amino acids, e.g., homoserine, norleucine, methionine sulfoxide, and methioninemethyl sulfonium. Such an analog has a modified R group (e.g., norleucine) or a modified peptide backbone, but retains a basic chemical structure that is the same as that of naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. The final construct can be achieved through any combination of substitutions, deletions, insertions, and modifications, so long as the final construct possesses the desired properties, such as reduced binding to Fc receptors. Amino acid sequence deletions and insertions include deletions and insertions at the amino-terminus and/or the carboxyl-terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacements with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, etc. It is contemplated that methods for altering amino acid side chain groups other than genetic engineering, such as chemical modifications, may also be used. Various names may be used herein to refer to the same amino acid mutation. Herein, the expression "position + amino acid residue" may be used to denote an amino acid residue at a specific position. For example, 82aR means that the amino acid residue at position 82a is R. S82aR means that the amino acid residue at position 82a (also referred to as 82A) is mutated from the original S to R.

The term "antibody" is used in the broadest sense and encompasses a variety of antibody structures, including but not limited to monoclonal antibodies, polyclonal antibodies; monospecific antibodies, multispecific antibodies (e.g., bispecific antibodies), full-length antibodies, and antibody fragments (or antigen-binding fragments, or antigen-binding moieties), so long as they exhibit the desired antigen-binding activity.

The term "bispecific antibody" refers to an antibody capable of specifically binding to two different antigens or at least two different antigenic epitopes of the same antigen (including an antibody or an antigen-binding fragment thereof, such as a single-chain antibody). Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of symmetry in their structures. Antibody fragment-based bispecific antibodies, e.g., Fab fragments lacking Fc fragments, are formed by combining two or more Fab fragments in one molecule. They have relatively low immunogenicity, are small in molecular weight, and have relatively high tumor tissue permeability. Typical antibody structures of this type include, for example, F(ab)₂, scFv-Fab, and (scFv)₂-Fab. IgG-like bispecific antibodies (e.g., comprising Fc fragments) are relatively large in molecular weight. The Fc fragments facilitate the purification of the antibodies and increase their solubility and stability, and the Fc portions may also bind to the receptor FcRn, increasing the serum half-life of the antibodies.

"Natural antibody" refers to a naturally occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain comprises one variable region (VH), also known as variable heavy domain or heavy chain variable region, followed by heavy chain constant regions. Generally, a natural IgG heavy chain constant region comprises three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL). The terms "full-length antibody", "intact antibody", and "whole antibody" are used herein interchangeably and refer to an antibody having a structure substantially similar to a natural antibody structure or comprising a heavy chain with an Fc region as defined herein. In a natural intact antibody, the light chain comprises a light chain variable region (VL) and a light chain constant region (CL), wherein the VL is located at the amino-terminus of the light chain, and the light chain constant region includes a κ chain and a λ chain; the heavy chain comprises a variable region (VH) and constant regions (CH1, CH2, and CH3), wherein the VH is located at the amino-terminus of the heavy chain, and the constant regions are located at the carboxyl-terminus, with the CH3 being the closest to the carboxyl-terminus of the polypeptide; the heavy chain may be of any isotype, including IgG (including subtypes IgG1, IgG2, IgG3, and IgG4), IgA (including subtypes IgA1 and IgA2), IgM, and IgE. The term "variable region" or "variable domain" of an antibody refers to the domain in an antibody heavy or light chain that is involved in the binding of the antibody to an antigen. Herein, the heavy chain variable region (VH) and light chain variable region (VL) of an antibody each comprise four conserved framework regions (FRs) and three complementarity determining regions (CDRs). The term "complementarity determining region" or "CDR" refers to the region in a variable domain that primarily contributes to antigen binding; "framework" or "FR" refers to variable domain residues other than CDR residues. A VH comprises 3 CDRs: HCDR1, HCDR2, and HCDR3, and a VL comprises 3 CDRs: LCDR1, LCDR2, and LCDR3. Each VH and VL is composed of three CDRs and four FRs arranged from the amino-terminus (also known as N-terminus) to the carboxyl-terminus (also known as C-terminus) in the following order: FR1, CDR1, FR2, CDR2, FR3, CDR3, and FR4.

The boundaries of the amino acid sequences of CDRs can be determined by a variety of well-known schemes, for example, the "Kabat" numbering scheme (see Kabat et al., (1991), Sequences of Proteins of Immunological Interest, 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), etc. The corresponding relationships between the various numbering schemes are well known to those skilled in the art. Illustratively, the relationships are shown in Table 1 below.

**Table 1. The relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure.

The term "antibody fragment" refers to a molecule different from an intact antibody, wherein the molecule comprises a moiety of an intact antibody, and the moiety binds to an antigen to which the intact antibody binds. Examples of antibody fragments include, but are not limited to, Fv, Fab, Fab', Fab'-SH, F(ab')2, single-domain antibodies, single-chain Fab (scFab), diabodies, linear antibodies, single-chain antibodies (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl-terminus. Suitable Fc regions used for the antibodies described herein include Fc regions of human IgG1, IgG2 (IgG2A and IgG2B), IgG3, and IgG4. In some embodiments, the boundaries of an Fc region may also vary; for example, the C-terminal lysine of the Fc region (residue 447 according to the EU numbering scheme) is deleted, or the C-terminal glycine and lysine of the Fc region (residues 446 and 447 according to the EU numbering scheme) are deleted. Unless otherwise stated, the numbering scheme for the Fc region is the EU numbering system, also known as the EU index.

The term "chimeric" antibody refers to an antibody in which a portion of the heavy and/or the light chain is derived from a particular source or species, while the rest of the heavy and/or the light chain is derived from a different source or species.

The term "humanized" antibody refers to an antibody that retains the reactivity of a non-human antibody while having relatively low immunogenicity in humans. For example, it can be achieved by keeping the non-human CDRs and replacing the rest of the antibody with its human counterparts (i.e., the constant regions and the framework region portions of the variable regions).

The terms "human antibody", "human-derived antibody", "fully human antibody", and "completely human antibody" are used interchangeably and mean antibodies whose variable regions and constant regions are human sequences. The term encompasses antibodies that are derived from human genes but have, for example, sequences that have been altered to reduce possible immunogenicity, increase affinity, and eliminate cysteines or glycosylation sites that may cause undesired folding. The term encompasses antibodies recombinantly produced in non-human cells (that may impart glycosylation without the characteristics of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to the internal binding affinity that reflects the 1:1 interaction between members of a binding pair (e.g., an antibody and an antigen). The affinity of molecule X for its ligand Y can be generally denoted by the dissociation constant (KD). Affinity can be measured by conventional methods known in the art, including those described herein.

As used herein, the term "kassoc" or "ka" refers to the association rate of a particular antibody-antigen interaction, and the term "kdis" or "kd" refers to the dissociation rate of a particular antibody-antigen interaction. The term "KD" refers to the dissociation constant, which is obtained from the ratio of kd to ka (i.e., kd/ka) and expressed as a molar concentration (M). The KD value of an antibody can be determined using a method well known in the art. For example, surface plasmon resonance is measured using a biosensing system such as a system (e.g., Biacore), or the affinity in a solution is measured by solution equilibrium titration (SET).

The term "surface plasmon resonance" refers to an optical phenomenon that enables analysis of real-time interactions by detecting changes in protein concentration within a biosensor matrix, for example, using a BIAcoreTM system (Biacore Life Sciences division of GE Healthcare, Piscataway, NJ).

The term "effector function" refers to biological activities that can be attributed to the Fc region of an antibody (either a natural sequence Fc region or an amino acid sequence variant Fc region) and vary with the antibody isotype. Examples of antibody effector functions include, but are not limited to: Clq binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a substantially homogeneous population of antibodies; that is, the amino acid sequences of the antibody molecules in the population are identical, except for natural mutations that may be present in small quantities. In contrast, a polyclonal antibody formulation generally comprises several different antibodies comprising different amino acid sequences in their variable domains, which are generally specific for different epitopes. "Monoclonal" refers to the characteristics of an antibody obtained from a substantially homogeneous antibody population and should not be construed as requiring any particular method to produce the antibody. In some embodiments, the antibody provided by the present disclosure is a monoclonal antibody. The term "antigen" refers to a molecule or a portion of the molecule that can be bound by a selective binding agent, such as an antigen-binding protein (including, for example, an antibody), and can also be used in an animal to produce an antibody capable of binding to the antigen. An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of an antigen of a protein nature). The difference between the conformational epitope and the linear epitope is that in the presence of a denaturing solvent, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (see Prot. Sci. 9 (2000) 487-496), and cross-blocking.

The term "capable of specifically binding", "specifically bind", or "bind" means that an antibody is capable of binding to a certain antigen or an epitope of the antigen with higher affinity than to other antigens or epitopes. Generally, an antibody binds to an antigen or an epitope in the antigen with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD of the binding of an antibody to an antigen is 10% or less (e.g., 1%) of the KD of the binding of the antibody to a non-specific antigen (e.g., BSA or casein). KD may be measured using known methods, for example, by a BIACORE^{®} surface plasmon resonance assay. However, an antibody that specifically binds to an antigen or an epitope in the antigen may have cross-reactivity with other related antigens, for example, with corresponding antigens from other species (homologous) (e.g., humans or monkeys, such as Macaca fascicularis (cynomolgus, cyno), Pan troglodytes (chimpanzee, chimp), or Callithrix jacchus (commonmarmoset, marmoset)).

The term "antibody-dependent cellular cytotoxicity", "antibody-dependent cell-mediated cytotoxicity", or "ADCC" is a mechanism for inducing cell death. The mechanism relies on the interaction of antibody-coated target cells with effector cells with lytic activity, such as natural killer cells (NK), monocytes, macrophages, and neutrophils, via an Fcy receptor (FcyR) expressed on the effector cells. For example, NK cells express FcγRIIIa, while monocytes express FcyRI, FcyRII, and FcγRIIIa. The ADCC activity of the antibodies provided herein can be assessed by *in vitro* assays, using cells expressing the antigen as target cells and NK cells as effector cells. Cell lysis is detected based on a label (e.g., radioactive substrate, fluorescent dye, or natural intracellular protein) released from lysed cells.

The term "antibody-dependent cellular phagocytosis (ADCP)" refers to a mechanism in which antibody-coated target cells are eliminated by the internalization effect of phagocytic cells (such as macrophages or dendritic cells).

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. The activation of the complement may also result in the deposition of complement components on the surface of the target cell, and these complement components promote CDC by binding to complement receptors on leukocytes (e.g., CR3).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring an alignment, including any algorithm required to achieve the maximum alignment on the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomously replicating in a host cell into which they are introduced (e.g., bacterial vectors having a bacterial origin of replication and episomal mammalian vectors). Other vectors (e.g., non-episomal mammalian vectors) can be integrated into the genome of a host cell after introduction into the host cell, so that they are replicated along with the host genome. The term "expression vector" or "expression construct" refers to a vector with which a host cell can be transformed, and the vector comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including the progeny of such cells. Host cells include "transformants" and "transformed cells", which include primary transformed cells and the progeny derived therefrom, regardless of the number of passages. The progeny may not be completely identical to the parental cells in terms of nucleic acid contents and may contain mutations. Mutant progeny that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia, Saccharomyces cerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens, and Neurospora crassa. Pichia, any Saccharomyces, Hansenula polymorpha, any Kluyveromyces, Candida albicans, any Aspergillus, Trichoderma reesei, Chrysosporium lucknowense, any Fusarium, Yarrowia lipolytica, and Neurospora crassa.

"Cell", "cell line", and "cell culture" are used interchangeably, and all such designations include progeny. Thus, the words "transformant" and "transformed cell" include the primary subject cell and cultures derived therefrom, regardless of the number of passages.

It will also be appreciated that not all progeny have precisely identical DNA contents due to deliberate or inadvertent mutations. Variant progeny that have the same function or biological activity as the original transformed cell are included.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). Preferably, the alkyl is an alkyl group having 1 to 12 carbon atoms (i.e., C₁₋₁₂ alkyl); more preferably, the alkyl is an alkyl group having 1 to 6 carbon atoms (i.e., C₁₋₆ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof, etc. Alkyl may be substituted or unsubstituted, and when it is substituted, it may be substituted at any accessible point of attachment, and the substituent is preferably selected from the group consisting of one or more of deuterium (D), halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include methoxy, ethoxy, propoxy, butoxy, etc. Alkoxy may be substituted or unsubstituted, and when it is substituted, it may be substituted at any accessible point of attachment, and the substituent is preferably selected from the group consisting of one or more of deuterium (D), halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). Preferably, the cycloalkyl is a cycloalkyl group having 3 to 12 ring atoms (i.e.,3- to 12-membered cycloalkyl); more preferably, the cycloalkyl is a cycloalkyl group having 3 to 8 ring atoms (i.e., 3- to 8-membered cycloalkyl); most preferably, the cycloalkyl is a cycloalkyl group having 3 to 6 ring atoms (i.e., 3- to 6-membered cycloalkyl).

Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The polycyclic cycloalkyl includes spirocycloalkyl, fused cycloalkyl, and bridged cycloalkyl.

The term "spirocycloalkyl" refers to a polycyclic system in which a carbon atom (referred to as a spiro atom) is shared between rings, and it may contain in the rings one or more double bonds, or it may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), provided that at least one all-carbon ring is contained and the point of attachment is on the all-carbon ring; it has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). Preferably, the spirocycloalkyl is a spirocycloalkyl group having 6 to 14 ring atoms (i.e., 6- to 14-membered spirocycloalkyl); more preferably, the spirocycloalkyl is a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl); monospirocycloalkyl or bispirocycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl is more preferred. Non-limiting examples include: 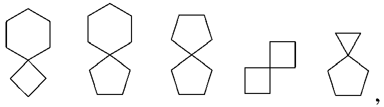 wherein the point of attachment may be at any position; 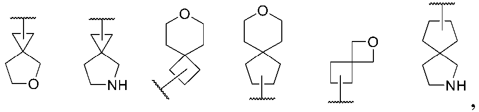 etc.

The term "fused cycloalkyl" refers to a polycyclic system in which two adjacent carbon atoms are shared between rings, and it is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group, and it may contain one or more double bonds in the rings and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). Preferably, the fused cycloalkyl is a fused cycloalkyl group having 6 to 14 ring atoms (i.e., 6- to 14-membered fused cycloalkyl); more preferably, the fused cycloalkyl is a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl); bicyclic fused cycloalkyl or tricyclic fused cycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl is more preferred. Non-limiting examples include: 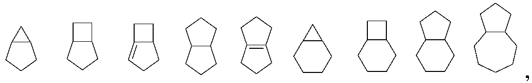 wherein the point of attachment 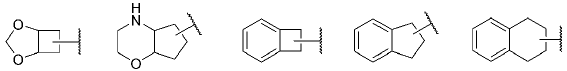 may be at any position; 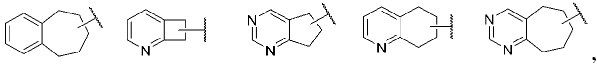 etc.

The term "bridged cycloalkyl" refers to an all-carbon polycyclic system in which two carbon atoms that are not directly connected are shared between rings, and it may contain one or more double bonds in the rings and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). Preferably, the bridged cycloalkyl is a bridged cycloalkyl group having 6 to 14 carbon atoms (i.e., 6- to 14-membered bridged cycloalkyl); more preferably, the bridged cycloalkyl is a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl); bicyclic bridged cycloalkyl or tricyclic bridged cycloalkyl is preferred. Non-limiting examples include: 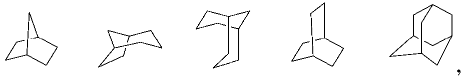 wherein the point of attachment may be at any position.

Cycloalkyl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). Preferably, the heterocyclyl is a heterocyclyl group having 3 to 12 ring atoms (i.e., 3- to 12-membered heterocyclyl), e.g., 4- to 12-membered heterocyclyl containing at least one nitrogen atom; further preferably, the heterocyclyl is a heterocyclyl group having 3 to 8 ring atoms (i.e., 3- to 8-membered heterocyclyl); more preferably, the heterocyclyl is a heterocyclyl group having 3 to 6 ring atoms (i.e., 3- to 6-membered heterocyclyl); most preferably, the heterocyclyl is a heterocyclyl group having 5 or 6 ring atoms (i.e., 5- or 6-membered heterocyclyl). Non-limiting examples of the monocyclic heterocyclyl include pyrrolidinyl, tetrahydropyranyl, 1,2,3,6-tetrahydropyridyl, piperidinyl, piperazinyl, morpholinyl, thiomorpholinyl, homopiperazinyl, etc.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), provided that at least one monocyclic heterocyclyl group is contained and the point of attachment is on the monocyclic heterocyclyl group; it has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). Preferably, the spiroheterocyclyl is a spiroheterocyclyl group having 6 to 14 ring atoms (i.e., 6- to 14-membered spiroheterocyclyl); more preferably, the spiroheterocyclyl is a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl); monospiroheterocyclyl or bispiroheterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl is more preferred. Non-limiting examples include: 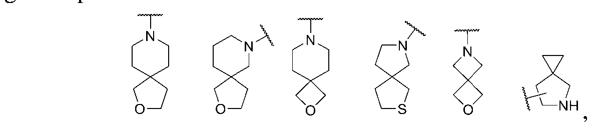 etc.

The term "fused heterocyclyl" refers to a polycyclic heterocyclic system in which two adjacent atoms are shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded); it is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group, and it has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). Preferably, the fused heterocyclyl is a fused heterocyclyl group having 6 to 14 ring atoms (i.e., 6- to 14-membered fused heterocyclyl); more preferably, the fused heterocyclyl is a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl); bicyclic fused heterocyclyl or tricyclic fused heterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl is more preferred. Non-limiting examples include: 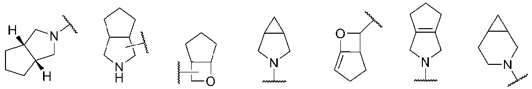 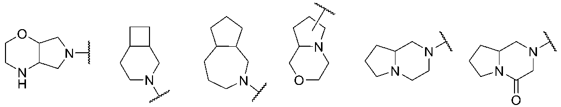 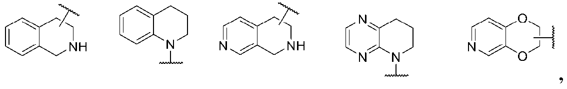 etc.

The term "bridged heterocyclyl" refers to a polycyclic heterocyclic system in which two atoms that are not directly connected are shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded); it has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). Preferably, the bridged heterocyclyl is a bridged heterocyclyl group having 6 to 14 ring atoms (i.e., 6- to 14-membered bridged heterocyclyl); more preferably, the bridged heterocyclyl is a bridged heterocyclyl group having 7 to 10 ring atoms (i.e., 7- to 10-membered bridged heterocyclyl). According to the number of constituent rings, the bridged heterocyclyl can be divided into bicyclic bridged heterocyclyl and polycyclic bridged heterocyclyl (e.g., tricyclic bridged heterocyclyl and tetracyclic bridged heterocyclyl); bicyclic bridged heterocyclyl or tricyclic bridged heterocyclyl is preferred. Non-limiting examples include: 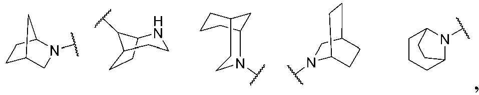 etc.

Heterocyclyl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, and it has 6 to 14 (e.g., 6, 7, 8, 9, 10, 11, 12, 13, or 14) ring atoms (i.e., 6- to 14-membered aryl). Preferably, the aryl is an aryl group having 6 to 10 ring atoms (i.e., 6- to 10-membered aryl). The monocyclic aryl is, for example, phenyl. Non-limiting examples of the polycyclic aryl include naphthyl, anthryl, phenanthryl, etc. The polycyclic aryl also includes those formed by fusing a phenyl group with one or more of a heterocyclyl group or a cycloalkyl group, or fusing a naphthyl group with one or more of a heterocyclyl group or a cycloalkyl group, wherein the point of attachment is on the phenyl group or the naphthyl group, and in the circumstances, the number of ring atoms continues to represent the number of ring atoms in the polycyclic aromatic ring system; non-limiting examples include: 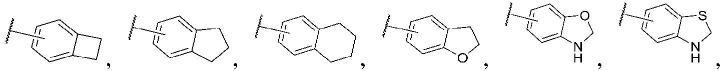 etc.

Aryl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 5 to 14 (e.g., 5, 6, 7, 8, 9, 10, 11, 12, 13, or 14) ring atoms (i.e., 5- to 14-membered heteroaryl). Preferably, the heteroaryl is a heteroaryl group having 5 to 10 ring atoms (i.e., 5- to 10-membered heteroaryl); more preferably, the heteroaryl is a monocyclic heteroaryl group having 5 or 6 ring atoms (i.e., 5- or 6-membered monocyclic heteroaryl) or a bicyclic heteroaryl group having 8 to 10 ring atoms (i.e., 8- to 10-membered bicyclic heteroaryl); most preferably, the heteroaryl is a 5- or 6-membered monocyclic heteroaryl group containing in the ring 1, 2, or 3 heteroatoms selected from the group consisting of nitrogen, oxygen, and sulfur or an 8- to 10-membered bicyclic heteroaryl group containing in the ring 1, 2, or 3 heteroatoms selected from the group consisting of nitrogen, oxygen, and sulfur.

Non-limiting examples of the monocyclic heteroaryl include furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridyl, pyrimidinyl, pyridonyl, N-alkylpyridinone (e.g., 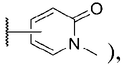 pyrazinyl, pyridazinyl, etc.

Non-limiting examples of the polycyclic heteroaryl include indolyl, indazolyl, quinolyl, isoquinolyl, quinoxalinyl, phthalazinyl, benzimidazolyl, benzothienyl, quinazolinyl, benzothiazolyl, carbazolyl, etc. The polycyclic heteroaryl also includes those formed by fusing a monocyclic heteroaryl group with one or more aryl groups, wherein the point of attachment is on an aromatic ring, and in the circumstances, the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances, the number of ring atoms continues to represent the number of ring atoms in the polycyclic heteroaromatic ring system. Non-limiting examples include: 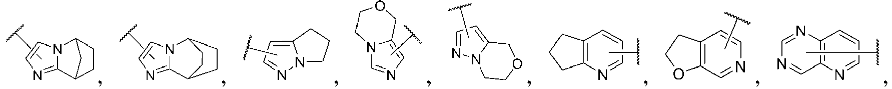 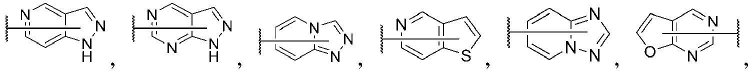 etc.

Heteroaryl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived by removing one hydrogen atom from a ring atom of the parental structure, or residues derived by removing two hydrogen atoms from the same ring atom or two different ring atoms of the parental structure, i.e., "divalent cycloalkyl", "divalent heterocyclyl", "arylene", and "heteroarylene".

In the chemical structures of the compounds of the present disclosure, the bond " 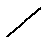 " indicates that the configuration is not specified; that is, if there are chiral isomers in the chemical structures, the bond " 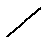 " may be " 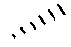 " " or " " 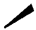 ", or includes both the configurations " 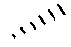 " and " 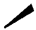 " simultaneously.

The compounds of the present disclosure include all suitable isotopic derivatives of the compounds thereof. The term "isotopic derivative" refers to a compound in which at least one atom is replaced with an atom having the same atomic number but having a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I; deuterium is preferred.

Compared to non-deuterated drugs, deuterated drugs have the advantages of reduced toxic and side effects, increased drug stability, enhanced efficacy, prolonged biological half-lives, etc. All isotopic variations of the compounds of the present disclosure, whether radioactive or not, are intended to be included within the scope of the present disclosure. Each available hydrogen atom attached to a carbon atom may be independently replaced with a deuterium atom, wherein the replacement with deuterium may be partial or complete. The partial replacement with deuterium refers to the replacement of at least one hydrogen atom with at least one deuterium atom.

"Optional" or "optionally" means that the event or circumstance subsequently described may, but does not necessarily, occur; this description includes the instance where the event or circumstance occurs or does not occur.

The term "pharmaceutical composition" refers to a mixture comprising one or more of the antibody-drug conjugates or the pharmaceutically acceptable salts thereof described herein and other chemical components; the other components are, for example, physiological/pharmaceutically acceptable carriers and excipients.

The term "pharmaceutically acceptable carriers, diluents, or excipients" refers to ingredients of a pharmaceutical formulation that are different from the active ingredient and are not toxic to subjects. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless otherwise indicated, the terms "patient" and "subject" are used interchangeably herein. In certain embodiments, an individual or subject is a human.

"Administer" or "give", when applied to animals, humans, experimental subjects, cells, tissues, organs, or biological fluids, refers to contact of an exogenous drug, a therapeutic agent, a diagnostic agent, or a composition with the animals, humans, subjects, cells, tissues, organs, or biological fluids.

The term "sample" refers to collected substances resembling fluids, cells, or tissues isolated from a subject, as well as fluids, cells, or tissues that are present in the subject. Exemplary samples are biological fluids (e.g., blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); and lavage fluids), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of treatment include, but are not limited to, preventing the occurrence or recurrence of a disease, palliating symptoms, palliating/reducing any direct or indirect pathological consequences of the disease, preventing metastasis, decreasing the rate of disease progression, ameliorating or palliating the disease state, and regression or improved prognosis. In some embodiments, an antibody of the present disclosure is used to delay the development of, or slow the progression of, a disease.

"Effective amount" is generally an amount sufficient to reduce the severity and/or frequency of symptoms, eliminate these symptoms and/or underlying causes, prevent the occurrence of symptoms and/or their underlying causes, and/or improve or ameliorate damage caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and body weight of the individual, and the ability of a therapeutic agent or a combination of therapeutic agents to induce the desired response in the individual. Exemplary indicators of an effective therapeutic agent or combination of therapeutic agents include, for example, improved patient health.

"Pharmaceutically acceptable salt" refers to a salt formed by reacting an active pharmaceutical ingredient (e.g., a cytotoxic agent) with an appropriate acid or base. Such salts retain the pharmacological activity of the active pharmaceutical ingredient while having better physicochemical properties, such as improved drug solubility, stability, and bioavailability. Upon ingestion by a subject, such salts can release the active pharmaceutical ingredient under physiological conditions to produce a therapeutic effect. Examples of pharmaceutically acceptable salts include, but are not limited to, sodium salts, potassium salts, calcium salts, magnesium salts, ammonium salts, hydrochlorides, sulfates, phosphates, acetates, citrates, tartrates, and maleates. "Remodeled glycan chain" refers to a glycan chain obtained by modifying or engineering the original glycan chain structure on a biomolecule (e.g., an antibody). In organisms, antibodies undergo glycosylation modifications. Such glycan chain structures play a critical role in the folding, stability, localization, activity, interaction with other molecules, etc., of proteins. For a remodeled glycan chain, the original glycan chain structure is altered by chemical or biological technical means to provide new properties.

### Exemplary Antibody-Drug Conjugate or Pharmaceutically Acceptable Salt Thereof

An example of the present disclosure relates to an anti-CLDN6 antibody comprising a heavy chain variable region and a light chain variable region, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 26, a LCDR2 that comprises the amino acid sequence of SEQ ID NO: 24, and a LCDR3 that comprises the amino acid sequence of SEQ ID NO: 19.

In some embodiments, provided is the anti-CLDN6 antibody according to the foregoing, wherein the amino acid sequence of the heavy chain variable region is set forth in SEQ ID NO: 12, and the amino acid sequence of the light chain variable region is set forth in SEQ ID NO: 32.

In some embodiments, provided is the anti-CLDN6 antibody according to the foregoing, wherein the amino acid sequence of the heavy chain is set forth in SEQ ID NO: 22, and the amino acid sequence of the light chain is set forth in SEQ ID NO: 38.

In another aspect, an example of the present disclosure relates to an anti-CLDN6 antibody comprising a heavy chain variable region and a light chain variable region, wherein the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region comprises a LCDR1 that comprises the amino acid sequence of SEQ ID NO: 25, a LCDR2 that comprises the amino acid sequence of SEQ ID NO: 24, and a LCDR3 that comprises the amino acid sequence of SEQ ID NO: 19.

In some embodiments, provided is the anti-CLDN6 antibody according to the foregoing, wherein the amino acid sequence of the heavy chain variable region is set forth in SEQ ID NO: 12, and the amino acid sequence of the light chain variable region is set forth in SEQ ID NO: 31.

In some embodiments, provided is the anti-CLDN6 antibody according to the foregoing, wherein the amino acid sequence of the heavy chain is set forth in SEQ ID NO: 22, and the amino acid sequence of the light chain is set forth in SEQ ID NO: 37.

In another aspect, the present disclosure relates to an antibody-drug conjugate or a pharmaceutically acceptable salt thereof, which is represented by general formula (II): 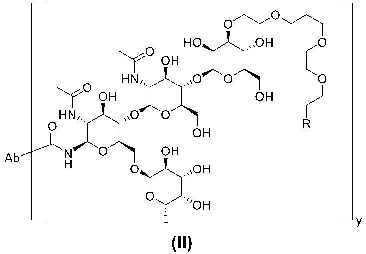 wherein: 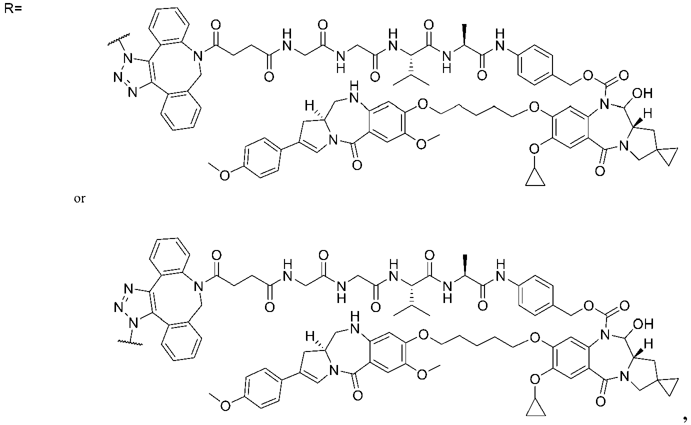 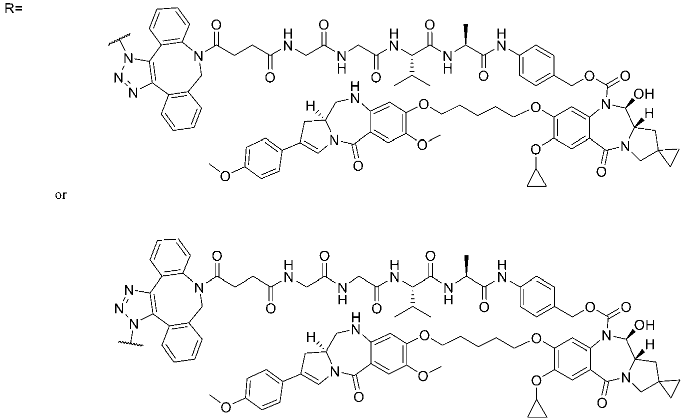 or 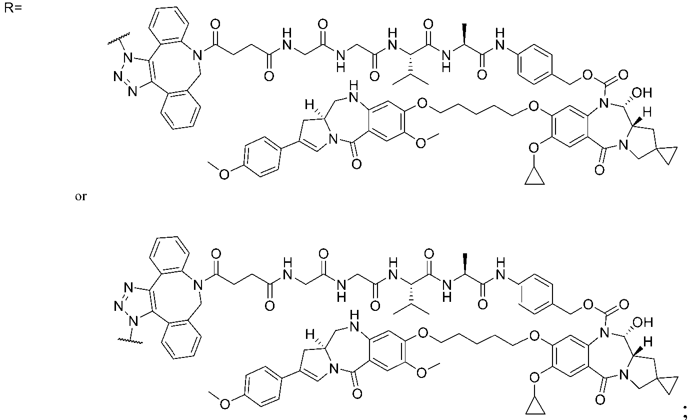
Ab is the anti-CLDN6 antibody according to any one of the foregoing;
y is 1 to 2; preferably, y is 2.

In some embodiments, the antibody-drug conjugate or the pharmaceutically acceptable salt thereof according to the foregoing is represented by general formula (II): 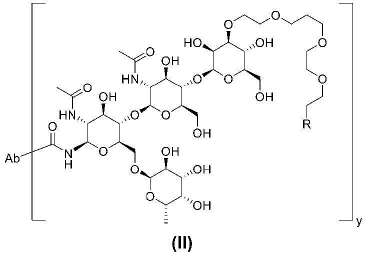 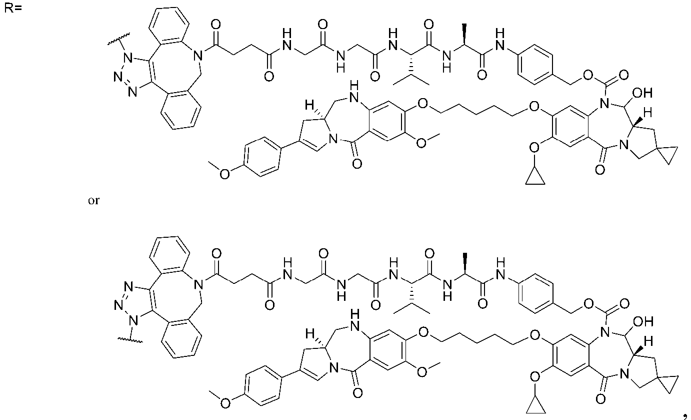 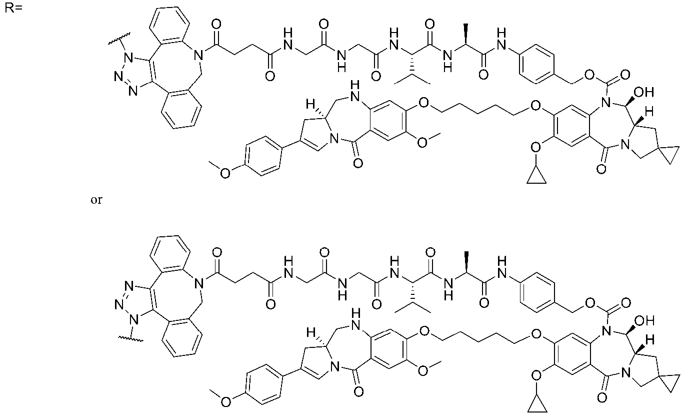 or 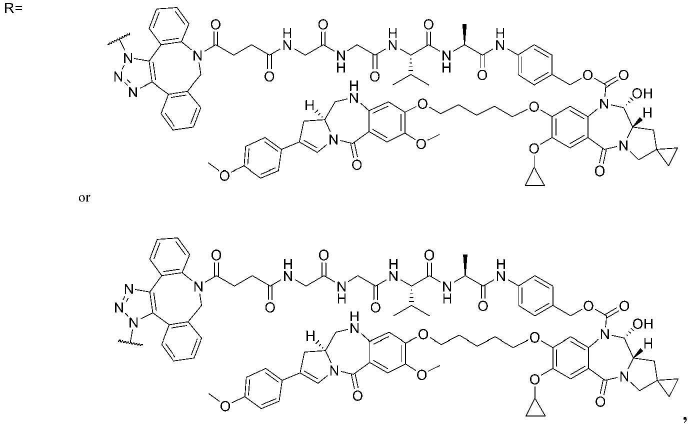 wherein:
the heavy chain variable region of Ab comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region of Ab comprises a LCDR1 that comprises the amino acid sequence of SEQ ID NO: 26, a LCDR2 that comprises the amino acid sequence of SEQ ID NO: 24, and a LCDR3 that comprises the amino acid sequence of SEQ ID NO: 19;
preferably, the amino acid sequence of the heavy chain variable region of Ab is set forth in SEQ ID NO: 12, and the amino acid sequence of the light chain variable region of Ab is set forth in SEQ ID NO: 32;
more preferably, the amino acid sequence of the heavy chain of Ab is set forth in SEQ ID NO: 22, and the amino acid sequence of the light chain is set forth in SEQ ID NO: 38; and y is 2.

In some embodiments, the antibody-drug conjugate or the pharmaceutically acceptable salt thereof according to any one of the foregoing is represented by general formula (II): 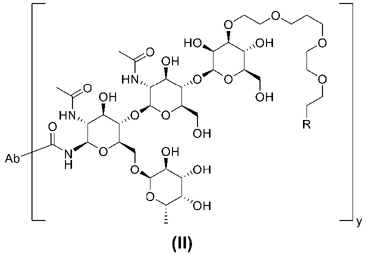 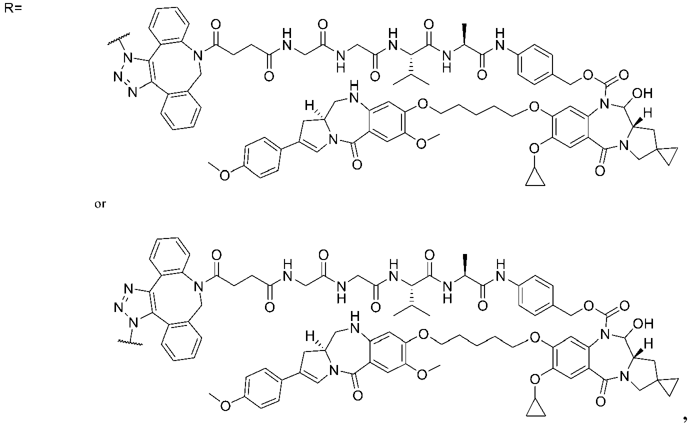 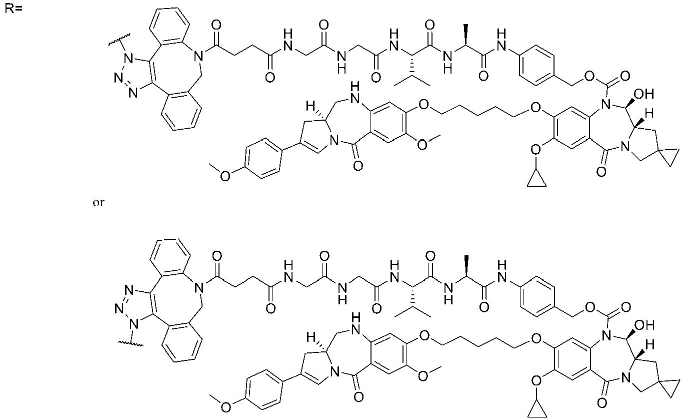 or 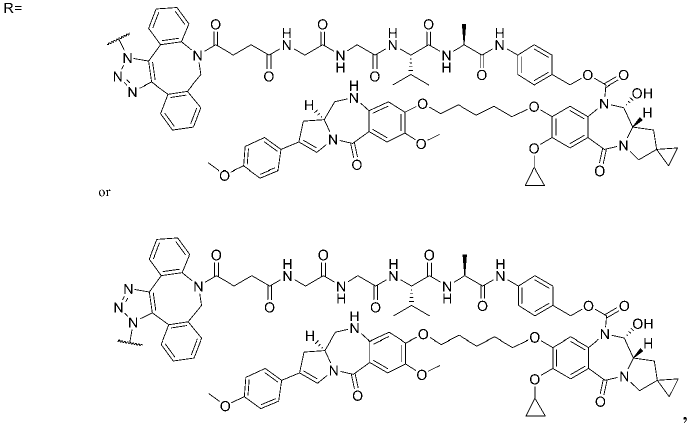 wherein:
the heavy chain variable region of Ab comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region of Ab comprises a LCDR1 that comprises the amino acid sequence of SEQ ID NO: 25, a LCDR2 that comprises the amino acid sequence of SEQ ID NO: 24, and a LCDR3 that comprises the amino acid sequence of SEQ ID NO: 19;
preferably, the amino acid sequence of the heavy chain variable region of Ab is set forth in SEQ ID NO: 12, and the amino acid sequence of the light chain variable region of Ab is set forth in SEQ ID NO: 31;
more preferably, the amino acid sequence of the heavy chain of Ab is set forth in SEQ ID NO: 22, and the amino acid sequence of the light chain is set forth in SEQ ID NO: 37; and y is 2.

In another aspect, the present disclosure relates to an antibody-drug conjugate or a pharmaceutically acceptable salt thereof, which is represented by general formula (IIIM), general formula (IIIM-R), or general formula (IIIM-S): 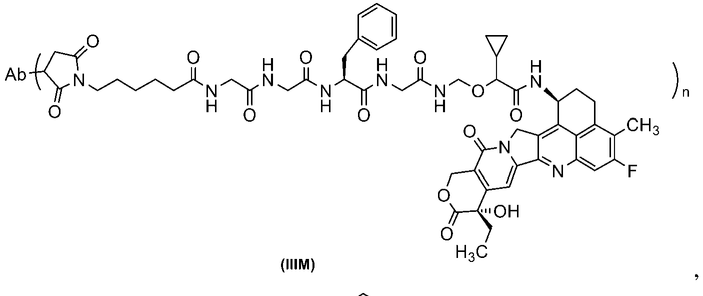 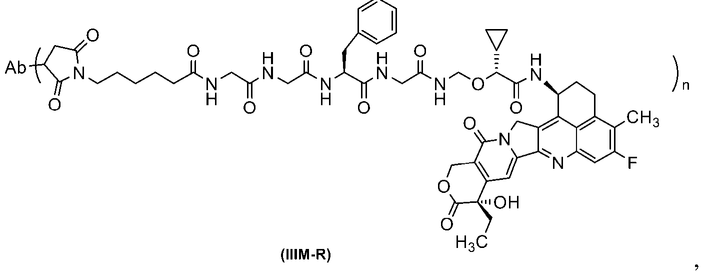 or 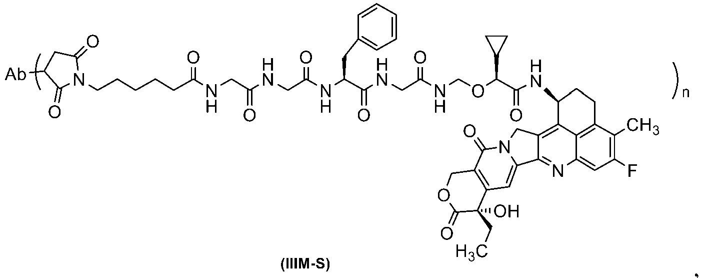 wherein Ab is the anti-CLDN6 antibody according to any one of the foregoing;
n is 4 to 8; preferably, n is 8.

In some embodiments, the antibody-drug conjugate or the pharmaceutically acceptable salt thereof according to the foregoing is represented by general formula (IIIM-R): 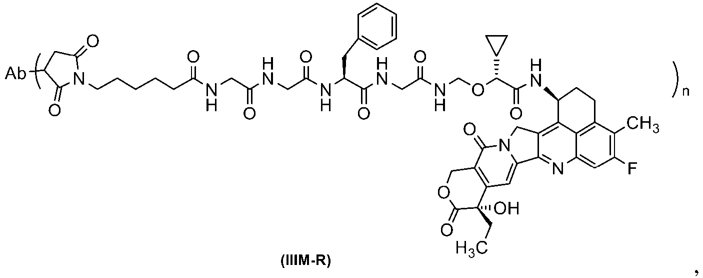 wherein Ab is the anti-CLDN6 antibody according to any one of the foregoing;
n is 4 to 8; preferably, n is 8.

In some embodiments, the antibody-drug conjugate or the pharmaceutically acceptable salt thereof according to the foregoing is represented by general formula (IIIM-R): 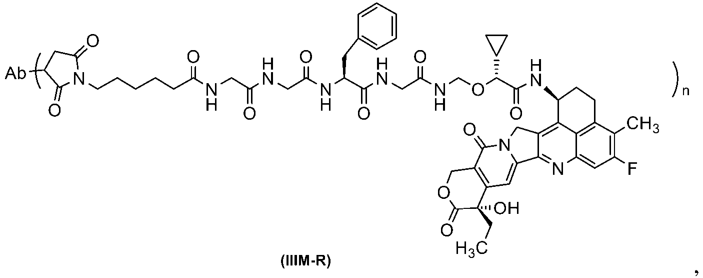 wherein:
the heavy chain variable region of Ab comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region of Ab comprises a LCDR1 that comprises the amino acid sequence of SEQ ID NO: 26, a LCDR2 that comprises the amino acid sequence of SEQ ID NO: 24, and a LCDR3 that comprises the amino acid sequence of SEQ ID NO: 19;
preferably, the amino acid sequence of the heavy chain variable region of Ab is set forth in SEQ ID NO: 12, and the amino acid sequence of the light chain variable region of Ab is set forth in SEQ ID NO: 32;
more preferably, the amino acid sequence of the heavy chain of Ab is set forth in SEQ ID NO: 22, and the amino acid sequence of the light chain is set forth in SEQ ID NO: 38; and n is 8.

In some embodiments, the antibody-drug conjugate or the pharmaceutically acceptable salt thereof according to the foregoing is represented by general formula (IIIM-R): 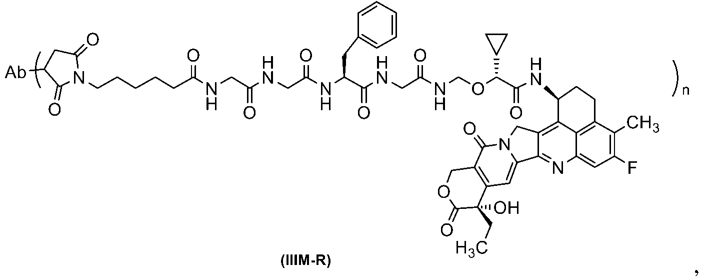 wherein:
the heavy chain variable region of Ab comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and the light chain variable region of Ab comprises a LCDR1 that comprises the amino acid sequence of SEQ ID NO: 25, a LCDR2 that comprises the amino acid sequence of SEQ ID NO: 24, and a LCDR3 that comprises the amino acid sequence of SEQ ID NO: 19;
preferably, the amino acid sequence of the heavy chain variable region of Ab is set forth in SEQ ID NO: 12, and the amino acid sequence of the light chain variable region of Ab is set forth in SEQ ID NO: 31;
more preferably, the amino acid sequence of the heavy chain of Ab is set forth in SEQ ID NO: 22, and the amino acid sequence of the light chain is set forth in SEQ ID NO: 37; and n is 8.

### Examples

The present disclosure is further described below with reference to examples and test examples, which, however, do not limit the scope of the present disclosure. In the examples or test examples of the present disclosure, the experimental methods whose specific conditions are not specified were generally performed under conventional conditions, such as those described in Antibodies: A Laboratory Manual and Molecular Cloning: A Laboratory Manual by Cold Spring Harbor Laboratory, or under conditions recommended by the manufacturer of the starting material or commercial product. The reagents whose specific sources are not specified were commercially available.

### I. Antibody Preparation

### Example 1: Preparation of Cell Strains Stably Transfected with CLDN6

CLDN6 and CLDN9 genes of different species and the human CLDN3 and CLDN4 genes were transfected into human embryonic kidney cells 293T to construct cell strains expressing CLDN6 and CLDN9 of different species and cell strains expressing human CLDN3 and CLDN4 for subsequent antibody screening and identification. The amino acid sequences of the related proteins are shown below:
> **Human CLDN6 full-length protein (Uniprot: P56747):** 
MASAGMQILGVVLTLLGWVNGLVSCALPMWKVTAFIGNSIVVAQVVWEGLW
MSCVVQSTGQMQCKVYDSLLALPQDLQAARALCVIALLVALFGLLVYLAGAK
CTTCVEEKDSKARLVLTSGIVFVISGVLTLIPVCWTAHAIIRDFYNPLVAEAQKR
ELGASLYLGWAASGLLLLGGGLLCCTCPSGGSQGPSHYMARYSTSAPAISRGPS
EYPTKNYV
SEQ ID NO: 1
> **Cynomolgus monkey CLDN6 full-length protein (Uniprot: G7Q0B0):** 
MASAGMQILGVVLTLLGWVNGLVSCALPMWKVTAFIGNSIVVAQVVWEGLW
MSCVVQSTGQMQCKVYDSLLALPQDLQAARALCVIALLVALFGLLVYLAGAK
CTTCVEEKDSKARLVLTSGIVFVISGVLTLIPVCWTAHAIIRDFYNPLVAEAQKRE
LGASLYLGWAASGLLLLGGGLLCCTCPSGGSRGPSHYMARYSTSAPAISRGPSE
YPTKNYV
SEQ ID NO: 2
**> Human CLDN9 full-length protein (Uniprot: O95484):** 
MASTGLELLGMTLAVLGWLGTLVSCALPLWKVTAFIGNSIVVAQVVWEGLW
MSCVVQSTGQMQCKVYDSLLALPQDLQAARALCVIALLLALLGLLVAITGAQC
TTCVEDEGAKARIVLTAGVILLLAGILVLIPVCWTAHAIIQDFYNPLVAEALKRE
 
LGASLYLGWAAAALLMLGGGLLCCTCPPPQVERPRGPRLGYSIPSRSGASGLD
KRDYV
SEQ ID NO: 3
**> Cynomolgus monkey CLDN9 full-length protein (Uniprot: A0A2K5UCC0):** 
MASTGLELLGMTLAVLGWLGTLVSCALPLWKVTAFIGNSIVVAQVVWEGLWM
SCVVQSTGQMQCKVYDSLLALPQDLQAARALCVIALLLALLGLLLAITGAQCT
TCVEDEGAKARIMLTAGVILLLAGILVLIPVCWTAHAIIQDFYNPLVAEALKRELG
ASLYLGWAAAALLMLGGGLLCCTCPPPQVERPRGPRLGYSIPSRSGASGLDKRD
YV
SEQ ID NO: 4
**> Human CLDN6 full-length protein variant (Uniprot: P56747 Variant p.Ile143Val):** 
MASAGMQILGVVLTLLGWVNGLVSCALPMWKVTAFIGNSIVVAQVVWEGLW
MSCVVQSTGQMQCKVYDSLLALPQDLQAARALCVIALLVALFGLLVYLAGAK
CTTCVEEKDSKARLVLTSGIVFVISGVLTLIPVCWTAHAVIRDFYNPLVAEAQK
RELGASLYLGWAASGLLLLGGGLLCCTCPSGGSQGPSHYMARYSTSAPAISRGP
SEYPTKNYV
SEQ ID NO: 5
**> Mouse CLDN6 full-length protein (Uniprot: Q9Z262):** 
MASTGLQILGIVLTLLGWVNALVSCALPMWKVTAFIGNSIVVAQMVWEGLWM
SCVVQSTGQMQCKVYDSLLALPQDLQAARALCVVTLLIVLLGLLVYLAGAKC
TTCVEDRNSKSRLVLISGIIFVISGVLTLIPVCWTAHSIIQDFYNPLVADAQKREL
GASLYLGWAASGLLLLGGGLLCCACSSGGTQGPRHYMACYSTSVPHSRGPSEY
PTKNYV
SEQ ID NO: 6
**> Mouse CLDN9 full-length protein (Uniprot: Q9Z0S7):** 
MASTGLELLGMTLAVLGWLGTLVSCALPLWKVTAFIGNSIVVAQVVWEGLW
MSCVVQSTGQMQCKVYDSLLALPQDLQAARALCVVALLLALLGLLVAITGAQ
CTTCVEDEGAKARIVLTAGVLLLLSGILVLIPVCWTAHAIIQDFYNPLVAEALKR
ELGASLYLGWAAAALLMLGGGLLCCTCPPSHFERPRGPRLGYSIPSRSGASGLD
KRDYV
SEQ ID NO: 7
**> Rat CLDN6 full-length protein (Uniprot: B4F7F0):** 
MASTGLQILGIVLTLLGWVNALVSCALPMWKVTAFIGNSIVVAQMVWEGLWM
SCVVQSTGQMQCKVYDSLLALPQDLQAARALCVITLLIVLLGLLLYLAGAKCT
TCVEDKNSKSRLVLISGVIFVISGVLTLIPICWTAHAIIQDFYNPLVADAQKRELG
ASLYLGWAASGLLLIGGGLLCCACSSGGTQGPSHYVARYSSPVPHSRGPSEYPS
KNYV
SEQ ID NO: 8
**> Rat CLDN9 full-length protein (Uniprot: Q5PPJ3):** 
MASTGLELLGMTLAVLGWLGTLVSCALPLWKVTAFIGNSIVVAQVVWEGLW
MSCVVQSTGQMQCKVYDSLLALPQDLQAARALCVVALLLALLGLLVAITGAQ
CTTCVEDEGAKARIVLTAGVLLLLSGILVLIPVCWTAHAIIQDFYNPLVAEALKR
ELGASLYLGWAAAALLMLGGGLLCCTCPPSHFERPRGPRLGYSIPSRSGASGLD
KRDYV
SEQ ID NO: 9
**> Human CLDN3 full-length protein (Uniprot: O15551):** 
MSMGLEITGTALAVLGWLGTIVCCALPMWRVSAFIGSNIITSQNIWEGLWMNC
VVQSTGQMQCKVYDSLLALPQDLQAARALIVVAILLAAFGLLVALVGAQCTN
CVQDDTAKAKITIVAGVLFLLAALLTLVPVSWSANTIIRDFYNPVVPEAQKREM
GAGLYVGWAAAALQLLGGALLCCSCPPREKKYTATKVVYSAPRSTGPGASLG
TGYDRKDYV
SEQ ID NO: 10
**> Human CLDN4 full-length protein (Uniprot: O14493):** 
MASMGLQVMGIALAVLGWLAVMLCCALPMWRVTAFIGSNIVTSQTIWEGLW
MNCVVQSTGQMQCKVYDSLLALPQDLQAARALVIISIIVAALGVLLSVVGGKC
TNCLEDESAKAKTMIVAGVVFLLAGLMVIVPVSWTAHNIIQDFYNPLVASGQK
REMGASLYVGWAASGLLLLGGGLLCCNCPPRTDKPYSAKYSAARSAAASNYV
SEQ ID NO: 11
Note: The underlined portions are protein extracellular domains (hereinafter referred to as "ECD" for short).

### Construction of Cell Strains Highly Expressing CLDN6, CLDN9, CLDN3, and CLDN4

A pCDH lentiviral expression vector plasmid containing SEQ ID NOs: 1-11 (synthesized by GENEWIZ), along with a pVSVG or pCMV lentiviral packaging vector, was transfected into 293T cells (Cell Bank of the Chinese Academy of Sciences, GNHu17) using the Lipofectamine 3000 (Invitrogen, L3000015) transfection reagent. The virus-containing culture medium supernatant was collected, filtered, and subjected to ultracentrifugation. After the supernatant was discarded, resuspension was performed in 0.2 mL of sterile PBS. The concentrated virus suspension was used to infect 293T cells (Cell Bank of the Chinese Academy of Sciences, GNHu17). After two to three weeks of screening with puromycin, FACS single-cell sorting was performed. The selected monoclonal cell strains were expanded and cryopreserved.

Note: The ECD sequences of SEQ ID NO: 1 and SEQ ID NO: 2 are identical, the ECD sequences of SEQ ID NO: 3 and SEQ ID NO: 4 are identical, and the ECD sequences of SEQ ID NO: 7 and SEQ ID NO: 9 are identical. Therefore, constructing one of these sequences is sufficient. That is, binding to human CLDN6 can be considered equivalent to binding to cynomolgus monkey CLDN6, and binding to human CLDN9 can be considered equivalent to binding to cynomolgus monkey CLDN9. Binding to mouse CLDN9 can be considered equivalent to binding to rat CLDN9.

### Example 2: Preparation of Anti-Human CLDN6 Monoclonal Antibody

The heavy chain variable region sequence VH45 and the light chain variable region sequence VL43 of CLDN6 were obtained from Patent No. WO2021006328A1 and subjected to humanization combination. The heavy and light chain variable region sequences were cloned into a pTT 5 vector plasmid containing a human IgG1 heavy chain constant region set forth in SEQ ID NO: 20 and a pTT 5 vector plasmid containing a κ light chain constant region set forth in SEQ ID NO: 21, respectively, and the resulting plasmids were transfected into HEK293 cells to obtain an anti-CLDN6 antibody, Ab-1.
> VH45 
QVQLVESGGGVVQPGRSLRLSCAASGFTFSSYVGSWVRQAPGQGLEWMGTISS
GVGRTYYPDVVKGRFTISRDNSKNTLYLQMNSLRAEDTAVYYCARGDFRYDGF
AYWGQGTLVTVSS
SEQ ID NO: 12
> VL43

DIQMTQSPSSLSASVGDRVTITCRASYNIDSYLAWYQQKPGKAPKLLIYYSTLLV
DGVPSRFSGSRSGTDFTLTISSLQPEDFATYYCQHYYSIPYTFGQGTKLEIK
SEQ ID NO: 13

**Table 2. The CDR sequences of antibody Ab-1**

| Antibody | Name | Sequence | No. |
|---|---|---|---|
| | HCDR1 | SYVGS | SEQ ID NO: 14 |
| | HCDR2 | TISSGVGRTYYPDVVKG | SEQ ID NO: 15 |
| Ab-1 | HCDR3 | GDFRYDGFAY | SEQ ID NO: 16 |
| | LCDR1 | RASYNIDSYLA | SEQ ID NO: 17 |
| | LCDR2 | YSTLLVD | SEQ ID NO: 18 |
| | LCDR3 | QHYYSIPYT | SEQ ID NO: 19 |

| | | | |
|---|---|---|---|
| Note: The amino acid residues of the CDRs of the VH/VL were determined by the Kabat numbering scheme and underlined; the same applies hereinafter. | | | |

> Human IgG1 (LALA) heavy chain constant region: 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 20
> Human κ light chain constant region: 


SEQ ID NO: 21
> The heavy chain of antibody Ab-1: 
QVQLVESGGGVVQPGRSLRLSCAASGFTFSSYVGSWVRQAPGQGLEWMGTI
SSGVGRTYYPDVVKGRFTISRDNSKNTLYLQMNSLRAEDTAVYYCARGDFRY
DGFAYWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDK
KVEPKSCDKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALH
NHYTQKSLSLSPGK
SEQ ID NO: 22
> The light chain of antibody Ab-1:

DIQMTQSPSSLSASVGDRVTITCRASYNIDSYLAWYQQKPGKAPKLLIYYSTL
LVDGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 23.

### Example 3: Hotspot-Removal Engineering of Anti-CLDN6 Monoclonal Antibody

The CDRs of VL43 were engineered by point mutation to remove the hotspots.

**Table 3. The CDR sequences of VL43 after hotspot removal**

| Amino acid engineering of variable regions | CDR | Sequence | SEQ ID NO |
|---|---|---|---|
| D56E | LCDR2 | YSTLLVE | 24 |
| S31R | LCDR1 | RASYNIDRYLA | 25 |
| S31Q | LCDR1 | RASYNIDQYLA | 26 |
| S31I | LCDR1 | RASYNIDIYLA | 27 |
| S31L | LCDR1 | RASYNIDLYLA | 28 |
| S31V | LCDR1 | RASYNIDVYLA | 29 |

Antibody light chain variable regions were reconstructed using the CDRs shown in Table 3. The specific sequences are shown below:
> VL43+D56E DIQMTQSPSSLSASVGDRVTITCRASYNIDSYLAWYQQKPGKAPKLLIYYSTLLV 
EGVPSRFSGSRSGTDFTLTISSLQPEDFATYYCQHYYSIPYTFGQGTKLEIK
SEQ ID NO: 30
> VL43+D56E+S31R 
DIQMTQSPSSLSASVGDRVTITCRASYNIDRYLAWYQQKPGKAPKLLIYYSTLLV
EGVPSRFSGSRSGTDFTLTISSLQPEDFATYYCQHYYSIPYTFGQGTKLEIK
SEQ ID NO: 31
> VL43+D56E+S31Q 
DIQMTQSPSSLSASVGDRVTITCRASYNIDQYLAWYQQKPGKAPKLLIYYSTLL
VEGVPSRFSGSRSGTDFTLTISSLQPEDFATYYCQHYYSIPYTFGQGTKLEIK
SEQ ID NO: 32
> VL43+D56E+S31I 
DIQMTQSPSSLSASVGDRVTITCRASYNIDIYLAWYQQKPGKAPKLLIYYSTLLV
EGVPSRFSGSRSGTDFTLTISSLQPEDFATYYCQHYYSIPYTFGQGTKLEIK
SEQ ID NO: 33
> VL43+D56E+S31L 
DIQMTQSPSSLSASVGDRVTITCRASYNIDLYLAWYQQKPGKAPKLLIYYSTLLV
EGVPSRFSGSRSGTDFTLTISSLQPEDFATYYCQHYYSIPYTFGQGTKLEIK
SEQ ID NO: 34
> VL43+D56E+S31V

DIQMTQSPSSLSASVGDRVTITCRASYNIDVYLAWYQQKPGKAPKLLIYYSTLL
VEGVPSRFSGSRSGTDFTLTISSLQPEDFATYYCQHYYSIPYTFGQGTKLEIK
SEQ ID NO: 35

Note: VL43+D56E means a light chain variable region sequence obtained by mutating the amino acid D at position 56 of VL43 to E; VL43+D56E+S31R means a light chain variable region sequence obtained by mutating the amino acid D at position 56 of VL43 to E and mutating the amino acid S at position 31 to R. The others are to be interpreted similarly.

The single-underlined portions are CDR regions, and the double-underlined portions are mutation sites.

Each of SEQ ID NOs: 30-35 was cloned into a pTT 5 vector plasmid containing the κ light chain constant region set forth in SEQ ID NO: 21, and the resulting plasmid, along with a plasmid obtained by cloning SEQ ID NO: 12 into a pTT 5 vector plasmid containing the human IgG1 heavy chain constant region set forth in SEQ ID NO: 20, was transfected into HEK293 cells to obtain anti-CLDN6 antibodies: Ab-2, Ab-3, Ab-4, Ab-5, Ab-6, and Ab-7.
> The heavy chains of antibodies Ab-2, Ab-3, Ab-4, Ab-5, Ab-6, and Ab-7: SEQ ID NO: 22, the same as the heavy chain of antibody Ab-1.
> The light chain of antibody Ab-2: 
DIQMTQSPSSLSASVGDRVTITCRASYNIDSYLAWYQQKPGKAPKLLIYYSTL
LVEGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 36
> The light chain of antibody Ab-3: 
DIQMTQSPSSLSASVGDRVTITCRASYNIDRYLAWYQQKPGKAPKLLIYYSTL
LVEGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 37
> The light chain of antibody Ab-4: 
DIQMTQSPSSLSASVGDRVTITCRASYNIDQYLAWYQQKPGKAPKLLIYYSTL
LVEGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 38
> The light chain of antibody Ab-5: 
DIQMTQSPSSLSASVGDRVTITCRASYNIDIYLAWYQQKPGKAPKLLIYYSTLL
VEGVPSRFSGSRSGTDFTLTISSLQPEDFATYYCQHYYSIPYTFGQGTKLEIKRT
VAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 39
> The light chain of antibody Ab-6: 
DIQMTQSPSSLSASVGDRVTITCRASYNIDLYLAWYQQKPGKAPKLLIYYSTL
LVEGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 40
> The light chain of antibody Ab-7:

DIQMTQSPSSLSASVGDRVTITCRASYNIDVYLAWYQQKPGKAPKLLIYYSTL
LVEGVPSRFSGSRSGTDFTLTISSLQPEDFATYYCQHYYSIPYTFGQGTKLEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 41

The VH/VL sequences of the negative control antibody used in the present disclosure, Isotype, were from Patent No. US6114143A, and its heavy and light chain constant region sequences were SEQ ID NO: 20 and SEQ ID NO: 21, respectively. Its full-length sequence is shown below:
The heavy chain of Isotype: 
QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
THTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK
SEQ ID NO: 42
The light chain of Isotype:

DIQMTQSPSSLSASVGDRVTMSCKSSQSLLNSGDQKNYLTWYQQKPGKAPKLLI
YWASTGESGVPSRFSGSGSGTDFTFTISSLQPEDIATYYCQNDYSYPWTFGQGTK
VEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 43

Note: In the sequences, the underlined portions are variable regions, and the italicized portions are constant regions.

### II. Preparation of ADCs

### Drug loading analysis of ADC stock solutions

ADCs refer to antibody-drug conjugates. They treat diseases by using the targeting capability of the antibody to deliver the drug into cells, thereby killing the cells or inhibiting the growth of the cells. The drug loading plays a decisive role in determining drug efficacy.

In the present disclosure, the drug loading was analyzed by RP-HPLC. The process was basically as follows:

### Reagents and instrument:

Trifluoroacetic acid (TFA): produced by sigma, 100 mL/bottle; acetonitrile: LC grade, 4 L/bottle, produced by Thermo Fisher; DTT: produced by sigma, 1 g/bottle.

High performance liquid chromatograph: Agilent 1200.

### Preparation of solutions:

1) 0.25 M DTT solution:
   Preparation example: 5.78 mg of DTT was completely dissolved in 150 µL of purified water to prepare a 0.25 M DTT solution, which was then stored at -20 °C.
2) Mobile phase A (0.1% TFA in water):
   Preparation example: 1000 mL of purified water was measured out using a graduated cylinder, and 1 mL of TFA was added. The solution was well mixed before use and was stored at 2-8 °C for 14 days.
3) Mobile phase B (0.1% TFA in acetonitrile):
   Preparation example: 1000 mL of acetonitrile was measured out using a graduated cylinder, and 1 mL of TFA was added. The solution was well mixed before use and was stored at 2-8 °C for 14 days.

The naked antibodies and the test samples (concentration: 1 mg/mL, about 200 µL) were reduced with 4 µL of DTT in a water bath at 37 °C for 1 h and then transferred to inserts so that they were ready for sample injection.

### Chromatography conditions:

Chromatography column: Agilent PLRP-S 1000A 8 µm 4.6 × 250 mm; column temperature: 80 °C;
DAD detector: detection wavelength: 280 nm; sample chamber temperature: 4 °C; flow rate: 1 mL/min;
Injection volume: 40 µL;
The chromatography gradients are shown in Table 4:

**Table 4**

| Time (min) | Mobile phase A% | Mobile phase B% |
|---|---|---|
| 0 | 80.0 | 20.0 |
| 5.00 | 64.0 | 36.0 |
| 32.00 | 40.0 | 60.0 |
| 32.10 | 80.0 | 20.0 |
| 45.00 | 80.0 | 20.0 |

### Data analysis:

By comparing the spectra of the samples and the naked antibodies, the locations of the light and heavy chains were identified. Then, the spectra of the test samples were integrated to calculate their DAR values. The calculation formulas are shown below:

**Table 5**

| Name | Number of linked drugs |
|---|---|
| LC | 0 |
| LC+1 | 2 |
| HC | 0 |
| HC+1 | 2 |
| HC+2 | 4 |
| HC+3 | 6 |

| | |
|---|---|
| Total LC peak area = LC peak area + LC+1 peak area; Total HC peak area = HC peak area + HC+1 peak area + HC+2 peak area + HC+3 peak area; LC DAR = Σ(number of linked drugs × percent peak area) / total LC peak area; HC DAR = Σ(number of linked drugs × percent peak area) / total HC peak area; DAR = LC DAR + HC DAR. | |

### Example 4-1: Synthesis of Glycan Chain OLS-4 (2S,3S,4S,5R,6R)-4-(2-(3-(2-(2-Azidoethoxy)ethoxy)propoxy)ethoxy)-2-(((3aR,5R,6S,7R,7aR)-7-hydroxy-5-(hydroxymethyl)-2-methyl-5,6,7,7a-tetrahydro-3aH-pyrano[3,2-d]oxazol-6-yl)oxy)-6-(hydroxymethyl)tetrahydro-2H-pyran-3,5-diol OLS-4

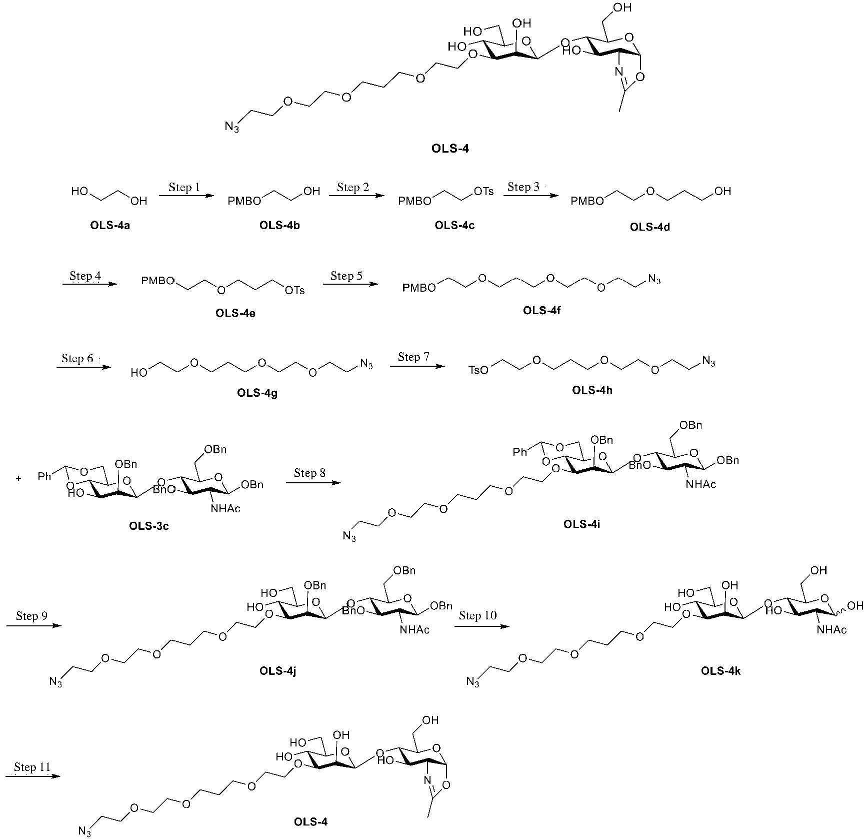

### Step 1

### 2-((4-Methoxybenzyl)oxy)ethan-1-ol OLS-4b

Ethylene glycol OLS-4a (50.00 g, 805.57 mmol) was dissolved in anhydrous tetrahydrofuran (300 mL), and sodium hydride (4.93 g, 128.89 mmol, content: 60%) and tetrabutylammonium iodide (5.37 g, 16.11 mmol) were sequentially added. The mixture was stirred at 0 °C for 30 min in a nitrogen atmosphere. Subsequently, p-methoxybenzyl chloride (20.19 g, 128.89 mmol) was slowly added dropwise over about 30 min. The mixture was heated to 80 °C and stirred for 16 h. The reaction mixture was poured into iced water, and the resulting mixture was stirred to quench the reaction. Ethyl acetate (500 mL) was added, and the resulting mixture was stirred and separated. The resulting aqueous phase was extracted with ethyl acetate (150 mL × 3), and the organic phases were combined, washed with water (250 mL × 3) and a saturated sodium chloride solution (250 mL × 2), dried over anhydrous sodium sulfate, and filtered to remove the drying agent. The filtrate was concentrated under reduced pressure, and the resulting residue was purified by silica gel column chromatography with eluent system C to give the title product OLS-4b (22.00 g, yield: 14.9%).

MS m/z (ESI): 205.2 [M+23].

### Step 2

### 2-((4-Methoxybenzyl)oxy)ethyl 4-methylbenzenesulfonate OLS-4c

OLS-4b (22.00 g, 120.74 mmol) was dissolved in pyridine (98 mL), and p-toluenesulfonyl chloride (27.62 g, 144.88 mmol) was added at 0 °C. In a nitrogen atmosphere, the mixture was stirred at 0 °C, slowly warmed to room temperature, and left to react for 16 h. The reaction mixture was diluted with ethyl acetate (300 mL) and washed with hydrochloric acid (10%, 150 mL × 3). The organic phase was sequentially washed with water (150 mL × 3) and a saturated sodium chloride solution (250 mL × 2), dried over anhydrous sodium sulfate for 30 min, and filtered to remove the drying agent. The filtrate was concentrated under reduced pressure, and the resulting residue was purified by silica gel column chromatography with eluent system C to give the title product OLS-4c (36.40 g, yield: 89.6%).

1H NMR (400 MHz, CDCl3) δ 7.81 (d, 2H), 7.33 (d, 2H), 7.21 (d, 2H), 6.88 (d, 2H), 4.43 (s, 2H), 4.23-4.17 (m, 2H), 3.82 (s, 3H), 3.68-3.61 (m, 2H), 2.45 (s, 3H).

### Step 3

### 3-(2-((4-Methoxybenzyl)oxy)ethoxy)propan-1-ol OLS-4d

OLS-4c (36.40 g, 108.20 mmol) and 1,3-propanediol (82.34 g, 1.08 mol) were dissolved in toluene (100 mL), and potassium hydroxide powder (15.18 g, 270.51 mmol) was added at room temperature. The mixture was heated to 50 °C in a nitrogen atmosphere and stirred for 72 h. The reaction mixture was poured into iced water (200 mL), and concentrated hydrochloric acid was added dropwise until the pH of the reaction mixture was 3-4. Ethyl acetate (300 mL) was added for extraction. The aqueous phase was isolated and extracted with ethyl acetate (100 mL × 3), and the organic phases were combined, dried over anhydrous sodium sulfate for 30 min, and filtered to remove the drying agent. The filtrate was concentrated under reduced pressure, and the resulting residue was purified by silica gel column chromatography with eluent system C to give the title product OLS-4d (15.60 g, yield: 60.0%).

1H NMR (400 MHz, CDCl3) δ 7.28 (d, 2H), 6.89 (d, 2H), 4.51 (s, 2H), 3.81 (s, 3H), 3.79 (t, 2H), 3.68 (t, 2H), 3.65-3.59 (m, 4H), 2.5i1 (br.s, 1H), 1.88-1.82 (m, 2H).

### Step 4

### 3-(2-((4-Methoxybenzyl)oxy)ethoxy)propyl 4-methylbenzenesulfonate OLS-4e

OLS-4d (15.60 g, 64.92 mmol) was dissolved in a mixed solution of pyridine (52 mL) and dichloromethane (52 mL), and p-toluenesulfonyl chloride (18.56 g, 97.38 mmol) was added at 0 °C. In a nitrogen atmosphere, the mixture was stirred at 0 °C, slowly warmed to room temperature, and left to react for 16 h. The reaction mixture was diluted with ethyl acetate (300 mL), sequentially washed with hydrochloric acid (10%, 150 mL × 3), water (150 mL × 3), and a saturated sodium chloride solution (250 mL × 2), dried over anhydrous sodium sulfate for 30 min, and filtered to remove the drying agent. The filtrate was concentrated under reduced pressure, and the resulting residue was purified by silica gel column chromatography with eluent system C to give the title product OLS-4e (18.00 g, yield: 70.3%).

MS m/z (ESI): 417.3 [M+23].

1H NMR (400 MHz, CDCl3) δ 7.80 (d, 2H), 7.34 (d, 2H), 7.27 (d, 2H), 6.90 (d, 2H), 4.48 (s, 2H), 4.16 (t, 2H), 3.83 (s, 3H), 3.53-3.49 (m, 6H), 2.45 (s, 3H), 1.96-1.91 (m, 2H).

### Step 5

### 14-Azido-1-(4-methoxyphenyl)-2,5,9,12-tetraoxatetradecane OLS-4f

OLS-4e (8.00 g, 20.28 mmol) and 2-(2-azidoethoxy)ethanol (3.72 g, 28.39 mol, Amatek) were dissolved in toluene (33 mL), and potassium hydroxide powder (2.84 g, 50.70 mmol) was added at room temperature. The mixture was heated to 60 °C in a nitrogen atmosphere and stirred for 16 h. The reaction mixture was poured into iced water (100 mL), and concentrated hydrochloric acid was added dropwise until the pH of the reaction mixture was 3-4. Ethyl acetate (100 mL) was added for extraction. The aqueous phase was isolated and extracted with ethyl acetate (50 mL × 3), and the organic phases were combined, dried over anhydrous sodium sulfate for 30 min, and filtered to remove the drying agent. The filtrate was concentrated under reduced pressure, and the resulting residue was purified by silica gel column chromatography with eluent system C to give the title product OLS-4f (6.3 g, yield: 87.9%).

MS m/z (ESI): 376.3 [M+23].

1H NMR (400 MHz, CDCl3) δ 7.29 (d, 2H), 6.89 (d, 2H), 4.52 (s, 2H), 3.82 (s, 3H), 3.70-3.56 (m, 14H), 3.40 (t, 2H), 1.93-1.87 (m, 2H).

### Step 6

### 2-(3-(2-(2-Azidoethoxy)ethoxy)propoxy)ethan-1-ol OLS-4g

OLS-4f (6.30 g, 17.83 mmol) was dissolved in dichloromethane (31 mL) and water (3.1 mL), and dichlorodicyanobenzoquinone (4.45 g, 19.61 mmol, Aladdin) was added at room temperature. The mixture was stirred at 25 °C for 1.5 h in a nitrogen atmosphere. The reaction mixture was filtered through diatomaceous earth. The filtrate was diluted with dichloromethane (150 mL), sequentially washed with a saturated sodium bicarbonate solution (100 mL × 2), a saturated sodium bisulfite solution (100 mL × 2), water (100 mL), and a saturated sodium chloride solution (100 mL × 2), dried over anhydrous sodium sulfate for 30 min, and filtered under vacuum. The filtrate was concentrated under reduced pressure, and the resulting residue was purified by silica gel column chromatography with eluent system C to give the title product OLS-4g (3.2 g, yield: 76.9%).

### Step 7

### 2-(3-(2-(2-Azidoethoxy)ethoxy)propoxy)ethyl 4-methylbenzenesulfonate OLS-4h

OLS-4g (3.20 g, 13.72 mmol) was dissolved in pyridine (11 mL) and dichloromethane (11 mL). The solution was cooled to 0 °C, and p-toluenesulfonyl chloride (3.14 g, 16.46 mmol) was added. In a nitrogen atmosphere, the mixture was stirred at 0 °C, slowly warmed to room temperature, and left to react for 16 h. The reaction mixture was diluted with ethyl acetate (200 mL), sequentially washed with hydrochloric acid (10%, 150 mL × 3), water (150 mL × 3), and a saturated sodium chloride solution (250 mL × 2), dried over anhydrous sodium sulfate for 30 min, and filtered to remove the drying agent. The filtrate was concentrated under reduced pressure, and the resulting residue was purified by silica gel column chromatography with eluent system C to give the title product OLS-4h (4.80 g, yield: 90.3%).

1H NMR (400 MHz, CDCl3) δ 7.79 (d, 2H), 7.34 (d, 2H), 4.14 (t, 2H), 3.67-3.57 (m, 8H), 3.51-3.46 (m, 4H), 3.38 (t, 2H), 2.44 (s, 3H), 1.81-1.75 (m, 2H).

### Step 8

### N-((2R,3R,4R,5S,6R)-5-(((2R,4aR,6S,7S,8S,8aR)-8-(2-(3-(2-(2-Azidoethoxy)ethoxy)propoxy)ethoxy)-7-(benzyloxy)-2-phenylhexahydropyrano[3,2-d] [1,3]dioxin-6-yl)oxy)-2,4-bis(benzyloxy)-6-((benzyloxy)methyl)tetrahydro-2H-pyran-3-yl)acetamide OLS-4i

OLS-3c (340 mg, 0.41 mmol), N,N-dimethylformamide (4.8 mL), and 2 mL of a solution of OLS-4h (398 mg, 1.03 mmol) in N,N-dimethylformamide were sequentially added to a reaction flask and cooled to 0 °C. Sodium hydride (86 mg, 2.15 mmol, content: 60%) was added, and the mixture was left to react at 0 °C for about 0.5 h and then at 20-30 °C for about 0.5 h. The reaction mixture was poured into a mixed solution of a saturated ammonium chloride solution (10 mL) and water (10 mL), and extraction was performed with ethyl acetate (10 mL × 3). The organic phases were combined, sequentially washed with water (10 mL) and a saturated sodium chloride solution (10 mL), dried over anhydrous sodium sulfate, and filtered to remove the drying agent. The filtrate was concentrated under reduced pressure, and the resulting residue was purified by thin-layer chromatography with developing solvent system C to give the title product OLS-4i (362 mg, yield: 84.6%).

MS m/z (ESI): 1047.4 [M+1].

1H NMR (400 MHz, CDCl3) δ 7.40-7.16 (m, 25H), 5.70 (d, 1H), 5.44 (s, 1H), 4.87 (d, 1H), 4.84-4.78 (m, 3H), 4.71-4.68 (m, 1H), 4.57-4.50 (m, 4H), 4.41-4.38 (m, 1H), 4.04-4.01 (m, 1H), 3.97 (t, 1H), 3.93-3.84 (m, 2H), 3.76-3.68 (m, 3H), 3.61-3.40 (m, 16H), 3.34-3.27 (m, 3H), 3.11-3.05 (m, 2H), 1.78-1.72 (m, 2H), 1.51 (s, 3H).

### Step 9

### N-((2R,3R,4R,5S,6R)-5-(((2S,3S,4S,5R,6R)-4-(2-(3-(2-(2-Azidoethoxy)ethoxy)propoxy)ethoxy)-3-(benzyloxy)-5-hydroxy-6-(hydroxymethyl)tetrahydro-2H-pyran-2-yl)oxy)-2,4-bis(benzyloxy)-6-((benzyloxy)methyl)tetrahydro-2H-pyran-3-yl)acetamide OLS-4j

Compound OLS-4i (360 mg, 0.34 mmol) and dichloromethane (26 mL) were sequentially added to a reaction flask. In a nitrogen atmosphere, the temperature was lowered to -30 to -20 °C, and trifluoroacetic acid (3.6 mL) was added dropwise. After the dropwise addition, the mixture was warmed to -5 to 5 °C and stirred for about 1.5 h. The reaction mixture was quenched with methanol (5.1 mL), diluted with dichloromethane (12.3 mL), sequentially washed with a saturated sodium bicarbonate solution (26 mL × 2) and a saturated sodium chloride solution (13 mL), dried over anhydrous sodium sulfate, and filtered to remove the drying agent. The filtrate was concentrated under reduced pressure, and the resulting residue was purified by thin-layer chromatography with developing solvent system C to give the title product OLS-4j (234 mg, yield: 71.0%).

MS m/z (ESI): 959.7 [M+1].

1H NMR (400 MHz, CDCl3) δ 7.31-7.17 (m, 20H), 5.64 (d, 1H), 4.87 (d, 1H), 4.84-4.79 (m, 2H), 4.70 (s, 2H), 4.58-4.50 (m, 3H), 4.43-4.39 (m, 2H), 4.05 (t, 1H), 3.82 (t, 1H), 3.74-3.36 (m, 23H), 3.30 (t, 2H), 3.10-3.07 (m, 1H), 3.00-2.97 (m, 1H), 2.21 (br.s, 1H), 1.81-1.75 (m, 2H), 1.66 (s, 3H).

### Step 10

### N-((3R,4R,5S,6R)-5-(((2S,3S,4S,5R,6R)-4-(2-(3-(2-(2-Azidoethoxy)ethoxy)propoxy)ethoxy)-3,5-dihydroxy-6-(hydroxymethyl)tetrahydro-2H-pyran-2-yl)oxy)-2,4-dihydroxy-6-(hydroxymethyl)tetrahydro-2H-pyran-3-yl)acetamide OLS-4k (a mixture of diastereomers)

OLS-4j (150 mg, 158.72 µmol) was dissolved in tetrahydrofuran (8 mL) and water (2 mL), and palladium hydroxide (50.0 mg, content: 15%), palladium on carbon (61 mg, content: 10%), and hydrochloric acid (1 drop) were added. The mixture was purged with hydrogen three times and stirred at room temperature for 12 h. The catalyst was removed by filtration. The filtrate was concentrated under reduced pressure, and the resulting residue was dissolved in methanol (7 mL). Imidazole-1-sulfonyl azide hydrochloride (6 mg, 220.41 µmol), potassium carbonate (68 mg, 492.00 µmol), and cupric sulfate (5.7 mg, 22.83 µmol) were added, and the mixture was stirred at room temperature for 12 h. The inorganic salts were removed by filtration, and the solid was rinsed with methanol (3 mL). The organic phases were combined, and the filtrate was concentrated under reduced pressure to give the title product OLS-4k (crude, 161 mg). The product was directly used in the next step without purification.

MS m/z (ESI): 599.3 [M+1].

### Step 11

### (2S,3S,4S,5R,6R)-4-(2-(3-(2-(2-Azidoethoxy)ethoxy)propoxy)ethoxy)-2-(((3aR,5R,6S,7R,7aR)-7-hydroxy-5-(hydroxymethyl)-2-methyl-5,6,7,7a-tetrahydro-3aH-pyrano[3,2-d]oxazol-6-yl)oxy)-6-(hydroxymethyl)tetrahydro-2H-pyran-3,5-diol OLS-4

In an ice bath, compound OLS-4k (crude, 161 mg, 148.01 µmol) was dissolved in purified water (8 mL), and 2-chloro-1,3-dimethyl-1H-benzimidazol-3-ium chloride (557 mg, 3.07 mmol) and cesium carbonate (3.00 g, 9.20 mmol) were added. The mixture was stirred at 0 °C for 12 h. The reaction mixture was purified by high performance liquid chromatography (column: Phenomenex C18, 150 × 25 mm, 10 µm; mobile phase: water (0.01% ammonia water) and acetonitrile, gradient ratio: acetonitrile 0%-30%, flow rate: 25 mL/min) and lyophilized in a 5 mol% sodium hydroxide solution to give the title product OLS-4 (35 mg, overall yield of steps 10 and 11: 38.2%).

MS m/z (ESI): 581.3 [M+1].

1H NMR (400 MHz, CDCl3) δ 6.02 (d, 1H), 4.65 (s, 2H), 4.30 (s, 2H), 4.09-3.93(m, 5H), 3.86-3.77 (m, 4H), 3.73-3.51 (m, 16H), 3.40-3.28 (m, 5H), 2.01 (s, 3H), 1.88-1.81 (m, 2H).

### Example 4-2: Synthesis of ADC-1, ADC-2, and ADC-3

The following general reaction scheme was applied to the preparation of ADC-1, ADC-2, and ADC-3: 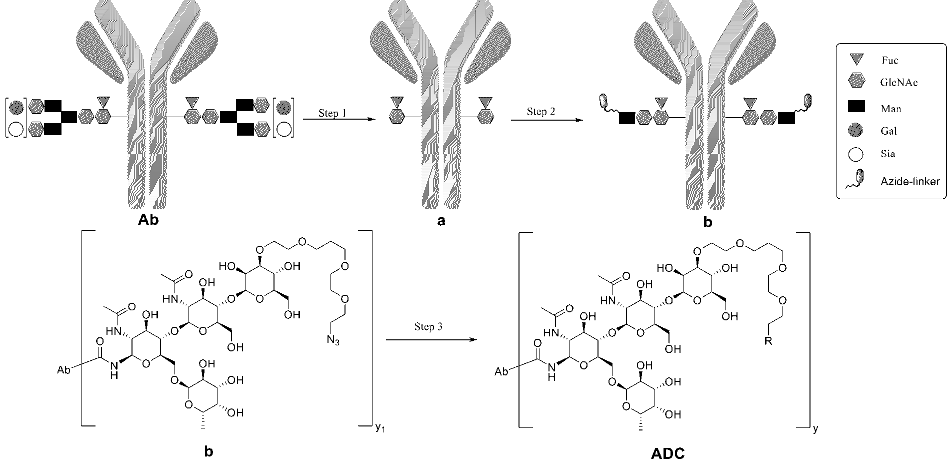 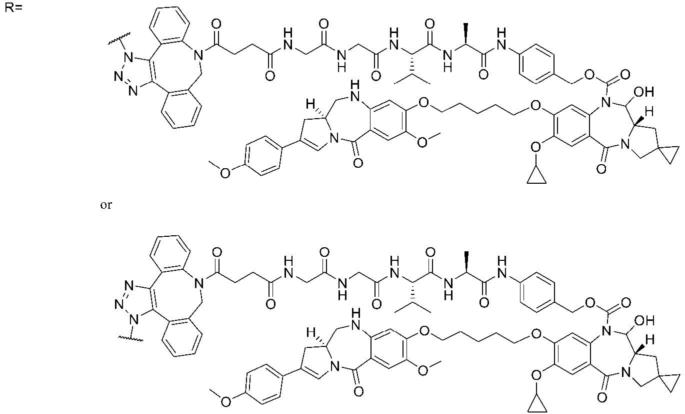 (The triazole ring formed in that step has a geometric structure, and the compound obtained contains the two structures represented by R.)

The linker-toxin used in the reaction of step 3 was compound LD-11 (prepared using the method disclosed in Example 3-11 on "pages 142-146 of the specification of Patent Application No. WO2023051814A1"). Its specific structure is shown below:

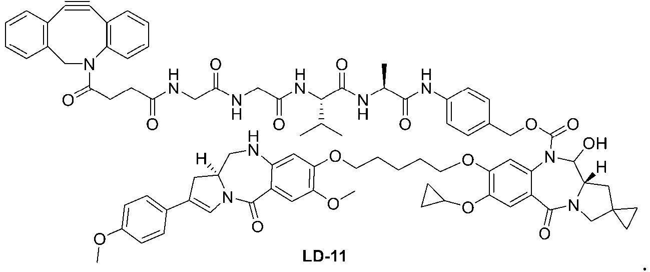

### Example 4-2-1: ADC-1

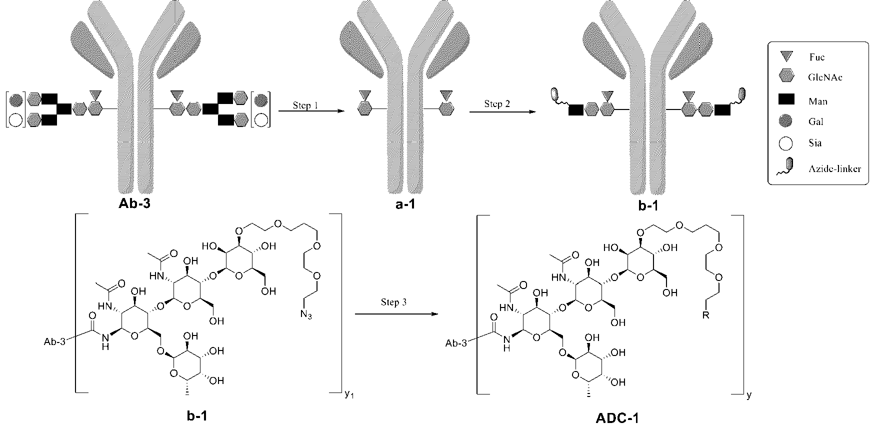

### Step 1

Endo S enzyme (19.16 mg/mL, 10.4 µL) was added to antibody Ab-3 in an aqueous PBS buffer solution (a 0.05 M aqueous PBS buffer solution (pH 6.3); 10 mg/mL, 2 mL) at 37 °C. The mixture was shaken on a water bath shaker at 37 °C for 12 h, and the reaction was stopped. The reaction mixture was purified using a Protein A protein purification column (elution phase: an aqueous acetic acid buffer solution (pH 3.0)) to give an affinity eluate of a-1. Then, the product was buffer-exchanged into a 0.05 M PBS buffer (pH 6.3), and the resulting solution was refrigerated at 4 °C.

### Step 2

Endo S enzyme (19.16 mg/mL, 10.4 µL) and OLS-4 (2.32 mg) were added to a-1 in an aqueous PBS buffer solution (a 0.05 M aqueous PBS buffer solution (pH 6.3); 10.0 mg/mL, 2 mL) at 37 °C. The mixture was shaken on a water bath shaker at 37 °C for 1 h, and the reaction was stopped. The reaction mixture was purified using a Protein A protein purification column (elution phase: an aqueous acetic acid buffer solution (pH 3.0)) to give an affinity eluate of b-1. Then, the product was buffer-exchanged into an aqueous PBS buffer solution (pH 7.4), and the resulting solution was refrigerated at 4 °C. y1 represents the average number of glycan chains remodeled at the N297 sites of the heavy chains of the antibody; two glycan chains or one glycan chain may be remodeled at the two N297 sites of the heavy chains of the antibody.

### Step 3

At 25 °C, 0.8 mL of 1,2-propanediol was added to b-1 in an aqueous PBS buffer solution (an aqueous PBS buffer solution (pH 7.4); 8.0 mg/mL, 1.0 mL). After shaking for thorough mixing, a solution of LD-11 (0.388 mg, 266 nmol, dissolved in a mixed solvent of 100 µL of dimethyl sulfoxide and 100 µL of 1,2-propanediol) was added, and the mixture was left to react on a shaker at room temperature for 12 h. The reaction mixture was centrifuged, and the supernatant was diluted with 15 mL of a PBS buffer solution (pH 7.4) and then purified using a Protein A protein purification column (elution phase: an aqueous acetic acid buffer solution (pH 3.0)). The pH value of the resulting solution was adjusted to about 5.0 with a tris(hydroxymethyl)aminomethane hydrochloride solution (1 M, pH 8.0) to give an affinity eluate of the title product ADC-1, which was then refrigerated at 4 °C.

The average number of bound drugs per antibody molecule was calculated by MS: y = 1.7.

### Example 4-2-2: ADC-2

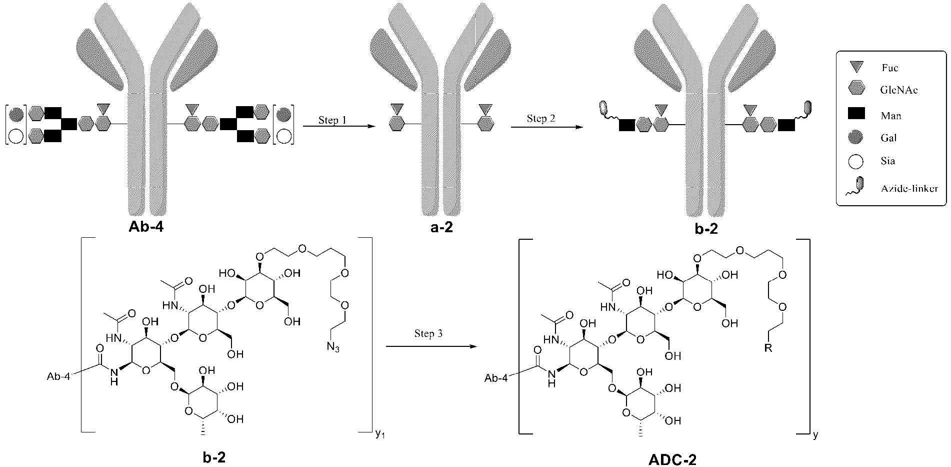

### Step 1

Endo S enzyme (19.16 mg/mL, 15.6 µL) was added to antibody Ab-4 in an aqueous PBS buffer solution (a 0.05 M aqueous PBS buffer solution (pH 6.3); 10 mg/mL, 3 mL) at 37 °C. The mixture was shaken on a water bath shaker at 37 °C for 12 h, and the reaction was stopped. The reaction mixture was purified using a Protein A protein purification column (elution phase: an aqueous acetic acid buffer solution (pH 3.0)) to give an affinity eluate of a-2. Then, the product was buffer-exchanged into a 0.05 M PBS buffer (pH 6.3), and the resulting solution was refrigerated at 4 °C.

### Step 2

Endo S enzyme (19.16 mg/mL, 10.4 µL) and OLS-4 (2.32 mg) were added to a-2 in an aqueous PBS buffer solution (a 0.05 M aqueous PBS buffer solution (pH 6.3); 10.0 mg/mL, 2 mL) at 37 °C. The mixture was shaken on a water bath shaker at 37 °C for 1 h, and the reaction was stopped. The reaction mixture was purified using a Protein A protein purification column (elution phase: an aqueous acetic acid buffer solution (pH 3.0)) to give an affinity eluate of b-2. Then, the product was buffer-exchanged into an aqueous PBS buffer solution (pH 7.4), and the resulting solution was refrigerated at 4 °C. y1 represents the average number of glycan chains remodeled at the N297 sites of the heavy chains of the antibody; two glycan chains or one glycan chain may be remodeled at the two N297 sites of the heavy chains of the antibody.

### Step 3

At 25 °C, 0.8 mL of 1,2-propanediol was added to b-2 in an aqueous PBS buffer solution (an aqueous PBS buffer solution (pH 7.4); 8.0 mg/mL, 1.0 mL). After shaking for thorough mixing, a solution of LD-11 (0.388 mg, 266 nmol, dissolved in a mixed solvent of 100 µL of dimethyl sulfoxide and 100 µL of 1,2-propanediol) was added, and the mixture was left to react on a shaker at room temperature for 12 h. The reaction mixture was centrifuged, and the supernatant was diluted with 15 mL of a PBS buffer solution (pH 7.4) and then purified using a Protein A protein purification column (elution phase: an aqueous acetic acid buffer solution (pH 3.0)). The pH value of the resulting solution was adjusted to about 5.0 with a tris(hydroxymethyl)aminomethane hydrochloride solution (1 M, pH 8.0) to give an affinity eluate of the title product ADC-2, which was then refrigerated at 4 °C.

The average number of bound drugs per antibody molecule was calculated by MS: y = 1.7.

### Example 4-2-3: ADC-3

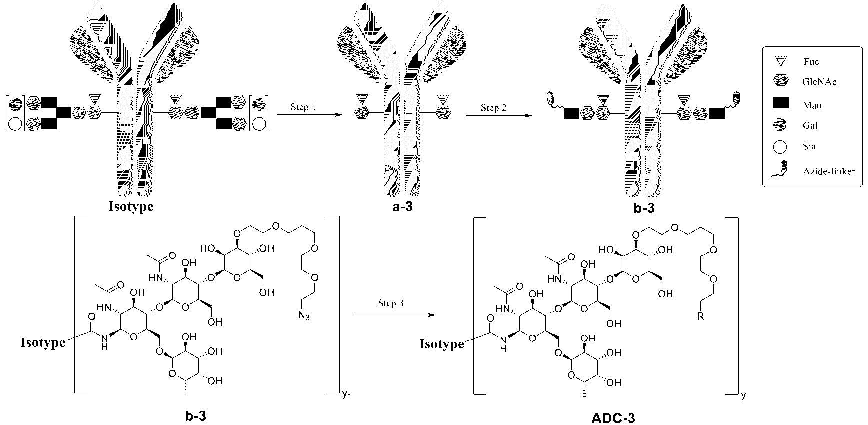

### Step 1

Endo S enzyme (19.16 mg/mL, 16.8 µL) was added to the antibody Isotype in an aqueous PBS buffer solution (a 0.05 M aqueous PBS buffer solution (pH 6.3); 10 mg/mL, 3.23 mL) at 37 °C. The mixture was shaken on a water bath shaker at 37 °C for 12 h, and the reaction was stopped. The reaction mixture was purified using a Protein A protein purification column (elution phase: an aqueous acetic acid buffer solution (pH 3.0)) to give an affinity eluate of a-3. Then, the product was buffer-exchanged into a 0.05 M PBS buffer (pH 6.3), and the resulting solution was refrigerated at 4 °C.

### Step 2

Endo S enzyme (19.16 mg/mL, 15.6 µL) and OLS-4 (3.48 mg) were added to a-3 in an aqueous PBS buffer solution (a 0.05 M aqueous PBS buffer solution (pH 6.3); 10.0 mg/mL, 3.0 mL) at 37 °C. The mixture was shaken on a water bath shaker at 37 °C for 1 h, and the reaction was stopped. The reaction mixture was purified using a Protein A protein purification column (elution phase: an aqueous acetic acid buffer solution (pH 3.0)) to give an affinity eluate of b-3. Then, the product was buffer-exchanged into an aqueous PBS buffer solution (pH 7.4), and the resulting solution was refrigerated at 4 °C. y1 represents the average number of glycan chains remodeled at the N297 sites of the heavy chains of the antibody; two glycan chains or one glycan chain may be remodeled at the two N297 sites of the heavy chains of the antibody.

### Step 3

At 25 °C, 2.8 mL of 1,2-propanediol was added to b-3 in an aqueous PBS buffer solution (an aqueous PBS buffer solution (pH 7.4); 8.0 mg/mL, 3.5 mL). After shaking for thorough mixing, a solution of LD-11 (1.36 mg, 931 nmol, dissolved in a mixed solvent of 350 µL of dimethyl sulfoxide and 350 µL of 1,2-propanediol) was added, and the mixture was left to react on a shaker at room temperature for 12 h. The reaction mixture was centrifuged, and the supernatant was diluted with 30 mL of a PBS buffer solution (pH 7.4) and then purified using a Protein A protein purification column (elution phase: an aqueous acetic acid buffer solution (pH 3.0)). The pH value of the resulting solution was adjusted to about 5.0 with a tris(hydroxymethyl)aminomethane hydrochloride solution (1 M, pH 8.0) to give an affinity eluate of the title product ADC-3, which was then refrigerated at 4 °C.

The average number of bound drugs per antibody molecule was calculated by MS: y = 1.71.

### Example 4-3: Synthesis of ADC-4, ADC-5, and ADC-6

The following general reaction scheme was applied to the preparation of ADC-4, ADC-5, and ADC-6:

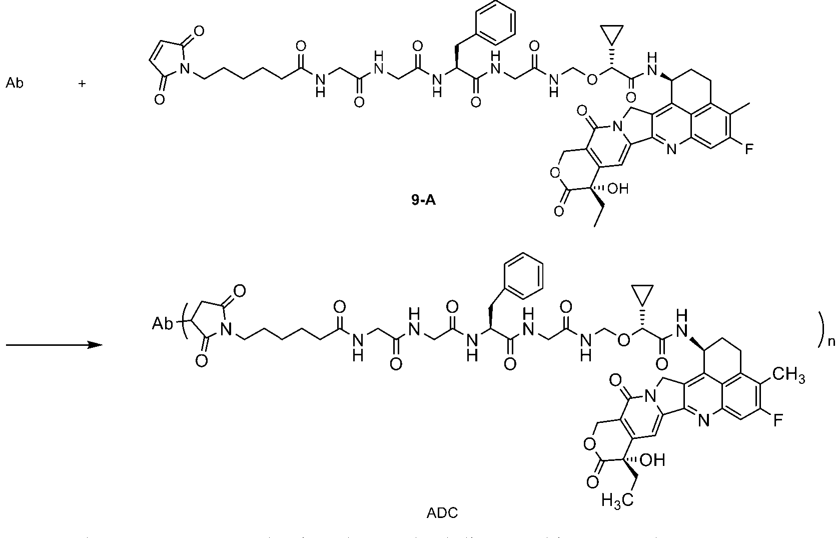

Compound 9-A was prepared using the method disclosed in Example 9 on "pages 58-61 of the specification of Patent Application No. WO2020063676A1".

### Example 4-3-1: ADC-4

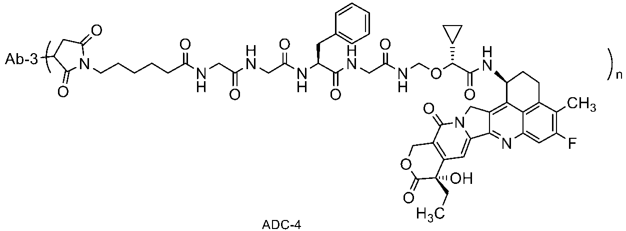

A prepared aqueous solution of tris(2-carboxyethyl)phosphine hydrochloride (TCEP.HCl) (10 mM, 73.3 µL, 733 nmol) was added to antibody Ab-3 in an aqueous PBS buffer solution (a 0.05 M aqueous PBS buffer solution (pH 6.3); 10.0 mg/mL, 2.0 mL, 133.3 nmol) at 37 °C. The mixture was shaken on a water bath shaker at 37 °C for 3 h, and the reaction was stopped. The reaction mixture was cooled to 25 °C, then buffer-exchanged into a 30 mM histidine-acetic acid buffer (pH 5.0) using a Sephadex G25 gel column, and concentrated to 10 mg/mL.

Compound 9-A (2.15 mg, 2.0 µmol) was dissolved in a proper amount (100 µL) of dimethyl sulfoxide, and the solution was added to the above reaction mixture. The resulting mixture was shaken on a thermostatic shaker at 25 °C for 3 h, and the reaction was stopped. The reaction mixture was desalted and purified using a Sephadex G25 gel column (elution phase: a 0.01 M aqueous His buffer solution (pH 5.5), containing 8% sucrose) to give the title product ADC-4 in the His buffer, which was then refrigerated at 4 °C.

Average calculated by RP-HPLC: n = 7.44.

### Example 4-3-2: ADC-5

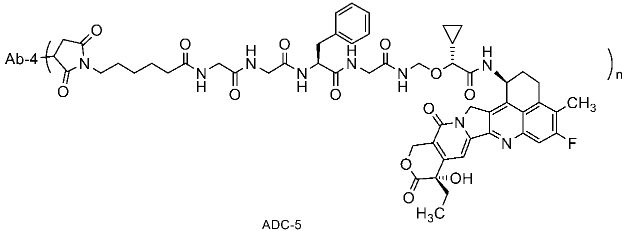

A prepared aqueous solution of tris(2-carboxyethyl)phosphine hydrochloride (TCEP.HCl) (10 mM, 366.7 µL, 3667 nmol) was added to antibody Ab-4 in an aqueous PBS buffer solution (a 0.05 M aqueous PBS buffer solution (pH 6.3); 10.0 mg/mL, 10.0 mL, 666.7 nmol) at 37 °C. The mixture was shaken on a water bath shaker at 37 °C for 3 h, and the reaction was stopped. The reaction mixture was cooled to 25 °C, then buffer-exchanged into a 30 mM histidine-acetic acid buffer (pH 5.0) using a Sephadex G25 gel column, and concentrated to 10 mg/mL.

Compound 9-A (10.74 mg, 10.0 µmol) was dissolved in a proper amount (500 µL) of dimethyl sulfoxide, and the solution was added to the above reaction mixture. The resulting mixture was shaken on a thermostatic shaker at 25 °C for 3 h, and the reaction was stopped. The reaction mixture was desalted and purified using a Sephadex G25 gel column (elution phase: a 0.01 M aqueous His buffer solution (pH 5.5), containing 8% sucrose) to give the title product ADC-5 in the His buffer, which was then refrigerated at 4 °C.

Average calculated by RP-HPLC: n = 7.56.

### Example 4-3-3: ADC-6

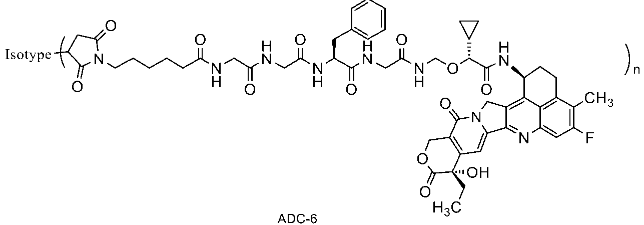

A prepared aqueous solution of tris(2-carboxyethyl)phosphine hydrochloride (TCEP.HCl) (10 mM, 81.8 µL, 818 nmol) was added to the antibody Isotype in an aqueous PBS buffer solution (a 0.05 M aqueous PBS buffer solution (pH 6.3); 10.0 mg/mL, 2.2 mL, 146.7 nmol) at 37 °C. The mixture was shaken on a water bath shaker at 37 °C for 3 h, and the reaction was stopped. The reaction mixture was cooled to 25 °C.

Compound 9-A (2.39 mg, 2.23 µmol) was dissolved in a proper amount (110 µL) of dimethyl sulfoxide, and the solution was added to the above reaction mixture. The resulting mixture was shaken on a thermostatic shaker at 25 °C for 3 h, and the reaction was stopped. The reaction mixture was desalted and purified using a Sephadex G25 gel column (elution phase: a 0.01 M aqueous His buffer solution (pH 5.5), containing 8% sucrose) to give the title product ADC-6 in the His buffer, which was then refrigerated at 4 °C.

Average calculated by RP-HPLC: n = 7.6.

### Test Examples

### Test Example 1: Assessment of Cellular-Level Binding of Antibodies by FACS

PA-1 cells (human ovarian teratoma cells, ATCC, Cat. No. CRL-1572), OVCAR3 cells (human ovarian cancer cells, ATCC, HTB-161), OV90 cells (human ovarian cancer cells, Shanghai Zhong Qiao Xin Zhou, ZQ0073), and 293T/human CLDN9 cells, which are CLDN6-expressing tumor cells, were suspended in a FACS buffer (1% BSA + pH 7.4 PBS) to prepare 1 × 10⁶/mL cell suspensions, and the suspensions were added to 96-well round-bottom plates (Corning, 3795) at 100 µL/well. The plates were centrifuged at 300 g for 5 min, and the supernatants were removed. The antibodies to be tested (different concentrations) were added at 100 µL/well. The plates were placed in a refrigerator at 4 °C and incubated in the dark for 1 h. After 3 centrifugal washes at 300 g, APC anti-human IgG Fc (BioLegend, 410712) (at a working concentration) was added, and the plates were placed in a refrigerator at 4 °C and incubated in the dark for 40 min. After 3 centrifugal washes at 300 g, the geometric mean fluorescence intensity was measured using an Invitrogen flow cytometer, and the binding EC₅₀ values of the antibodies to these cells were calculated. The results are shown in Tables 6-1 to 6-2.

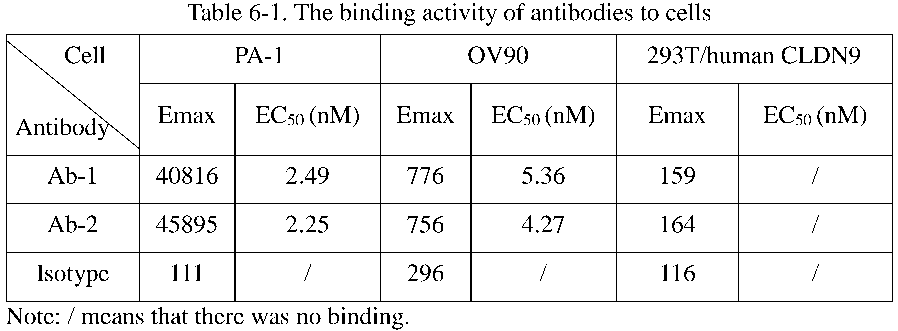

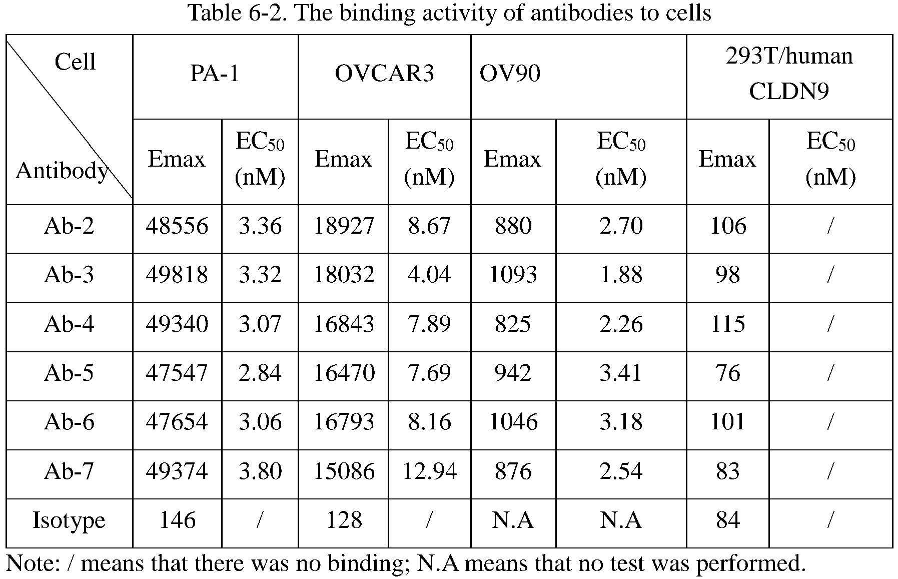

The results show that: the antibodies of the present disclosure could all specifically bind to cell-expressed CLDN6 and did not bind to CLDN9.

### Test Example 2: Assessment of Binding of Antibodies to 293T/Human CLDN3 and CLDN4 Cells by FACS

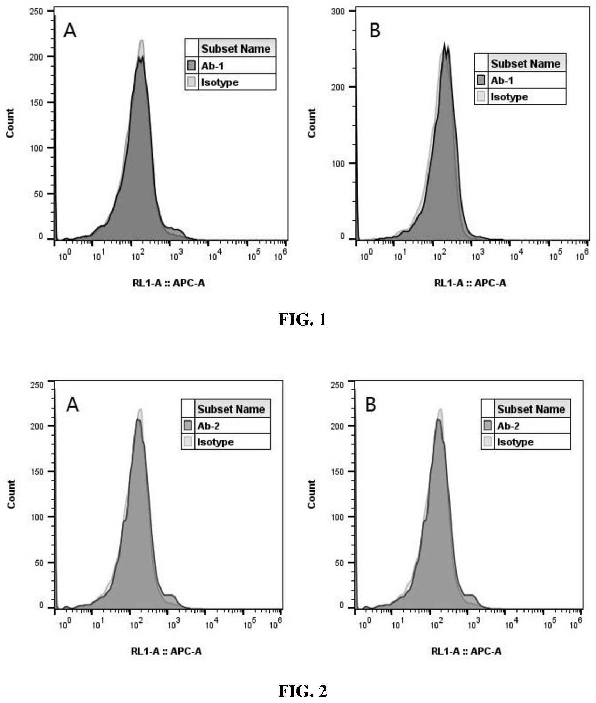

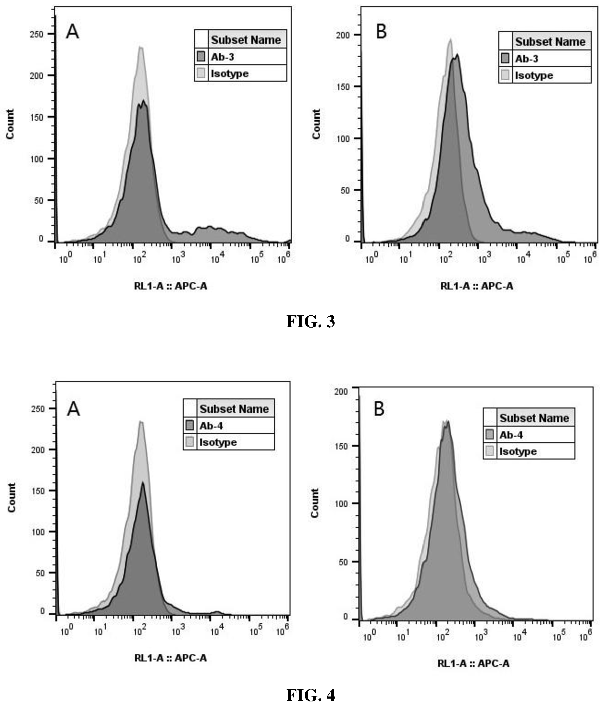

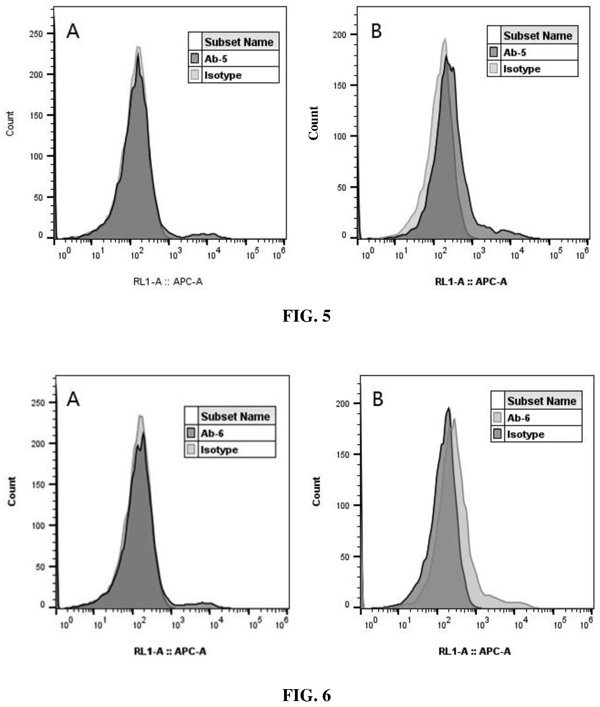

293T/human CLDN3 and CLDN4 cells were suspended in a FACS buffer (containing 1% BSA and pH 7.4 PBS) to prepare 1 × 10⁶/mL cell suspensions, and the suspensions were added to 96-well round-bottom plates (Corning, 3795) at 100 µL/well. The plates were centrifuged at 300 g for 5 min, and the supernatants were removed. The antibodies to be tested (100 µg/mL) were added at 100 µL/well, and the plates were placed in a refrigerator at 4 °C and incubated in the dark for 1 h. After 3 centrifugal washes at 300 g, APC F(ab')2-goat anti-human IgG Fc (BioLegend, 410712) (at a working concentration) was added, and the plates were placed in a refrigerator at 4 °C and incubated in the dark for 40 min. After 3 centrifugal washes at 300 g, the geometric mean fluorescence intensity was measured using an Invitrogen flow cytometer. The results are detailed in FIGs. 1 to 7.

The results show that: as with the negative control antibody Isotype, the antibodies of the present disclosure did not bind to either human CLDN3 or CLDN4.

### Test Example 3: Assessment of Binding of Antibodies to 293T/Human/Rat and Mouse CLDN6/9 Cells by FACS

293T/mouse/rat CLDN6, 293T/human CLDN6-I143V, and 293T/mouse CLDN9 cells were suspended in a FACS buffer (1% BSA + pH 7.4 PBS) to prepare 1 × 10⁶/mL cell suspensions, and the suspensions were added to 96-well round-bottom plates (Corning, 3795) at 100 µL/well. The plates were centrifuged at 300 g for 5 min, and the supernatants were removed. The antibodies to be tested (different concentrations) were added at 100 µL/well. The plates were placed in a refrigerator at 4 °C and incubated in the dark for 1 h. After 3 centrifugal washes at 300 g, APC anti-human IgG Fc (BioLegend, 410712) (at a working concentration) was added, and the plates were placed in a refrigerator at 4 °C and incubated in the dark for 40 min. After 3 centrifugal washes at 300 g, the geometric mean fluorescence intensity was measured using an Invitrogen flow cytometer, and the binding EC₅₀ values of the antibodies to these cells were calculated. The results are shown in Tables 7-1 to 7-4.

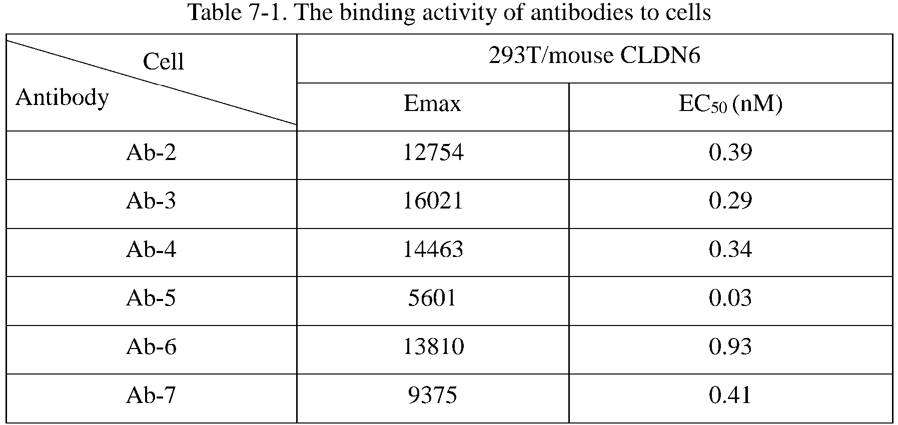

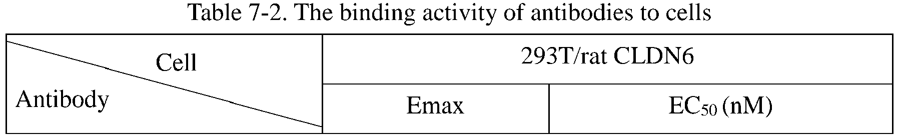

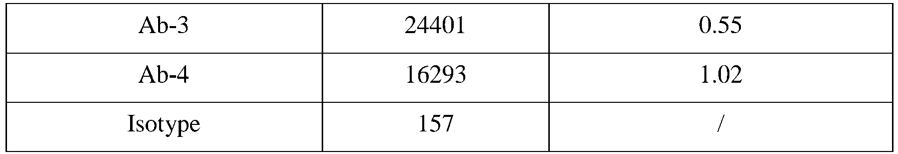

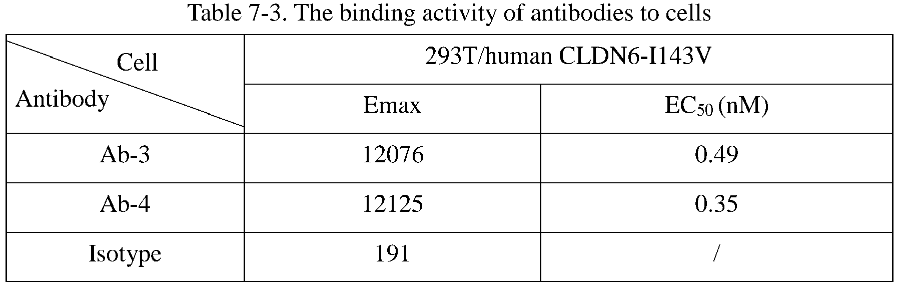

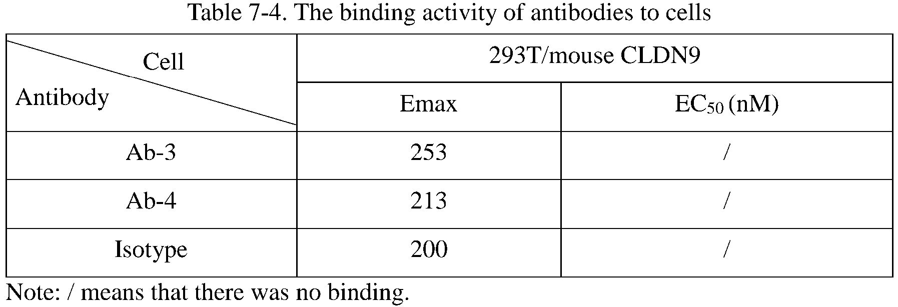

The results show that: the antibodies of the present disclosure exhibited species cross-activity with both mouse CLDN6 and rat CLDN6 and did not bind to mouse CLDN9. In addition, the antibodies of the present disclosure also exhibited binding activity to the human CLDN6 variant I143V.

### Test Example 4: Assay of Anti-CLDN6 Antibodies for Endocytic Activity

DT3C is a recombinantly expressed fusion protein formed by fusing fragment A (toxin-only portion) of diphtheria toxin to fragment 3C (IgG-binding portion) of group G Streptococcus. This protein has high affinity for the Fc structures of antibodies. When antibodies are endocytosed, it enters the cell along with the antibodies. Activated diphtheria toxin (DT) kills cells, indirectly indicating the extent of antibody endocytosis. The *in vitro* endocytic activity of antibodies was evaluated based on IC₅₀ and Imax.

A suspension of PA-1 cells with a cell density of 4 × 10⁴ cells/mL was prepared using a fresh cell culture medium containing 20% low IgG FBS (BioSun, BS-0007-500) and added to a cell culture plate at 50 µL/well, i.e., 2000 cells/well, and the cells were cultured with 5% carbon dioxide at 37 °C for 16 h.

A 4× DT3C solution (2400 nM or 600 nM) was prepared using a serum-free MEM culture medium and sterilized by filtration through a 0.22 µm filter. A 4× antibody solution (400 nM or 100 nM) was prepared using a serum-free MEM culture medium. 75 µL of the DT3C solution (about 70 KD) and 75 µL of the antibody solution (about 150 KD) were well mixed in a 1:1 volume ratio, and the mixture was incubated at room temperature for 30 min and then serially diluted 5-fold with an FBS-free MEM culture medium. A total of 9 doses were obtained, and the 10th point corresponded to the culture medium alone. The wells to which only the culture medium was added were used as the zero point for killing rate calculations. 50 µL of the diluted antibody was added to 50 µL of cells, and the mixture was incubated at 37 °C with 5% CO₂ for 1-3 days. 50 µL of CellTiter-Glo^{™} (CTG) (Promega, G7573) was added to each well, and the plate was incubated at room temperature in a dark place for 10 min. The chemiluminescence was measured using a VICTOR3 multilabel plate reader (PerkinElmer). The results are shown in Tables 8-1 and 8-2. The results show that the antibodies of the present disclosure exhibited relatively good endocytic activity.

**Table 8-1. The endocytic activity of antibodies**

| Antibody | PA-1 | |
|---|---|---|
| | IC₅₀ (nM) | Imax (%) |
| Ab-1 | 0.35 | 71.60 |
| Ab-2 | 0.47 | 72.71 |
| Isotype | >400 | 0.00 |

**Table 8-2. The endocytic activity of antibodies**

| Antibody | PA-1 | |
|---|---|---|
| | IC₅₀ (nM) | Imax (%) |
| Ab-3 | 0.2983 | 69.31 |
| Ab-4 | 0.2556 | 70.44 |
| Ab-5 | 0.3322 | 67.47 |
| Ab-6 | 0.1695 | 71.02 |
| Ab-7 | 0.2375 | 67.63 |
| Isotype | >400 | 11.03 |

### Test Example 5: Assessment of Cellular-Level Binding of ADCs by FACS

PA-1 cells (ATCC, Cat. No. CRL-1572), which are CLDN6-expressing tumor cells, were suspended in a FACS buffer (1% BSA + pH 7.4 PBS) to prepare a 1 × 10⁶/mL cell suspension, and the suspension was added to 96-well round-bottom plates (Corning, 3795) at 100 µL/well. The plates were centrifuged at 300 g for 5 min, and the supernatants were removed. The ADCs to be tested (different concentrations) were added at 100 µL/well. The plates were placed in a refrigerator at 4 °C and incubated in the dark for 1 h. After 3 centrifugal washes at 300 g, APC anti-human IgG Fc (BioLegend, 410712) (at a working concentration) was added, and the plates were placed in a refrigerator at 4 °C and incubated in the dark for 40 min. After 3 centrifugal washes at 300 g, the geometric mean fluorescence intensity was measured using an Invitrogen flow cytometer, and the binding EC₅₀ values of the antibodies to CLDN6-expressing cells were calculated. The results are shown in Tables 9-1 to 9-2.

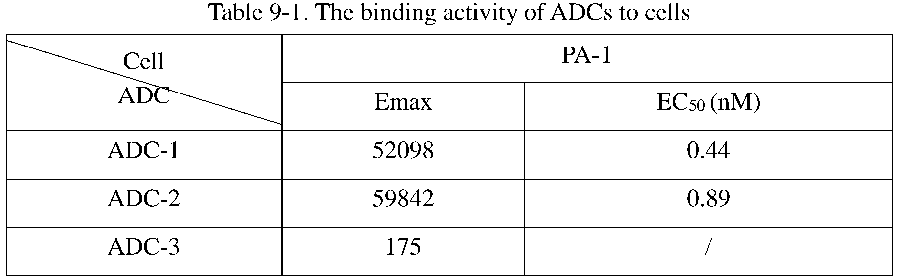

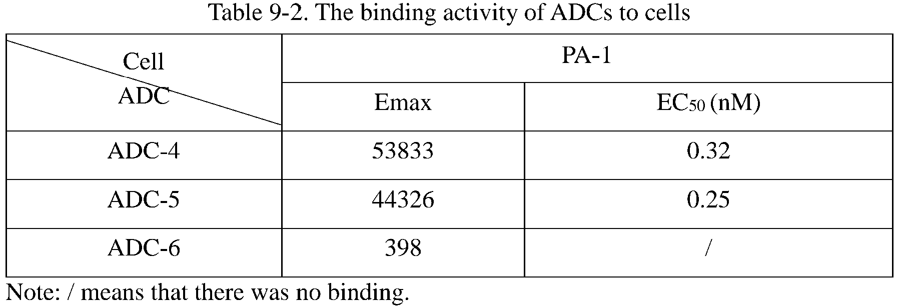

The results show that: the ADC-1, ADC-2, ADC-4, and ADC-5 of the present disclosure could all bind to PA-1 cells well.

### Test Example 6: Assay of ADCs for Killing Activity Against Cells with Different CLDN6 Expression Levels

PA-1 cells (ATCC, Cat. No. CRL-1572), OVCAR3 cells (ATCC, HTB-161), and OV90 cells (Shanghai Zhong Qiao Xin Zhou, ZQ0073), which are CLDN6-expressing tumor cells, were digested with trypsin, neutralized with a fresh culture medium, centrifuged at 1000 rpm, and then resuspended in a culture medium. After counting, the density of the cell suspension of PA-1 cells was adjusted to 3700 cells/mL, the density of the cell suspension of OVCAR3 cells was adjusted to 7400 cells/mL, and the density of the cell suspension of OV90 cells was adjusted to 7400 cells/mL. 135 µL of each cell suspension was added to a 96-well cell culture plate. Specifically, OV90 cells were seeded into a round-bottom 96-well plate (CLS7007-24EA, Corning), and the other cells were seeded into flat-bottom 96-well plates (62096, Sybio). That is, PA-1 cells were added at 500 cells/well, OVCAR3 cells were added at 1000 cells/well, and OV90 cells were added at 1000 cells/well. Cells were not added to the peripheral wells of the 96-well plates, and only 135 µL of the culture medium was added. The cells were cultured with 5% carbon dioxide at 37 °C for 16 h.

The ADC samples were diluted with PBS to 5 µM (10× concentration) as the starting concentration, and the resulting solutions were serially diluted five-fold with PBS. A total of 9 concentrations were obtained, and the 10th concentration point corresponded to drug-free control wells. For OV90 cells, drug solutions were prepared otherwise: The ADC samples were diluted with PBS to 10 µM (10× concentration) as the starting concentration, and the resulting solutions were serially diluted three-fold with PBS. A total of 9 concentrations were obtained, and the 10th concentration point corresponded to drug-free control wells. After drug dilution, the 10× serially diluted solutions were added at 15 µL/well, with the culture medium added to the 10th well, and replicate wells were set for each concentration point. The cells were cultured with 5% carbon dioxide at 37 °C for 6 days.

After the culture was completed, 75 µL of CTG (G7573, Promega) was added to each well of the 96-well plates for PA-1 and OVCAR3 cells, and the plates were incubated at room temperature in a dark place for 10 min. A white film was attached to the bottom of each cell culture plate, the plates were placed on ENVISION, and the chemiluminescence was measured. For the OV90 cell plate: 75 µL of 3D CTG (G9683, Promega) was added to each well, and after 20 min of shaking for lysis at room temperature, 100 µL of liquid was transferred to a white 96-well plate with a film attached to the bottom using a multichannel pipette, the plate was placed on ENVISION, and the chemiluminescence was measured.

The *in vitro* cell killing activity results are shown in Tables 10-1 to 10-3.

**Table 10-1. The in vitro cell killing activity of ADCs**

| ADC | PA-1 | | OVCAR3 | | OV90 | |
|---|---|---|---|---|---|---|
| | IC₅₀ (nM) | Imax (%) | IC₅₀ (nM) | Imax (%) | IC₅₀ (nM) | Imax (%) |
| ADC-1 | 0.014 | 100.00 | 2.07 | 94.36 | 265.9 | 87.22 |
| ADC-2 | 0.011 | 100.00 | 4.16 | 96.34 | 242.5 | 87.15 |
| ADC-3 | 1.56 | 99.97 | 28.46 | 96.56 | 294.00 | 83.09 |

**Table 10-2. The in vitro cell killing activity of ADCs**

| ADC | PA-1 | | OVCAR3 | | OV90 | |
|---|---|---|---|---|---|---|
| | IC₅₀ (nM) | Imax (%) | IC₅₀ (nM) | Imax (%) | IC₅₀ (nM) | Imax (%) |
| ADC-4 | 0.059 | 98.83 | 0.25 | 86.01 | 41.12 | 97.25 |
| ADC-6 | 379.1 | 93.46 | 42.70 | 86.30 | 198.00 | 94.47 |

**Table 10-3. The in vitro cell killing activity of ADCs**

| ADC | PA-1 | | OVCAR3 | | OV90 | |
|---|---|---|---|---|---|---|
| | IC₅₀ (nM) | Imax (%) | IC₅₀ (nM) | Imax (%) | IC₅₀ (nM) | Imax (%) |
| ADC-5 | 0.25 | 99.22 | 1.75 | 93.63 | 36.02 | 94.07 |
| ADC-6 | 309.20 | 95.10 | 60.89 | 94.32 | 194 | 86.96 |

The results show that: the ADC-1, ADC-2, ADC-4, and ADC-5 of the present disclosure all exhibited relatively good killing activity against PA-1, OVCAR3, and OV90 cells.

### Biological Evaluations of In Vivo Activity

### Test Example 7-1: In Vivo Efficacy Evaluation in PA-1 Cell CDX Mouse Model

200 µL of human ovarian cancer PA-1 cells (ATCC, Cat. No. CRL-1572) (4-5 × 10⁶ cells, containing 50% Matrigel, #356234) was subcutaneously inoculated into the right flank of each NOD SCID mouse. Ten to twelve days after inoculation, when the tumor volume reached ~175 to ~190 mm³, mice that were overweight or underweight or had tumors that were too big or too small were excluded, and mice were randomized into groups of 8 according to tumor volume. Administration was started on the same day. ADCs were administered once by tail vein injection at doses of 0.3-0.1 or 0.3-0.15 mg/kg, and the tumor growth was observed. Tumor volume and body weight were measured twice weekly, and the data were recorded. The data were recorded using Excel statistical software: averages were calculated using avg, SD values were calculated using STDEV, and SEM values were calculated using STDEV/SQRT (number of animals per group). Plots were generated using GraphPad Prism software. The data were statistically analyzed using two-way ANOVA or one-way ANOVA.

Tumor volume (V) was calculated using the formula: V = 1/2 × L_{lang} × Lₛₕₒᵣₜ².

Relative tumor proliferation rate T/C (%) = (T - T₀) / (C - C₀) × 100%, where T and C represent the tumor volumes of the treatment groups and the control group at the end of the experiment; T₀ and C₀ represent the tumor volumes at the beginning of the experiment. Tumor growth inhibition rate TGI (%) = 1 - T/C (%).

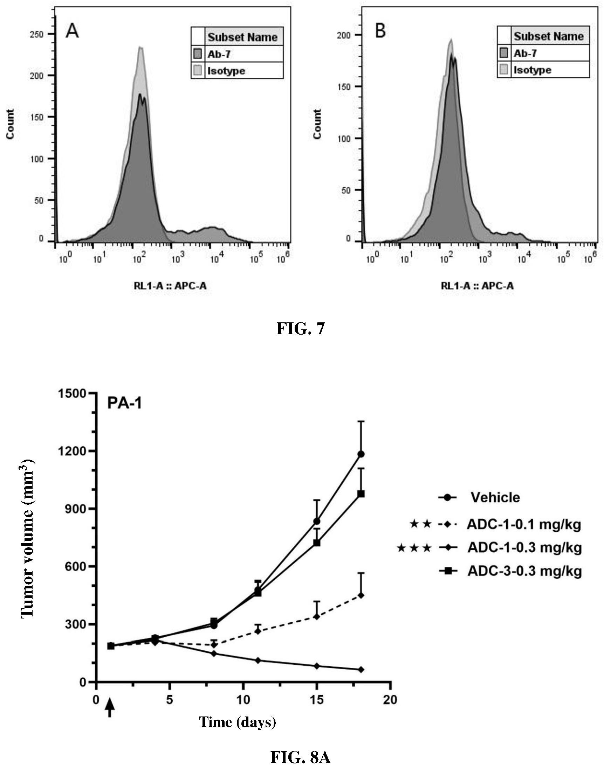

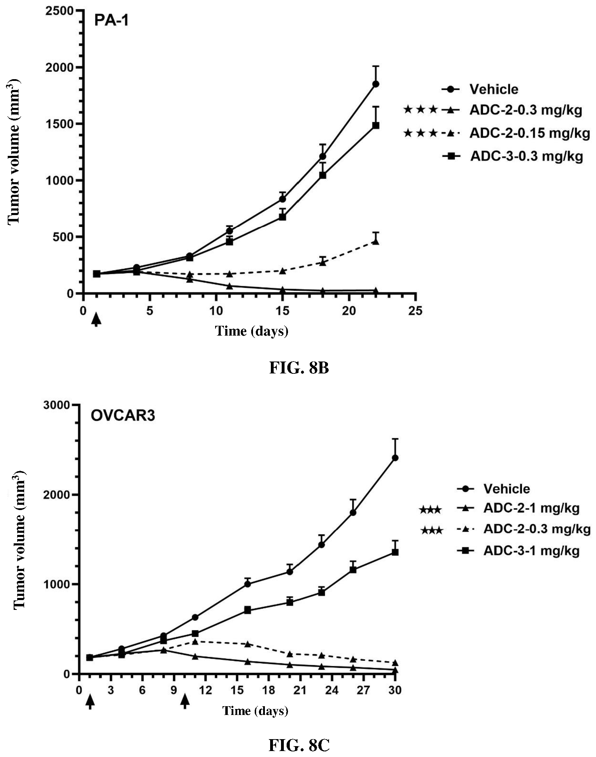

The results are shown in Tables 11-1 and 11-2 and FIGs. 8A and 8B (vehicle refers to Isotype-ADC).

**Table 11-1. The efficacy of ADCs against PA-1 xenograft tumors in NOD SCID mice**

| **ADC** | **Dose** (mg/kg) | **Day-18 TGI** (%) | **P value (vs Isotype-ADC)** |
|---|---|---|---|
| ADC-1 | 0.3 | 112% | p<0.001 |
| | 0.1 | 74% | p<0.01 |
| ADC-3 | 0.3 | 21% | ns |

**Table 11-2. The efficacy of ADCs against PA-1 xenograft tumors in NOD SCID mice**

| **ADC** | **Dose** (mg/kg) | **Day-22 TGI** (%) | **P value (vs Isotype-ADC)** |
|---|---|---|---|
| ADC-2 | 0.3 | 109% | p<0.001 |
| | 0.15 | 83% | p<0.001 |
| ADC-3 | 0.3 | 22% | ns |

| | | | |
|---|---|---|---|
| Note: ns means that there is no statistical significance. | | | |

The results show that ADC-1 could significantly inhibit the growth of subcutaneous xenograft tumors of PA-1 cells at the dose of 0.3 mpk. ADC-2 could significantly inhibit the growth of subcutaneous xenograft tumors of PA-1 cells at the doses of 0.3 mpk and 0.15 mpk.

### Test Example 7-2: In Vivo Efficacy Evaluation in OVCAR3 Cell CDX Mouse Model

200 µL of human ovarian cancer OVCAR3 cells (ATCC, HTB-161) (1 × 10⁷ cells, containing 50% Matrigel/animal, #356234) was subcutaneously inoculated into the right flank of each NDG mouse. Eighteen days after inoculation, when the tumor volume reached ~185 mm³, mice that were overweight or underweight or had tumors that were too big or too small were excluded, and mice were randomized into groups of 8 according to tumor volume. Administration was started on the same day. ADCs were administered twice by tail vein injection at doses of 1-0.3 mg/kg, and the tumor growth was observed. Tumor volume and body weight were measured twice weekly, and the data were recorded. The data were recorded using Excel statistical software: averages were calculated using avg, SD values were calculated using STDEV, and SEM values were calculated using STDEV/SQRT (number of animals per group). Plots were generated using GraphPad Prism software. The data were statistically analyzed using two-way ANOVA or one-way ANOVA.

Tumor volume (V) was calculated using the formula: V = 1/2 × L_{lang} × Lₛₕₒᵣₜ².

Relative tumor proliferation rate T/C (%) = (T - T₀) / (C - C₀) × 100%, where T and C represent the tumor volumes of the treatment groups and the control group at the end of the experiment; T₀ and C₀ represent the tumor volumes at the beginning of the experiment. Tumor growth inhibition rate TGI (%) = 1 - T/C (%).

The results are shown in Table 11-3 and FIG. 8C (vehicle refers to Isotype-ADC).

**Table 11-3. The efficacy of ADCs against OVCAR3 xenograft tumors in NDG mice**

| **ADC** | **Dose (mg/kg)** | **Day-30 TGI (%)** | **P value (vs Isotype-ADC)** |
|---|---|---|---|
| ADC-2 | 1 | 106% | P<0.001 |
| | 0.3 | 103% | P<0.001 |
| ADC-3 | 1 | 47% | ns |

The results show that ADC-2 could significantly inhibit the growth of subcutaneous xenograft tumors of OVCAR3 cells at the doses of 0.3 mpk and 1 mpk.

### Test Example 7-3: In Vivo Efficacy Evaluation in OV90 Cell CDX Mouse Model

200 µL of OV90 cells (Shanghai Zhong Qiao Xin Zhou, ZQ0073) (3 × 10⁶ cells, containing 50% Matrigel/animal, #356234) was subcutaneously inoculated into the right flank of each NOD SCID mouse. Seven days after inoculation, when the mean tumor volume reached ~185 mm³, mice that were overweight or underweight or had tumors that were too big or too small were excluded, and mice were randomized into groups of 8 according to tumor volume. Administration was started on the same day. ADCs were administered once by tail vein injection at doses of 1-0.3 mg/kg. Tumor volume and body weight were measured twice weekly, and the data were recorded. The data were recorded using Excel statistical software: averages were calculated using avg, SD values were calculated using STDEV, and SEM values were calculated using STDEV/SQRT (number of animals per group). Plots were generated using GraphPad Prism software. The data were statistically analyzed using two-way ANOVA or one-way ANOVA.

Tumor volume (V) was calculated using the formula: V = 1/2 × L_{lang} × Lₛₕₒᵣₜ².

Relative tumor proliferation rate T/C (%) = (T - T₀) / (C - C₀) × 100%, where T and C represent the tumor volumes of the treatment groups and the control group at the end of the experiment; T₀ and C₀ represent the tumor volumes at the beginning of the experiment. Tumor growth inhibition rate TGI (%) = 1 - T/C (%).

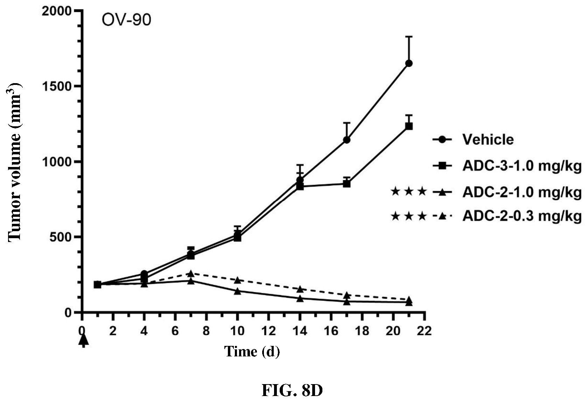

The results are shown in Table 11-4 and FIG. 8D (vehicle refers to Isotype-ADC).

**Table 11-4. The efficacy of ADCs against OV90 xenograft tumors in NOD SCID mice**

| **ADC** | **Dose** (mg/kg) | **Day-21 TGI** (%) | **P value (vs Isotype-ADC)** |
|---|---|---|---|
| ADC-2 | 1 | 108% | P<0.001 |
| | 0.3 | 107% | P<0.001 |
| ADC-3 | 1 | 28% | / |

The results show that ADC-2 could significantly inhibit the growth of OV90 tumors at 1 mpk and 0.3 mpk.

## Claims

1. An anti-CLDN6 antibody, comprising a heavy chain variable region and a light chain variable region, wherein:
the heavy chain variable region comprises a HCDR1, a HCDR2, and a HCDR3 that comprise the amino acid sequences of a HCDR1, a HCDR2, and a HCDR3 from SEQ ID NO: 12, respectively, and
the light chain variable region comprises a LCDR1, a LCDR2, and a LCDR3 that comprise the amino acid sequences of a LCDR1, a LCDR2, and a LCDR3 from SEQ ID NO: 32, 31, 30, 33, 34, or 35, respectively;
preferably,
the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and
the light chain variable region comprises a LCDR1 that comprises the amino acid sequence of SEQ ID NO: 26, 25, 17, 27, 28, or 29, a LCDR2 that comprises the amino acid sequence of SEQ ID NO: 24, and a LCDR3 that comprises the amino acid sequence of SEQ ID NO: 19;
more preferably,
the heavy chain variable region comprises a HCDR1 that comprises the amino acid sequence of SEQ ID NO: 14, a HCDR2 that comprises the amino acid sequence of SEQ ID NO: 15, and a HCDR3 that comprises the amino acid sequence of SEQ ID NO: 16, and
the light chain variable region comprises a LCDR1 that comprises the amino acid sequence of SEQ ID NO: 26 or 25, a LCDR2 that comprises the amino acid sequence of SEQ ID NO: 24, and a LCDR3 that comprises the amino acid sequence of SEQ ID NO: 19.

2. The anti-CLDN6 antibody according to claim 1, wherein the antibody is a murine-derived antibody, a chimeric antibody, a humanized antibody, or a fully human-derived antibody, preferably a humanized antibody.

3. The anti-CLDN6 antibody according to claim 1 or 2, wherein:
the heavy chain variable region comprises the amino acid sequence of SEQ ID NO: 12, and the light chain variable region comprises the amino acid sequence of SEQ ID NO: 32, 31, 30, 33, 34, or 35;
preferably, the heavy chain variable region comprises the amino acid sequence of SEQ ID NO: 12, and the light chain variable region comprises the amino acid sequence of SEQ ID NO: 32 or 31.

4. The anti-CLDN6 antibody according to any one of claims 1 to 3, wherein the anti-CLDN6 antibody is an antibody fragment; preferably, the antibody fragment is a Fab, Fab', F(ab')2, Fd, Fv, scFv, dsFv, or dAb.

5. The anti-CLDN6 antibody according to any one of claims 1 to 3, wherein the anti-CLDN6 antibody comprises a heavy chain constant region and a light chain constant region;
preferably, the heavy chain constant region is a heavy chain constant region of human IgG1, IgG2, IgG3, or IgG4 or a variant thereof, and the light chain constant region is a light chain constant region of human κ or λ or a variant thereof;
more preferably, the heavy chain constant region comprises the amino acid sequence of SEQ ID NO: 20, and the light chain constant region comprises the amino acid sequence of SEQ ID NO: 21.

6. The anti-CLDN6 antibody according to claim 5, wherein the anti-CLDN6 antibody comprises a heavy chain and a light chain, wherein:
the heavy chain comprises the amino acid sequence of SEQ ID NO: 22, and the light chain comprises the amino acid sequence of SEQ ID NO: 38, 37, 36, 39, 40, or 41;
preferably, the heavy chain comprises the amino acid sequence of SEQ ID NO: 22, and the light chain comprises the amino acid sequence of SEQ ID NO: 38 or 37.

7. An antibody-drug conjugate or a pharmaceutically acceptable salt thereof, being represented by general formula (I): 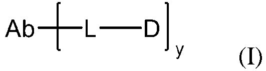
wherein Ab is the anti-CLDN6 antibody according to any one of claims 1 to 6;
L is a linker linking Ab and D;
Ab binds directly to L via an amino acid thereof, or Ab binds to L via a glycan chain or remodeled glycan chain thereof; preferably, Ab binds directly to L via an amino acid residue at position 297 thereof, or Ab binds to L via a glycan chain or remodeled glycan chain at position 297 thereof; more preferably, Ab binds to L via a remodeled glycan chain at Asn297 thereof;
y is 1 to 10;
D is represented by general formula (D1), formula (D1-S), or formula (D1-R): 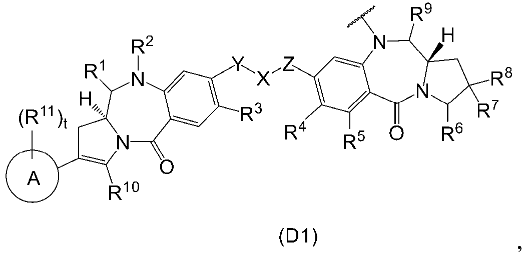 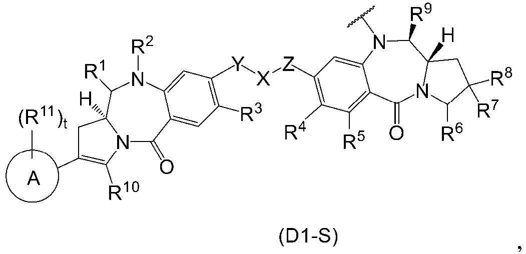 or 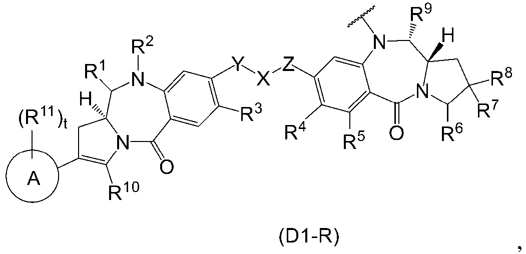
wherein:
X is (CR^{a}R^{b})ₛ;
Y and Z are identical or different and are each independently selected from the group consisting of oxygen, sulfur, and NR^{c};
R¹ is selected from the group consisting of hydrogen, halogen, and alkyl; or R¹, together with the carbon atom to which it is attached, forms C=O;
R² is hydrogen or alkyl;
or R¹ and R², together with the carbon atom and the nitrogen atom to which they are attached, form C=N;
R³ and R⁴ are identical or different and are each independently selected from the group consisting of hydrogen, alkyl, OR^{d}, halogen, haloalkyl, hydroxyalkyl, SH, S-alkyl, and NR^{e}R^{f};
R⁵ is selected from the group consisting of hydrogen, halogen, alkyl, and haloalkyl;
R⁶ is selected from the group consisting of hydrogen, halogen, alkyl, and haloalkyl; or R⁶, together with the carbon atom to which it is attached, forms C=O;
R⁷ and R⁸ are identical or different and are each independently selected from the group consisting of hydrogen, halogen, alkyl, and haloalkyl; or R⁷ and R⁸, together with the carbon atom to which they are attached, form C=O; or R⁷ and R⁸, together with the carbon atom to which they are attached, form cycloalkyl or heterocyclyl, wherein the cycloalkyl or heterocyclyl is independently and optionally substituted with one or more substituents selected from the group consisting of oxo, halogen, alkyl, haloalkyl, cyano, NR^{g}R^{h}, and ORⁱ;
R⁹ is hydroxy or alkoxy;
R¹⁰ is selected from the group consisting of hydrogen, hydroxy, alkyl, haloalkyl, hydroxyalkyl, and alkoxy;
ring A is selected from the group consisting of cycloalkyl, heterocyclyl, aryl, and heteroaryl;
R¹¹ is selected from the group consisting of hydrogen, halogen, alkyl, haloalkyl, OR^{j}, C(O)R^{k}, C(O)OR^{k}, cycloalkyl, heterocyclyl, cycloalkylalkyl, heterocyclylalkyl, aryl, heteroaryl, arylalkyl, and heteroarylalkyl;
R^{a} and R^{b} are identical or different and are each independently selected from the group consisting of hydrogen, halogen, alkyl, haloalkyl, alkoxy, hydroxy, cyano, amino, and hydroxyalkyl;
R^{c}, R^{e}, R^{f}, R^{g}, and R^{h} are identical or different and are each independently selected from the group consisting of hydrogen, alkyl, and haloalkyl;
R^{d}, Rⁱ, and R^{j} are identical or different and are each independently selected from the group consisting of hydrogen, alkyl, haloalkyl, hydroxyalkyl, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, cyano, amino, hydroxy, alkoxy, and hydroxyalkyl;
R^{k} are identical or different and are each independently selected from the group consisting of hydrogen, alkyl, haloalkyl, hydroxy, and hydroxyalkyl;
s is 1, 2, 3, 4, 5, or 6;
t is 1, 2, 3, 4, or 5.

8. The antibody-drug conjugate or the pharmaceutically acceptable salt thereof according to claim 7, wherein Y and Z are both oxygen.

9. The antibody-drug conjugate or the pharmaceutically acceptable salt thereof according to claim 7 or 8, wherein R¹ and R² are both hydrogen.

10. The antibody-drug conjugate or the pharmaceutically acceptable salt thereof according to any one of claims 7 to 9, wherein ring A is 6- to 10-membered aryl; preferably, ring A is phenyl.

11. The antibody-drug conjugate or the pharmaceutically acceptable salt thereof according to any one of claims 7 to 10, wherein D is represented by general formula (D2), formula (D2-S), or formula (D2-R): 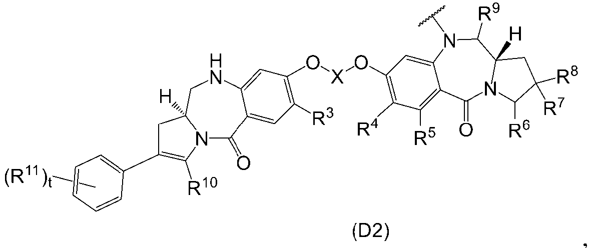 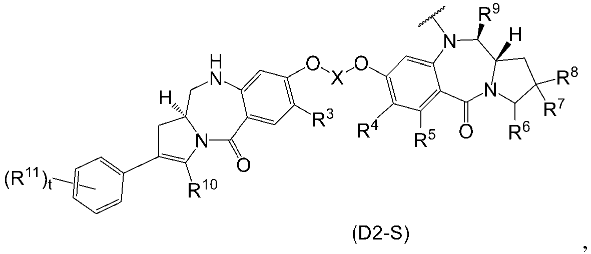 or 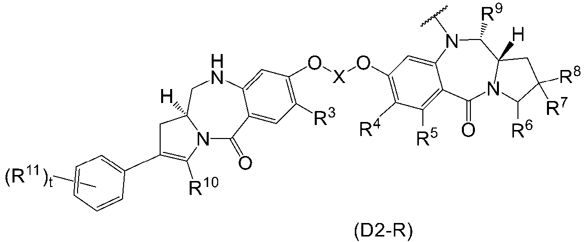 wherein X, R³ to R¹¹, and t are as defined in claim 7.

12. The antibody-drug conjugate or the pharmaceutically acceptable salt thereof according to any one of claims 7 to 11, wherein:
X is (CR^{a}R^{b})ₛ, wherein R^{a} and R^{b} are identical or different and are each independently selected from the group consisting of hydrogen, halogen, C₁₋₆ alkyl, C₁₋₆ haloalkyl, C₁₋₆ alkoxy, hydroxy, and C₁₋₆ hydroxyalkyl;
preferably, X is (CH₂)ₛ, wherein s is as defined in claim 7.

13. The antibody-drug conjugate or the pharmaceutically acceptable salt thereof according to any one of claims 7 to 12, wherein:
R³ and R⁴ are both OR^{d}, and R^{d} is selected from the group consisting of hydrogen, C₁₋₆ alkyl, C₁₋₆ haloalkyl, C₁₋₆ hydroxyalkyl, 3- to 6-membered cycloalkyl, and 3- to 6-membered heterocyclyl, wherein the 3- to 6-membered cycloalkyl or 3- to 6-membered heterocyclyl is independently and optionally substituted with one or more substituents selected from the group consisting of oxo, halogen, C₁₋₆ alkyl, C₁₋₆ haloalkyl, cyano, amino, hydroxy, C₁₋₆ alkoxy, and C₁₋₆ hydroxyalkyl;
preferably, R³ and R⁴ are both OR^{d}, and R^{d} is C₁₋₆ alkyl or 3- to 6-membered cycloalkyl;
more preferably, R³ is C₁₋₆ alkoxy, and R⁴ is 3- to 6-membered cycloalkyloxy; most preferably, R³ is methoxy, and R⁴ is cyclopropyloxy.

14. The antibody-drug conjugate or the pharmaceutically acceptable salt thereof according to any one of claims 7 to 13, wherein R⁵ and R⁶ are both hydrogen.

15. The antibody-drug conjugate or the pharmaceutically acceptable salt thereof according to any one of claims 7 to 14, wherein R⁷ and R⁸ are identical or different and are each independently selected from the group consisting of hydrogen, halogen, C₁₋₆ alkyl, and C₁₋₆ haloalkyl; or R⁷ and R⁸, together with the carbon atom to which they are attached, form 3- to 6-membered cycloalkyl or 3- to 6-membered heterocyclyl, wherein the 3- to 6-membered cycloalkyl or 3- to 6-membered heterocyclyl is independently and optionally substituted with one or more substituents selected from the group consisting of oxo, halogen, C₁₋₆ alkyl, C₁₋₆ haloalkyl, hydroxy, and amino; preferably, R⁷ and R⁸, together with the carbon atom to which they are attached, form 3- to 6-membered cycloalkyl, wherein the 3- to 6-membered cycloalkyl is optionally substituted with one or more substituents selected from the group consisting of oxo, halogen, C₁₋₆ alkyl, C₁₋₆ haloalkyl, hydroxy, C₁₋₆ hydroxyalkyl, and amino; more preferably, R⁷ and R⁸, together with the carbon atom to which they are attached, form 3- to 6-membered cycloalkyl; most preferably, R⁷ and R⁸, together with the carbon atom to which they are attached, form cyclopropyl.

16. The antibody-drug conjugate or the pharmaceutically acceptable salt thereof according to any one of claims 7 to 15, wherein R⁹ is hydroxy.

17. The antibody-drug conjugate or the pharmaceutically acceptable salt thereof according to any one of claims 7 to 16, wherein R¹⁰ is selected from the group consisting of hydrogen, hydroxy, C₁₋₆ alkyl, C₁₋₆ haloalkyl, C₁₋₆ hydroxyalkyl, and C₁₋₆ alkoxy; preferably, R¹⁰ is hydrogen.

18. The antibody-drug conjugate or the pharmaceutically acceptable salt thereof according to any one of claims 7 to 17, wherein R¹¹ is selected from the group consisting of hydrogen, halogen, C₁₋₆ alkyl, C₁₋₆ haloalkyl, hydroxy, and C₁₋₆ alkoxy; preferably, R¹¹ is hydrogen or C₁₋₆ alkoxy.

19. The antibody-drug conjugate or the pharmaceutically acceptable salt thereof according to any one of claims 7 to 18, wherein D is selected from the group consisting of the following structures: 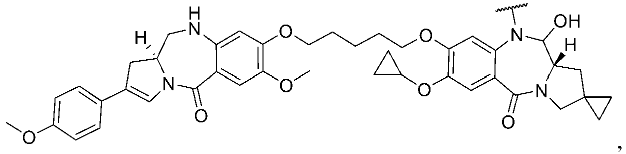 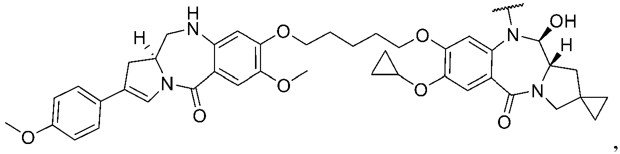 and

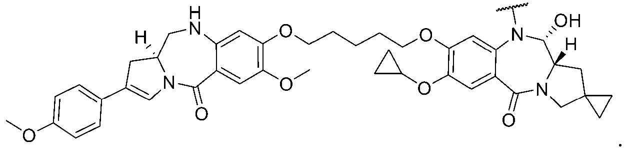

20. The antibody-drug conjugate or the pharmaceutically acceptable salt thereof according to any one of claims 7 to 19, wherein L is -L^{a}-L^{b}-L^{c}-L^{d}-;
L^{a} is selected from the group consisting of 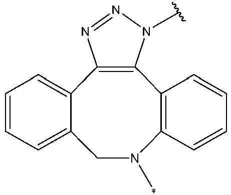 and 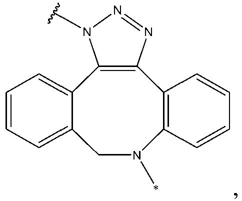 wherein the asterisk * indicates binding to L^{b}, and the wavy line 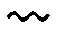 indicates binding to the glycan chain or remodeled glycan chain of Ab;
L^{b} is selected from the group consisting of -C(O)-(CR^{m}Rⁿ-CR^{p}R^{q})ₜ₁-C(O)-, -C(O)-(CR^{m}Rⁿ-CR^{p}R^{q})ₜ₁-C(O)-NR^{s}-(CR^{m}Rⁿ-CR^{p}R^{q})ₜ₂-C(O)-, -C(O)-(CR^{m}Rⁿ-CR^{p}R^{q})t₁-C(O)-NR^{s}-(CR^{m}Rⁿ-CR^{p}R^{q}-O)ₜ₂-CR^{u}R^{v}-C(O)-, -C(O)-(CR^{m}Rⁿ-CR^{p}R^{q})ₜ₁-NR^{s}-C(O)-(CR^{m}Rⁿ-CR^{p}R^{q}-O)ₜ₂-(CR^{m}Rⁿ-CR^{p}R^{q})ₜ₃-C(O)-, and -(CR^{u}R^{v})ₜ₄-O-C(O)-;
L^{c} is a peptide residue composed of 2 to 7 amino acids;
L^{d} is -NR^{w}-W-CR^{x}R^{y}O-C(O)-, -NR^{w}-CR^{x}R^{y}O-R^{z}-C(O)-, or a chemical bond;
R^{m}, Rⁿ, R^{p}, R^{q}, R^{u}, R^{v}, R^{x}, and R^{y} are identical or different and are each independently selected from the group consisting of hydrogen, halogen, alkyl, haloalkyl, alkoxy, hydroxyalkyl, hydroxy, cyano, amino, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
or R^{m} and Rⁿ, together with the carbon atom to which they are attached, form cycloalkyl or heterocyclyl, R^{p} and R^{q}, together with the carbon atom to which they are attached, form cycloalkyl or heterocyclyl, R^{u} and R^{v}, together with the carbon atom to which they are attached, form cycloalkyl or heterocyclyl, and R^{x} and R^{y}, together with the carbon atom to which they are attached, form cycloalkyl or heterocyclyl, wherein the cycloalkyl or heterocyclyl is independently and optionally substituted with one or more substituents selected from the group consisting of oxo, halogen, alkyl, haloalkyl, alkoxy, hydroxyalkyl, hydroxy, cyano, and amino;
or R^{m} and R^{p}, together with the carbon atoms to which they are attached, form cycloalkyl or heterocyclyl, wherein the cycloalkyl or heterocyclyl is independently and optionally substituted with one or more substituents selected from the group consisting of oxo, halogen, alkyl, haloalkyl, alkoxy, hydroxyalkyl, hydroxy, cyano, and amino;
R^{s}, R^{w}, and R^{z} are identical or different and are each independently selected from the group consisting of hydrogen, alkyl, haloalkyl, hydroxyalkyl, cycloalkyl, heterocyclyl, aryl, and heteroaryl, wherein the cycloalkyl, heterocyclyl, aryl, and heteroaryl are each independently and optionally substituted with one or more substituents selected from the group consisting of oxo, halogen, alkyl, haloalkyl, alkoxy, hydroxyalkyl, hydroxy, cyano, and amino;
W is aryl or heteroaryl, wherein the aryl or heteroaryl is independently and optionally substituted with one or more substituents selected from the group consisting of halogen, hydroxy, cyano, amino, alkyl, haloalkyl, and alkoxy; preferably, W is phenyl or 5- or 6-membered heteroaryl, wherein the phenyl or 5- or 6-membered heteroaryl is independently and optionally substituted with one or more substituents selected from the group consisting of halogen, oxo, hydroxy, cyano, amino, C₁₋₆ alkyl, C₁₋₆ haloalkyl, and C₁₋₆ alkoxy;
t1, t2, t3, and t4 are identical or different and are each independently 1, 2, 3, 4, 5, or 6.

21. The antibody-drug conjugate or the pharmaceutically acceptable salt thereof according to claim 20, wherein L^{b} is -C(O)-(CR^{m}Rⁿ-CR^{p}R^{q})ₜ₁-C(O)-, wherein R^{m}, Rⁿ, R^{p}, and R^{q} are identical or different and are each independently hydrogen or C₁₋₆ alkyl, and t1 is 1, 2, 3, 4, 5, or 6; preferably, L^{b} is -C(O)-CH₂-CH₂-C(O)-.

22. The antibody-drug conjugate or the pharmaceutically acceptable salt thereof according to claim 20 or 21, wherein L^{c} is selected from the group consisting of peptide residues formed from amino acids among phenylalanine (F), alanine (A), proline (P), isoleucine (I), leucine (L), glycine (G), valine (V), lysine (K), citrulline (Cit), serine (S), glutamic acid (E), and aspartic acid (D), wherein the peptide residues are each independently and optionally substituted with one or more substituents selected from the group consisting of halogen, hydroxy, cyano, amino, C₁₋₆ alkyl, C₁₋₆ haloalkyl, C₁₋₆ alkoxy, 3- to 6-membered cycloalkyl, 3- to 6-membered heterocyclyl, 6- to 10-membered aryl, and 5- to 10-membered heteroaryl; preferably, L^{c} is selected from the group consisting of -GGVA-, -GG-(D-)VA-, -VA-, -GGFG-, -GGPI-, -GGVCit-, -GGVK-, -GG(D-)PI-, and - GGPL-; more preferably, L^{c} is -GGVA-.

23. The antibody-drug conjugate or the pharmaceutically acceptable salt thereof according to any one of claims 20 to 22, wherein L^{d} is -NR^{w}-W-CR^{x}R^{y}O-C(O)-, wherein R^{w}, R^{x}, and R^{y} are identical or different and are each independently hydrogen or C₁₋₆ alkyl, and W is selected from the group consisting of 1,4-phenyl, 2,5-pyridyl, 3,6-pyridyl, 2,5-pyrimidinyl, and 2,5-thienyl;
preferably, L^{d} is -NH-W-CH₂-O-C(O)-, and W is 1,4-phenyl.

24. The antibody-drug conjugate or the pharmaceutically acceptable salt thereof according to any one of claims 20 to 23, wherein L is

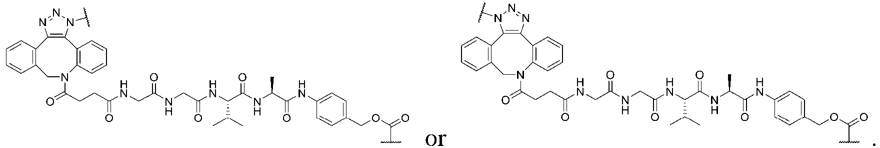

25. The antibody-drug conjugate or the pharmaceutically acceptable salt thereof according to any one of claims 20 to 24, wherein the remodeled glycan chain has a structure of: 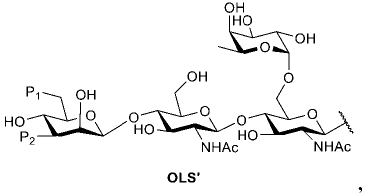
wherein the wavy line 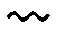 indicates binding to the Asn at position 297 of a heavy chain of Ab;
P₁ and P₂ are identical or different and are each independently selected from the group consisting of hydroxy, *-(CR^{p1}R^{q1}-CR^{s1}R^{t1}-O)s¹-, and *-(CR^{p1}R^{q1}-CR^{s1}R^{t1}-O)s²-(CR^{p1}R^{q1}-CR^{s1}R^{t1}-CR^{x1}R^{y1}-O)s³-(CR^{p1}R^{q1}-CR^{s1}R^{t1}-O)s⁴-, wherein R^{p1}, R^{q1}, R^{s1}, R^{t1}, R^{x1}, and R^{y1} are identical or different and are each independently selected from the group consisting of hydrogen, halogen, C₁₋₆ alkyl, C₁₋₆ haloalkyl, C₁₋₆ alkoxy, C₁₋₆ hydroxyalkyl, hydroxy, cyano, amino, 3- to 6-membered cycloalkyl, 3- to 6-membered heterocyclyl, 6-to 10-membered aryl, and 5- to 10-membered heteroaryl, wherein the 3- to 6-membered cycloalkyl, 3- to 6-membered heterocyclyl, 6- to 10-membered aryl, and 5- to 10-membered heteroaryl are each independently and optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxy, cyano, and amino; the asterisk * indicates binding to the linker L;
s¹ is 1-10, preferably 1-5; s² is 0-10, preferably 1-5; s³ is 1-10, preferably 1-5; s⁴ is 0-10, preferably 1-5;
provided that P₁ and P₂ are not simultaneously hydroxy or *-(CH₂CH₂O)s¹-;
preferably, P₁ is hydroxy, and P₂ is *-(CR^{p1}R^{q1}-CR^{s1}R^{t1}-O)ₛ₂-(CR^{p1}R^{q1}-CR^{s1}R^{t1}-CR^{x1}R^{y1}-O)ₛ₃-(CR^{p1}R^{q1}-CR^{s1}R^{t1}-O)ₛ₄-, wherein R^{p1}, R^{q1}, R^{s1}, R^{t1}, R^{x1}, and R^{y1} are identical or different and are each independently selected from the group consisting of hydrogen, halogen, C₁₋₆ alkyl, and C₁₋₆ haloalkyl, s2 is 1, s3 is 1, and s4 is 1;
more preferably, the remodeled glycan chain has a structure of:

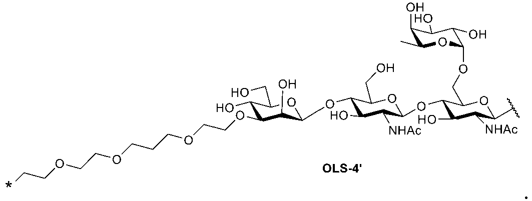

26. The antibody-drug conjugate or the pharmaceutically acceptable salt thereof according to any one of claims 7 to 25, being represented by general formula (II): 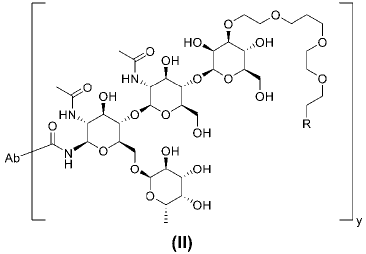 wherein: 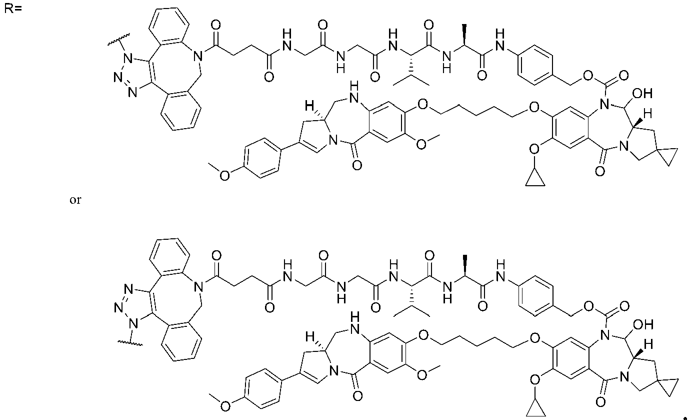 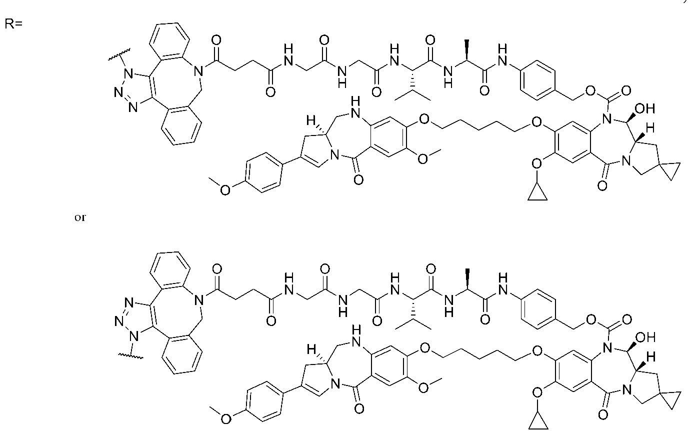 or 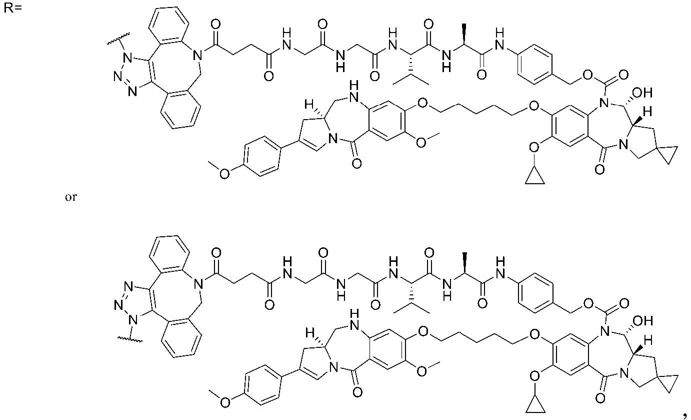
Ab is the anti-CLDN6 antibody according to any one of claims 1 to 6;
y is 1 to 10; preferably, y is 1 to 4; more preferably, y is 1 to 2; most preferably, y is 2.

27. A method for preparing an antibody-drug conjugate represented by general formula (II) or a pharmaceutically acceptable salt thereof, comprising the following step: 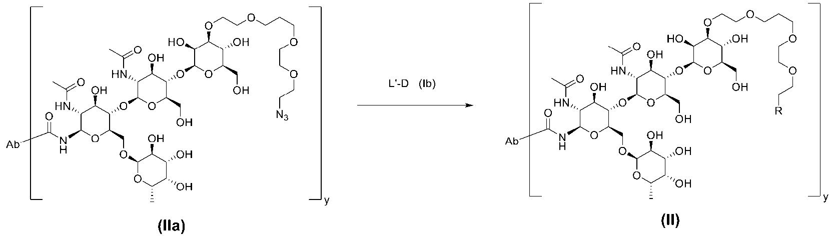 reacting a compound represented by general formula (IIa) or a salt thereof with a compound represented by general formula (Ib) or a salt thereof to give the antibody-drug conjugate represented by general formula (II) or the pharmaceutically acceptable salt thereof,
wherein:
L' is L^{aa}-L^{b}-L^{c}-L^{d}-;
L^{aa} is selected from the group consisting of 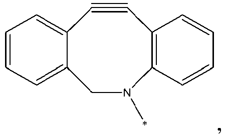 wherein the asterisk * indicates binding to L^{b};
Ab is the anti-CLDN6 antibody according to any one of claims 1 to 6;
y is 1 to 10;
L^{b}, L^{c}, and L^{d} are as defined in any one of claims 20 to 26;
R is as defined in claim 26;
D is as defined in any one of claims 7 to 19.

28. A pharmaceutical composition, comprising: the anti-CLDN6 antibody according to any one of claims 1 to 6 or the antibody-drug conjugate or the pharmaceutically acceptable salt thereof according to any one of claims 7 to 26, and one or more pharmaceutically acceptable carriers, diluents, or excipients.

29. An isolated nucleic acid, encoding the anti-CLDN6 antibody according to any one of claims 1 to 6.

30. A host cell, comprising the isolated nucleic acid according to claim 29.

31. A method for preventing or treating a tumor, comprising administering to a subject the anti-CLDN6 antibody according to any one of claims 1 to 6, the antibody-drug conjugate or the pharmaceutically acceptable salt thereof according to any one of claims 7 to 26, or the pharmaceutical composition according to claim 28, wherein:
preferably, the tumor is selected from the group consisting of ovarian cancer, lung cancer, endometrial cancer, gastric cancer, cervical cancer, testicular cancer, placental choriocarcinoma, renal cancer, urothelial carcinoma, colorectal cancer, prostate cancer, glioblastoma multiforme, brain tumor, pancreatic cancer, breast cancer, melanoma, liver cancer, bladder cancer, and esophageal cancer;
more preferably, the tumor is selected from the group consisting of ovarian cancer, lung cancer, endometrial cancer, and testicular cancer;
most preferably, the tumor is ovarian cancer.
